# EUROPEAN PATENT SPECIFICATION

(11) **EP 1 680 508 B1**
(45) Date of publication and mention of the grant of the patent: **30.03.2011**
(21) Application number: 03759088.2
(22) Date of filing: 28.10.2003
(51) Int. Cl.: C12N 15/74, C12N 15/62, A61K 48/00

(54) **SITE-SPECIFIC INTESTINAL DELIVERY AND/OR PRODUCTION OF BIOLOGICALLY ACTIVE SUBSTANCES**
ORTSPEZIFISCHE VERABREICHUNG VON AKTIVEN SUBSTANZEN IM DARM
PRODUCTION ET/OU ADMINISTRATION INTESTINALE SPECIFIQUE DE SITE DE SUBSTANCES ACTIVES

(43) Date of publication of application: 19.07.2006
(73) Proprietor: Friesland Brands B.V., 7943 PE Meppel (NL)
(72) Inventor: BRON, Peter, Allard, NL-6701 DB Wageningen (NL); DE VOS, Willem, Meindert, NL-6721 SJ Bennekom (NL); KLEEREBEZEM, Michiel, NL-6716 GG Ede (NL)
(74) Representative: van Westenbrugge, Andries
(86) International application number: PCT/NL2003/000733
(87) International publication number: WO 2005/040387

(56) References cited:
- EP-A- 1 084 709
- WO-A-96/16179
- WO-A-96/32486
- WO-A-99/11284
- WO-A-03/076463
- STEIDLER L ET AL: "Treatment of murine colitis by Lactococcus lactis secreting interleukin-10" SCIENCE, AMERICAN ASSOCIATION FOR THE ADVANCEMENT OF SCIENCE,, US, vol. 289, no. 5483, 25 August 2000 (2000-08-25), pages 1352-1355, XP002208404 ISSN: 0036-8075 cited in the application -& STEIDLER L ET AL.: "Treatment of Murine Colitis by Lactococcus lactis secreting Interleukine-10 - Supplementary material" SCIENCE MAGAZINE, [Online] 25 August 2000 (2000-08-25), pages 1-4, XP002285361 Retrieved from the Internet: URL:www.sciencemag.org/feature/data/104799 7.shl> [retrieved on 2004-06-22]
- WALTER JENS ET AL: "Identification of Lactobacillus reuteri genes specifically induced in the mouse gastrointestinal tract." APPLIED AND ENVIRONMENTAL MICROBIOLOGY, vol. 69, no. 4, April 2003 (2003-04), pages 2044-2051, XP002285323 ISSN: 0099-2240 cited in the application
- ANGELICHIO MICHAEL J ET AL: "In vivo expression technology." INFECTION AND IMMUNITY, vol. 70, no. 12, December 2002 (2002-12), pages 6518-6523, XP002285324 ISSN: 0019-9567 cited in the application
- KLEEREBEZEM MICHIEL ET AL: "Complete genome sequence of Lactobacillus plantarum WCFS1." PROCEEDINGS OF THE NATIONAL ACADEMY OF SCIENCES OF THE UNITED STATES OF AMERICA, vol. 100, no. 4, 18 February 2003 (2003-02-18), pages 1990-1995, XP002285325 ISSN: 0027-8424

### SEQUENCE LISTING

<110> wageningen centre for Food sciences
<120> Site-specific Intestinal Delivery and/or Production of Biologically Active substances
<130> P211063PCT
<160> 62
<170> PatentIn version 3.1
<210> 1
   <211> 2140
   <212> DNA
   <213> unknown
<220>
   <223> lp_0185
<220>
   <221> startcodon
   <222> (553)..(555)
   <223>
<400> 1
   cggcgtatct ggtcgaatcg ctaagccctc atcatgccaa tccaccaagt tcttagaggt    60
   cacgtgttgc catgacttca gtccgtgtac cggaccgaac ggaaagactt ggtagaacag   120
   gtgccactga ccatcaaagt atgaaaagcc attggggtca tttaacaaac ctgatgtagg   180
   ctgaatatga tgttgcatcc gccacttaga ctggcgtacc tgggcaacga gctgtggtag   240
   cttagttgct ggccactgtt cataaggggt ataacgggtt ttacgattcc atatcatgac   300
   gatgagctcc tttttaacgt tcgacttaat tgtcactatt ataaaaccgc ttacactgat   360
   atgtcaatcg tttgacattt ttatattata ttttcatgtc agcgtttcaa attcggcttt   420
   ttaacaagat tatcaagcaa ttcaattttg tatcaaacgc ttgacatatt ttccaaactt   480
   gttattattg ggtctgtaaa gcgcttgcat tattaaatta aaaataattt tagactcagg   540
   gagtgaatta ttatgaatca tcaagaagtt gccgaccgcg tacttaatgc aattggtaaa    600
   aacaacattc aagccgccgc ccactgtgcg acacgccttc gtttggtcat caaggatgaa    660
   tccaagattg atcaacaagc cttagatgac gacgcggacg ttaaggggac cttcgaaact    720
   aacggccaat accaaatcat tattggccct ggcgatgtcg ataaagtcta tgacgcctta    780
   atcgccaaaa caggtcttaa agaagcgacc cccgatgaca tcaaggcagt tgccgctgcg    840
   ggtcaaaaga aaaatccatt aatggacttc ctcaaagtct tatctgatat ttttattcca    900
   atcgtccctg cactagttgc tgggggtcta ttaatggcac taaacaacgt tttgactgcc    960
   gagcatcttt ttatggcgaa gtcagttgtt gaagtttacc ctggcctcaa aggtatcgcc   1020
   gaaatgatta acgcgatggc tagtgcgccg tttactttct tgccaatcct cttaggattt   1080
   tcagcaacga agcgcttcgg tggcaacccc tatctgggtg ctacgatggg catgatcatg   1140
   gtcttaccat cactagttaa cggctatagc gttgcaacga ccatggctgc tggcaagatg   1200
   gtttactgga acgtctttgg tttacacgtt gcacaagcag gctatcaagg ccaagtgctc   1260
   cctgttttgg ccgttgctta tattttggct actttggaga agttcttcca caaacatatc   1320
   aagggtgcat ttgactttac gttcacgcca atgtttgcaa ttgtcatcac tggctttcta   1380
   actttcacaa tcgtcggtcc tgtcttacga acggtgagcg acgcattaac taacggttta   1440
   gtaggcttat ataacacgac tggttggatt ggcatgggca tctttggcct tctctactcg   1500
   gccattgtca ttactgggct ccaccaaacc ttcccggcta ttgaaaccca gttattggct   1560
   aacgtcgcca aaacgggagg ttcttttatc ttcccagtcg cctcgatggc caacattggg   1620
   caaggtgccg ccactttagc tattttcttt gccactaaga gccaaaagca aaaagccctg   1680
   acttcttcag ccggggtctc agcgttactc ggaattacgg aacctgctat tttcggggtc   1740
   aacctcaaga tgaaatttcc atttgtcttt gcagcgattg cctcaggaat tgcttcagct   1800
   ttcttaggac tattccacgt tctgtccgtg gcgatgggtc ccgcatcagt catcgggttc   1860
   atttcgattg cttcaaagtc gattccagca ttcatgctca gtgctgtcat ctcgttcgtc   1920
   gtcgcattta ttccaacttt tatctatgcc aaacgaacac tcggtgacga tcgtgatcaa   1980
   gtaaaatcac cagcaccaac gagtaccgtc atcaatgtta atgatgagat tatcagtgca   2040
   ccagtaacgg ggactagcga aagtctcaaa caagttaacg accaagtttt ctcagccgaa   2100
   atcatgggta aaggcgccgc aatcgtgcca agcgctgatc                         2140
<210> 2
   <211> 1654
   <212> DNA
   <213> unknown
<220>
   <223> lp_1164
<220>
   <221> startcodon
   <222> (431)..(433)
   <223>
<400> 2
   aatggtgtgt tttggaatcc cgtagctgat ttcagttttt aaattcggtg cggacgtggt      60
   ggcgacgaat tgttgtcgct gccgcatggc atcctttgcg tgagcggctt ccgcgtctag     120
   taattcagat agaaccgggt cgagtgcggg ggtgtggtag gcgctgtcat ccaccacgta     180
   gagcgcagtc aggtttgctt ggtgccgctg ggcaatgttg accgcttcgg taaaagcggc     240
   ttgtgcagca tcggaactgt caacgggtac taaaatattt gtgtaacggc ttggcattat     300
   aaccacctcc agttatcttt acgataatta taatggatta gtgtcgggca ataaaattaa     360
   acgcacctag tgggcaaggc cgatgaacta gcgtatatta tagacagtaa atttcttttg     420
   ggggttaggt atgttaaagc atgtacagaa cagcctattg acctttgaac gggtcgttga     480
   gcggcaacgt tattggcgcg ccttacgtga tacgttgacg ctgctttttc cattcgcatt     540
   agttggggcg tacgttagtt ttatgaacca ggcaatcttt cagcgcaatg gttttttgaa    600
   tcatatttat tatctcagtc actggctgcc ggggtttaaa catttggcaa cttacacgac     660
   gatgcttaat ctgagtatca acggcattct tgccgtgatt gcggcttttt cagccgctaa     720
   ttttgtcgcg cggagtgccc agcgtgataa tctgttagcg ggattgacgg cggcaattag     780
   ttttatcatg cttaatatca attataattt ttttagtacc cagggtcagc ctgctggcag     840
   tcggttttta gaagataatt tgggaactca gggcatcttc ctcgcactat tggtgggcct     900
   gttgactggt tggttgttca gtcatttgac acggtatcca cacatccacc aggagatcga     960
   gacccagacg cacttgctag cgcgggcaca cgcgaactgg ttaccgatag tcgctgcttt    1020
   gctggtgttc agtgccatcg ggtatggtgt tagttttgtc agtaaagggg gattaaacgg    1080
   cctgttttac gcggtcttca ccttaccatt cagtaatccg ggggcgtcgc tactagcgat     1140
   aattggcgtg gcatcgctca gcaatttata ctggttaatc gggattatcg gtccggttga    1200
   ctttagtggt aacagtacta caagtacggt tcaaaatttg gaatatgcgt tgcaacacgg    1260
   ttcggcgtgg ggcgcgccaa atccagtaac gctccatacg ttatttgatg cgtttgcgaa    1320
   cgtaggtggt cccggaatga cgttagcgtt attgattgca attttgtggc gttcacgtaa    1380
   ccgtaattat cgaacggtgg ccaagaccag tgccttgcca gtactgttta atatcaacca    1440
   accgttgctc gtcggcttac cgattgctta tagtccgatt ttagcgattc cgtttctatt    1500
   agcgccactg gctagcatgg tcatcacgtg gtgcgcgttg aagcttcaat ggatgccacc    1560
   ggtcgtttat ccggttggta gaacgatgcc gggcttcttg accggttggc tgggaactgg    1620
   tggcgactgg cgggccttca tcgttagtct gatc                                1654
<210> 3
   <211> 973
   <212> DNA
   <213> unknown
<220>
   <223> lp_2647
<220>
   <221> startcodon
   <222> (509)..(511)
   <223>
<400> 3
   attgcattgt ttggtccatt ggctggtatc ggggacgcga ttacatggtt cacaatctta      60
   ccaattgttg ctggaattac agcgtctttt gctaaacagg gcagtattct tggcccatta     120
   gtttttttcc tagtctattt agcaatgttt gtagcacggg cgccaattgc attattggga     180
   tacaacgccg gcacaaaagc tatttcaaaa attcgcgaaa attcaggaat cgtatcacat     240
   tgtgctagca ttctggggtg cactgttatt gggggattaa ttgctactta tgtacaaatt     300
   gcggttaaaa caaagattcc agtttcagca gggcatacga ttgcaattca aacacaattt     360
   tttgatcgga ttttccctaa tttattacca ttagggtata ccttcttatt gtattggcta     420
   ttaaagaaga aaaatgtgag tcccgtagtc ttaatattag gaacatttat gttggcaatc     480
   cttttctcat ggatgggggt tctttagaat gaagataata gttagtggac acggaaatta     540
   tgcaagtgga ctacaatcaa caattcattt gttagcaggt gatattgcca atatcgagta     600
   tatagatttt actgataaca tggatgatat tcagcttagt gagcgatttc aacgtgctgt     660
   aattggtgag aataatgttg tctttatgtg tgatttacta ggtggtacgc cattcaagga     720
   agctgttaag ttaagtcaag tgagtgaaaa agatattgcg gttacaagtg gttgcaatgt     780
   tggtgcattg cttgaagtgg gttttgagct tacaacatat tcagctccgg ctaaaaaatt     840
   ggctgaaaaa ttagttaaaa tcagccaggt acagactaaa gtttttcatc atcaagctat     900
   tcaagttgat gatggggtag aaggtattta gcgaaatata cagattgtcc gattaacaag     960
   ttatctgttg atc                                                        973
<210> 4
   <211> 1262
   <212> DNA
   <213> unknown
<220>
   <223> lp_3473
<220>
   <221> startcodon
   <222> (1010)..(1012)
   <223>
<400> 4
   tctgtgtttt ctcgaataat tatatcatgc ttttgattgg cgtcatcatt accgggctag      60
   cttcgggtgc tgatttacca attagtttaa ccgtagtttc gcatgatgca ccggatgata     120
   agacctcggc tagtttagta tcgtcgacgc aaattttctg gcaaattggc gttttcatgt     180
   cttatgtttg tgcctttatt gtttctaaaa tggccggtgc ttccggtgct cggattgttt     240
   tttcgatttt agcaattttt gccttgatta cttgggcatg gcggactttt tcaactaagt     300
   tccgagcatt tcatgaagaa ggtgaacaac ggcgtattga tcgcgaagca gcttcacaga     360
   caacagaaaa ggtctcgatt aagacggtct tcgctggtgc caacaaggga cgctttatgg     420
   cattcttctt cagtatttta atcttctatg tttgctggaa tctactggcc aatacgttcg     480
   gacagtttca aacgtttacg ctggttcaag cacatgcgag tcaaagtttc gcaactggtg     540
   ctgggatagt cctaaacttt atctcgttgt tggcaaccat ggcttttgca tcagtcgctg     600
   ggggtaagta tcgtaataag ttcttcttag ttgggattac cgttcaattt ttggcaatgc     660
   tcggactagc gatgagtagt cattcgttat ggccaattgt catcacgatt ggctgttaca     720
   acattggaaa taatattgct ggtgaagcga tgtataaagt ttggacacag gaagcatttc     780
   cagttgaagt tcgagcatca gttcaaggat ttatcaatgg cttttcacgg gtatgttgtg     840
   gcctatttgc cttaattaca cctgcgttag ttgtgccatc agcaattcgt aacacgatgt     900
   ttgggttcgc tgccattgtc tttattagct ttgtagcagg gctgacgatg atgcgattgg     960
   aaaagaagca tggtattaat aacttttaaa caaagtattt agaaagcgag tggaagagat    1020
   ggcgtttact tttcaaatca ataatgatgt tcaatttcag cataatcaag cattattaga    1080
   caagtcggca agctatcggc caatactgaa agaaaccaaa gttaaggcgg catcgattgt    1140
   ggcgctcgag cgtgatacgc aataccttga aggatggggt gttaaacaaa ttgcacctat    1200
   tgaacgatta tcaagttatg agttgaaacg tgatgaccaa attatcatag attttggcga    1260
   tc                                                                   1262
<210> 5
   <211> 798
   <212> DNA
   <213> unknown
<220>
   <223> lp_3522
<220>
   <221> startcodon
   <222> (519)..(521)
   <223>
<400> 5
   acgtcccatg atactgttgt tggggttgca gcaagcggtc gaacacccta cgtgattggt     60
   ggattagatt atgcatgcga agtaggtgca acaactgtaa gtattgcttg taacgctgat    120
   gctagtatta gtcagcatgc tcaaattccg attgaagttg aagttggacc ggaaatatta    180
   acaggctcaa cgcggttgaa atcaggtacg gcacaaaaat tagttttgaa catgttatcg    240
   actgcatcaa tggtcggaat cggtaaggtc tataagaact tgatggttga cgtcaaacca    300
   acgaatgaaa agttggtaga acgtgctaaa agaattatcg ttcaagctac ggactgttcg    360
   gatgagacag cggtgaaagt ctttatgact gctgatcaga acgttaagtt ggccattgta    420
   atggttttga ccaatatgag caaagctgaa gctagtgttc gattagatca tgccaacggt    480
   tttgtaagac aagcagttaa ttaaaggagg agtttcatat ggctgaagat aagtatactc    540
   ggattgcaaa tgctatctat gcgcatgtag gtgggccaca gaatattgag aagttgattc    600
   attgcatgac acgtgttcgg atgacaattc gcgattactc attagttgat atggatggat    660
   taaaggctat tgatggcgtg atgggagttg tccaagaaga tactttacag gttattgttg    720
   gccctggaac tgttgataag gttgcagacg taatggttaa gcaagttggg gttggacttg    780
   gggagccatt tcctgatc                                                  798
<210> 6
   <211> 754
   <212> DNA
   <213> unknown
<220>
   <223> lp_3526
<220>
   <221> startcodon
   <222> (553)..(555)
   <223>
<400> 6
   tggccggcgg tgtttcagta gaaacgacac caactggtgc agcaactaaa gtagaaccaa     60
   acgtcactta caatgcggcc actaagttaa gtagccccga aacgggccaa atcatcccac    120
   taagtgaagt taaggatgag gtcttctcat caggtgcaat gggcaaaggt gttgcaatcg    180
   aaccaactga tggtgtctta tacgctccag ctgacggcga agtagcactc gtgttcccaa    240
   ctggacatgc cgttggttta aacacaacag atggtgctga aatcttgatg catattggaa    300
   tggatactgt cgaattagag ggcaaaggtt ttgaaacgct agttcaaaag ggcgagacgg    360
   tcaaagctgg tcagccgcta gtgaagtttg atattgaagc gattaaggcg gcagggtatc    420
   ccgttacaac gccaatggtt gtgactaatt ctaagaacta tcatgacgtt aaactggctt    480
   ttccagctgc tacggaagtt aatgcaactg aaccattatt acaattaaac taattaaagg    540
   gagttttcaa ttatgtattc aaaaacaatg ccaactggct ttccaaagaa ctttttgtgg    600
   ggcggcgcga ctgctgccaa ccaagttgaa gggggttgga acgttgatgg gaagggccta    660
   acaactgctg aagttgttca gaaggctact gaccgcaaag taatgtcgat gaacgaggtt    720
   actaaagaga gtgtccaagc tgccattgat gatc                                754
<210> 7
   <211> 983
   <212> DNA
   <213> unknown
<220>
   <223> lp_3617 and lp_3618
<220>
   <221> startcodon
   <222> (756)..(758)
   <223> lp_3617 start codon
<220>
   <221> startcodon
   <222> (356)..(358)
   <223> lp_3618 start codon
<400> 7
   aaatatctgg ggcttgctaa tagtgggttt tatctgttca ttgccgtttt tatcaccatt 60
   attaggtccc ggggcagtaa ttggtcaaat tattggaacg ttgattggtg tcgaaattgg 120
   taagggaaat atagcaccac aatatgcgct accggccttg tttgcgatta acactcaaaa 180
   tgcttgtgat tttatcccag ttggattggg tctggaagag gccgaggcaa aaacggttga 240
   ggttggtgtt ccatcagtat tgtattcacg cttcctaaac ggtgttccgc gagtgtttgt 300
   tgcgtggatt gctagctttg ggttgtattc aaaataagaa tggggtgacg cacatatgaa 360
   gactaatgaa gcaattatgt atcagactaa tgttaaggaa attggtcaag atgcgcagga 420
   tttttcggaa atcaaaatgg cgatcttttt cggtgatact gcacctgatg cattgcgatc 480
   gtcatgttac ctgattgagg tgtatccggt taatggggtg attaaaccag gaatgagtct 540
   ggttgttgat aatgaagact ttgaaattac agcagtaggg cgcgaggtgc aacgcaactt 600
   ggttaatcta gggcatatgg cagttagttt tactggtaaa cagcaagctg aattggcagg 660
   aactatgtat gtcgcggata aagaattgcc tataattagt attggaagta cgatcacgat 720
   taaagaataa tccacattca aggagcgtaa taaatatgga actcttatta gatacggtta 780
   acctagatac tattaagaag tatgctgatg ttatttcctt agctggtgta acttcgaatc 840
   catcaattgt gaaaaaagaa cggcccattg atttttataa gcatatgcgt gaggttcggc 900
   aaattatagg caaggaacga acactccatg tacaagtggt tgggcagact gctgaggcga 960
   tgcttgcgga tgcacatatg atc 983
<210> 8
   <211> 1616
   <212> DNA
   <213> unknown
<220>
   <223> lp_3659 and lp_3660
<220>
   <221> startcodon
   <222> (1276)..(1278)
   <223> lp_3659 start codon
<220>
   <221> startcodon
   <222> (353)..(355)
   <223> lp_3660 start codon
<400> 8
   gtatacttag gcatggctga acatgataag cggataccgg atgattacag tgtcattggc      60
   tatgacgatg tcgaaatgtg ccaatatgtc gcgccacagc ttacaaccat cgcacaaccg     120
   atttttgagt tggggcaaac gacagccaac cagttactcg accgtattgc taatccgcag     180
   cagccatggg aagaacacca cttaccagtt aaattaattg aaagaaattc gacggcacat     240
   ttgaattaat tattcagatg tgttatattt tttacgagtt attaaaacgt tttcataaaa     300
   cgttttaata aaaactaaat tcagattaat ttaactattt ggagggacca ttatgaatac     360
   ggtaaccgtt attgggagta tcaacttaga ccggacgatt cgggttgaaa acatgccaaa     420
   gcctggcgaa acgattcaca ctaaggaaat cttttcagcc ggtggtggta agggcgcgaa     480
   ccaggccgtc gcagcgcaac gttctggtgc taatacccac tttattggtg cggttggtga     540
   cgatgctgct ggtaaaacca tgcttgattt attgacgcag gaaaagatca acttggcagg     600
   gatcacaaag atgactaatc aatcgacagg tcaagcttac gtcacggttg atgatgccgg     660
   tgaaaaccaa attatgattc atggtggtgc caacatggcc tttacgccag cagacgttga     720
   agcccaccgt gatatcattg aaactagtga ttttgtggtt gcacaatttg aaagcgctgt     780
   cgatagtacg gtcgaagcct ttaaaattgc acaggccgct ggcgtaagaa caatcttgaa     840
   tcccgctcca gccatggaaa aagtccccgc tgaactttta gcagttactg acatgattgt     900
   accaaatgag acggaaacgg aaactttgac tgggattgca atcaccgatg aagtcagcat     960
   gttaaaggcg tccgctgcat tacacgccct cggtatttca gctgttatca ttacgattgg    1020
   tagtaagggt gcgttttatg atatcgatgg gcgacatggt atcgtaccag cttttaaagt    1080
   cgaagccgtg gatacgactt cagccggaga tacgtttatt ggcgcgatga gtagtgtgtt    1140
   aaataaagat tttagtaatc ttgaagatgc gattcgctat ggtaaccggg cgtcatcgat    1200
   tgccgtacaa cgtttcggtg cgcaaccatc aattccttat aaaaatgaaa ttacagccgc    1260
   ggagggtaaa taattatgaa aaaaacaaaa gttattaatt cagatttgtc acgagttatt    1320
   gcaacgatgg gccatttcga taagttgagt attggtgatg ctggcatgcc agtaccaagt    1380
   tcaaccgaaa aaatcgattt agccgttgat aatggtattc ctagttttat gcaagtcttg    1440
   aataacgtct tggaagagct tgaagtacaa cgaatctatt tggcagaaga aattaaagtt    1500
   caaaatccta agatgttgac ggccatcaag aagcgcttac cagacacccc aattaccttc    1560
   attccacatg aagaaatgaa gaaggacttg gctgactgca aggcctttgt tcggac         1616
<210> 9
   <211> 1209
   <212> DNA
   <213> unknown
<220>
   <223> lp_0017
<220>
   <221> startcodon
   <222> (68)..(70)
   <223>
<400> 9
   tatttttcaa attttagcag gcgaaccagt cggaacgcta ttcggttaag ttggggaggt      60
   tatcaaaatg gtagatttag aacagttggg tcagcaggct caacaagcta gctatacgct     120
   ggggttactt tcaacggcgc aaaaaaatca agtgttgacc gcaatggcaa cggcgttaac     180
   gagccatcaa gatgagatat tggcagctaa tgcgcaggat ttagagaacc cgcaagtacc     240
   agttaagttt gttgaccggc tccggctaac agcagaccgc atccaggata tggcgatcgg     300
   gctcaaacag gtcgtgagtt tgccagatcc aattggaaat gtggaccgcg cgtggcgcaa     360
   cgaggccggg ttgatgattg ccaaggaacg ggtaccatta ggtgttatcg gcatgatttt     420
   tgaagcgcgc cccaatgtga cggtggatgc atcagcactg tgttttaaaa ccggaaacgc     480
   tgtaatctta cgcggtggta aggaagccct tcactctaat caagcacttg tccaagtctt     540
   acgtggtgca ttacgggccg aagcggtcga tgagaatgcg attcaactga ttacggatac     600
   gtcgcatgcg actgcggcta agttcatgca gctcactgat tacgtggacg tcttaattcc     660
   acgcgggagc gcccgtttga ttcaaacggt gttggctaaa gcaacggtac ccgttattga     720
   aacgggagcc ggtaattgtc acgtatacgt ggacaaagac gcgcaattgc aaatggcaac     780
   cgatatcgtg atcaatggga aagttcagcg gccatcagtt tgcaacgcca ctgaaaagct     840
   tctaattcat cgtgaggttg cggccgatta tttacctagc atgattcagg cacttcgtga     900
   acaaggcgta gaagtacgcg gggatgccgc aactcaagcc attgttcctg atgtggttcc     960
   agcaacggat gcggactggg gaacggaata taatgattta attattgcag tgaaagttgt    1020
   tgattcagaa gcagcggcca ttacgcacat caatcgttac aatacccaac attccgaagc    1080
   tattgtgacg gataattatc aagctgggaa gctcttccag caacgggtca acgcagcgtg    1140
   tgtttatatc aatgcttcca cccggtttac ggatggcttt gaatttgggt ttggcgctga    1200
   aatcgggat                                                            1209
<210> 10
   <211> 1147
   <212> DNA
   <213> unknown
<220>
   <223> lp_0228
<220>
   <221> startcodon
   <222> (916)..(918)
   <223>
<400> 10
   atccagtcaa caccggcagc catagcagca cttgcaccag caaccaatgg gccgattgca    60
   aagtaaccca ggattgctag taacatcccg ataatcccag cggagaagtt gttaaccaac   120
   atttcaaacc cgctcttaat cttatcggaa atggcgtcat cccacttctt aatgcaccaa   180
   ccgcctaaag gccccataat catggcaccg ataaacattg ggattgaaga accaacgatg   240
   acccccatag tggcaatcgc accaacgacc ccaccacgat gaccggcaaa caaagtccca   300
   ccagtaaacg ctatcaataa tggcaataaa taatggacca ttggttgaat catcttagct   360
   aatccagcat taggccacca accaccagcc ataaagatgg cggtaattaa gccccaagca   420
   atgaaggcgc cgatgttcgg cataaccata ttacttagtg cactcccaaa tttttgaacg   480
   cgtgccttaa cgcccagttt ctttttcgca acaggagcgc tttgggttgc attcgtattt   540
   acattttcac tcattaggta aagctccttc ctttttctta acaacttaat catatccatc   600
   gtggtaagcg gtttcaagaa cttctagtta gacccgtggc acaggaaact gtgccacaat   660
   taaactcact aattcacaaa gttaaaattt ggcacagtat cgataaaaac ttttatatcg   720
   gcaataccag cagactaatt aaactttttt cgtcagatat gatttaattt tcacaatcaa   780
   attcaacatt ttatgcagaa aacacctggc aattctctca tcattttatt atttaaatga   840
   ttacgaatac gatttaacgt tgaaatcccc ccgcaagctc ctatacttat aattaactaa   900
   ggagtgacct atgtaatgaa aaaagacact gattgtaccg aaatattggt tgggaaaact   960
   gctagcatgg atggttcgac catcgttgcc cgcaacgaag atggctacgg cccaattaat  1020
   ccgattaagt ttattgtgca cgaagcccac gaccaaacta acgccagcta cacttccgta  1080
   actaccggcg tcaccgttcc gctgcctgac catgcctacc gctacaccgc aaccccccaa  1140
   gccgatc                                                            1147
<210> 11
   <211> 512
   <212> DNA
   <213> unknown
<220>
   <223> lp_0696
<220>
   <221> startcodon
   <222> (417)..(419)
   <223>
<400> 11
   ttgggaaccg gatatggtcc cattgggatg gcgctcgcgt atcaatcacc cgaacgaacg   60
   gttgatatgg tcgatgtgaa cgagttagcg ctaagcttag cgcgtaaaaa tgttgctttg  120
   aaccaaatta cgaataccaa tatttttacg tctgatgtct atcaacaagt gaccgcgact  180
   aattacgcgg cgattgtcac caacccaccg gtgcgcgcag gtaaggcagt ggtggatgcg  240
   atgttaacgg gggccgtttc acggctagtc gttggtggaa cactcaccgt cgttttacag  300
   aaaaagcagg gcgcaccatc tgcgaagaaa ttgatgcaaa cgacctttgg taattgtcag  360
   gtgattaaga aggacaaagg gtattacatc ctacaaagtg taaaggaggg atagtcgtga  420
   cggaaccaac agcggaagac tggatgcaag aagcattaca tgaagcgcgg atggcctact  480
   taattggcga agtgccgatt ggggccgtga tc                                512
<210> 12
   <211> 1445
   <212> DNA
   <213> unknown
<220> .
   <223> lp_0775
<220>
   <221> startcodon
   <222> (707)..(709)
   <223>
<400> 12
   gatttgcggt tagttcaacc taacaagacc gctattgata cggcgggctt acacacgatt   60
   gaacatatgt tggctggatt attacgtgat cggatggatg gcgtgattga ctgctcacca  120
   tttggttgtc ggactggttt tcatttgatt acttggggtg aacatgacac cgttgaagtt  180
   gctaaggcat tgaagtcctc attagaattc attgctggcc cagctaagtg ggaagacgta  240
   caagggacga ccatcgatag ctgtgggaat tacaaggatc attctttatt ctcagctaag  300
   gaatgggcca agttgatttt atcgcaagga atttcatcgg atccatttgt acgcaaagtc  360
   gttgaataga ccaaaatata aaaagtaatg gtggtgccgg ccaccaatta aaaagagcct 420
   agcttttcat aacgaaaagc taggctcctt tttgctgttt actgacattg tgcgagctta 480
   gcggtcctga atcgtgggaa aacaatctaa gattagatta aggtagtgtt tcaaaaataa 540
   caaaaaatat actgatattg gtgaatatct gcataaaaat tctatatttt agtgaatatt 600
   gtgcaaatga tgaatatatt taaaatatta ttgaataaag ttaaattggg tggtacgata 660
   ctgtcattga taaaagcaaa gataaaaatt tcggaggtac aaatcaatgg tcaaacaaaa 720
   tgataaaatt attctcgctt attctggtgg tttagatacg tcggtcgcaa ttagttggtt 780
   gaaggataag ggctatgatg ttgtggcctg cggaattgat gttggtgaag gcaaggacat 840
   ggatgcaatc aaagaaaagg ccctaaaatt aggggctgtt tcatcatata tgattgatgc 900
   taagcaagaa tttgctgaag aatatgcgtt aattgcttta caaggtcata ccttgtatga 960
   aggtgaatac ccgctggtct cagcactttc acgtccttta attgctaaga agttagtcac 1020
   attagccaag caagagcacg ctgttgcaat tgcccatggt tgtactggga agggtaatga 1080
   tcaggttcgg tttgaggttg ctatccatgc tcttgcaccg gatatcaaga tcgaagcgcc 1140
   tgtacgtgat tggcactggt cacgggaaga agaaatcgat tacgctaagg aacacaatat 1200
   tcccgtacca atcaatttgg acagccctta ctcgattgac gaaaacttat ggggccgggc 1260
   caatgaatgt ggcattctag aagatccatg gcagggcgcg cctgccgatg cgtttgaccg 1320
   gacgaaggcc ttagctgata cgccggatac gccaacgact ttggaaatca cttttgaagc 1380
   tggggtgcca gttgctttag atggtgaatc acttaaccta gctgatttaa tcatcaagtt 1440
   ggatc 1445
<210> 13
   <211> 1464
   <212> DNA
   <213> unknown
<220>
   <223> lp_0854
<220>
   <221> startcodon
   <222> (643)..(645)
   <223>
<400> 13
   cctaattggt cgtacattga aacttggacg cctaaatctg ccagttcctc accaaagttt 60
   tcccagtatt cgtgattgcc accagggcca ccgtgtagac ataataattg aatgtcgcct 120
   ttgccctgag tattcgtcca taaatggtag ccattatcca gcgtgatgat tgtcgttcct    180
   tgtttcaaat gatcacctaa tttcttcata ttttaattgt tatcttacca ctgttaacta    240
   agcagggtca aagagactag gcaatcataa agaattgcat taagtagata attggtaagg    300
   tcgtgttcgc taattaactg aatcagtacg gtcgcgtcat agtagctgag catgggaggg    360
   gaattcggtc gaaacgatat aaaaataata attagaacgg gccgtttagt ctgaatttgg    420
   tttatactag ttattatgct ggtcggtacg actgatagac caagtttcag tcgtattgtg    480
   accgattgta cacaattcat ttgacaagcg gttacatttt cagcgataat aagtaaactg    540
   ctcagcacag aaagatgtaa tattacattc tttaaattca attgcggtta gttaaccaat    600
   atgaaataat gggttaacta attagagatg ggtgagattg atatggcaaa ccaagtcgca    660
   gttttacgct tgttaatcca agcggcgccc gcgtactgtt cgggtgactg gttggcacaa    720
   cgacttgcaa tcagtcgaac ggctatttgg aaattagtgc aaggattgca agcggcgggc    780
   catactattg agagccggca tgggcgaggt tatcggtatg tggccaataa tcaattgagt    840
   gcgccggtga ttgaatacgc gcttacagcg ttaccaccgt ttgatttgac cgtgctacca    900
   acggttgatt caacgaatgc ctatttacgg cggttagtca cgcagacgcc tttagcactt    960
   ccgaaaatcg tggttgcgga tacgcaaact gctggttacg gtcggtcggg gcgggcattt   1020
   tattcgccag cacagactgg aatttacatg agcattgggt taccactcag tgaccagaag   1080
   gcgttagacg ctggtttgtt gacaacgagt actgcggtag tcgttgccca gacgctgcaa   1140
   cagctgtttg cggttgacgt ttcattaaag tgggtcaatg atgtgttagt caatcatcac   1200
   aaggtggttg gtatcttatc agaaggcatc actagtcttg aaacggggca aatttcagca   1260
   gtcattattg gaattggcat taacttaaca acgacagctt ttccagcgct actagcgcac   1320
   aaagcgggcg cagtcgttgc caatccaaat ggtgtgacgc gcaatcaagt gatttgtgag   1380
   ctactgcggc gctttttcaa tagctatggg acttatcgta gtgggacttt catggacgct   1440
   tatcggaaac tctccatggt gatc                                          1464
<210> 14
   <211> 1270
   <212> DNA
   <213> unknown
<220>
   <223> lp_1058
<220>
   <221> startcodon
   <222> (673)..(675)
   <223>
<400> 14
   atgtttgtaa cgtgggtcga acaagctaat gaccgaattc caattcaata cactcgtcgt 60
   gcagctggtg cacctgacag tagttaccta ccattaaagg tgaacgttgc cggggttatt 120
   cctgttattt tcgcgagttc cttcattgca acgccccaaa cgattttgct agggttccaa 180
   tccagtcatg gtgacgaaac ctggtatacc gttttgagta atatcttcaa tatgcagtcg 240
   acgactggtg ctattcttta caccattctg atcgtgctct tcacgttctt ctatgcgttt 300
   gttcaggtca atcctgaaaa gctctctaag aacttacaaa agcaaggcag ctatattcct 360
   ggtgtttggc caggtaagga tactcaagat tgggtttcaa aattgttaat gcgactaagt 420
   acggtcggtg ccctctacct tgggttgatt tccttgattc cattattagc ttctgatatt 480
   tggggtctcg atgaatcaat cggtttaggt gggacgagtt tactaatcgt tgttggggtt 540
   gcccttgaaa caattcgtca aattaaagga ttgatgatga aacgtgacta tgtcggtttc 600
   attcgttaat cagcgaatgt gttggggaga ctcaacacat ttgtgcattc aggaggaaaa 660
   aacatgtcaa caatgaacct gattttaatg ggattaccgg gtgccggtaa gggtactcaa 720
   gcacagaaga ttcttgaaga ttttgatatt ccacatatct cgaccggaga tatcttccgt 780
   gcagccatta agaacgaaac gaagatgggc ttggaagcca agaagtatat tgatgcaggt 840
   aacttagtac cagacgaagt cactaacggc atcgtccgtg atcggttagc tgaagcggat 900
   actaaaaatg gtttcctatt agatggttac ccacgtaaca tcgatcaagc tcatgctttg 960
   aaacaaatcg gtgaagaact caataagcca cttgatgggg tcatcaacat tcatgttgaa 1020
   cccgcagtac ttgttgaacg actttccggt cggtttatct gccggacttg cggtgcaact 1080
   taccataagt tgtacaacaa gcctaaggta gaaggaacct gtgatgtgtg tggcggtcat 1140
   gatttctatc aacgtgatga tgacaagcca gcaaccgtta agaatcggtt agacgtgaac 1200
   attaagttga acacgccatt aattgattat tacggtcaag aaaagttact ctacaatgtc 1260
   gacggtgatc 1270
<210> 15
   <211> 676
   <212> DNA
   <213> unknown
<220>
   <223> lp_1319
<220>
   <221> startcodon
   <222> (391)..(393)
   <223>
<400> 15
   aaatcgtgat tcagttacca tgtatctatt tcctatcaac ggcggggccc ctggttgcga 60
   ccgcgattgg gatgatggtt gcgagtctga tggcgatgta tgatttggaa gtcaactttg 120
   gaattcggta cgtgaagtta ttaccaaaaa tcaatcgaat tttagtttat tcgattttaa 180
   cgtacgtcac ggcacgatta gttgtttatg ggctgaactt tgtgttgaat gagcacagta 240
   agacgcaatc gttcttgatc gtggccctag cagttatcgc tggtggtgcg gtttacgttt 300
   acttagcact ccgttcacgg atggctgatt tgatgattgg aacgaaagct gctgggttac 360
   gacggaaatt acggattaaa taggtgaatt atgcgagtag ataaattttt acatgcgatg 420
   cgaattggca cgcggactca agtacgtcgt ctgattaaag atggtcatat cactgccaat 480
   ggtgagccag taatctctgg caaacaacag attaaccctg gcagtgatac tgtcttttta 540
   gatgacgtgc aggtccgtta ccaacaatac ttctattatg tgatgaataa gccagtcggg 600
   gtcatcacgg caacgacgga ttccgctgcg aaaacggtga tggacctgtt caatacgacg 660
   gattttcgtg acgatc 676
<210> 16
   <211> 1393
   <212> DNA
   <213> unknown
<220>
   <223> lp_1602 and lp_1603
<220>
   <221> startcodon
   <222> (116)..(118)
   <223> lp_1602 start codon
<220>
   <221> startcodon
   <222> (1021)..(1023)
   <223> lp_1603 start codon
<400> 16
   aagggcgtgg cacttagcaa gcagttgcaa gcaacccttg aaggtgctga aaagacgctc 60
   actaagatga tgaatgaaaa tggcgacgaa gttccatttg agcaagctga cgccaatgaa     120
   taagcaatta ttaactgaat ttgaatccaa gtggcgtcct cagattaatc agtatttaga     180
   tgagcagctt caagcgtgtt ctgaccaatc aaccctgact gacgccatgc gttattcggt     240
   actagcaggt ggcaaacggt tacggccgtt attgacgtta gccattttag atacttttga     300
   tatcacaacg acggctgcaa acttgcgggc aagtgtggcc gttgagttga tgcatactta     360
   ttcgctaatt cacgatgatt taccggcgat ggataatgat cagctacgac gtggcgaacc     420
   gactaaccat gttaagtttg gggaagacgt tgccattctt gcaggggatg ctttacaacc     480
   gttgactttc gaatggattg cggatagtgg gttaccggca tcgatcgtcg ctaatcaaac     540
   cttagcatta gcgcaggcca ccggacctcg cgggatggtg gctggtcaag ttgctgatgt     600
   ccttggagcg ggacaacatc tggctttgcc agccttacaa cagctgcatc gcgagaagac     660
   gggggcgtta attcactacg ctgtccaggc agggttgatt caagctcaag tgcaaccaac     720
   cgtgcaggaa ttgctattac aatatgctga tgcctatgga ttggcgtttc aaatttacga     780
   tgatatttta gacgtgacga gtacgcctgc tcagttagga aaagctacgc ataaggatgc     840
   cgatgagcat aagaatacgt atccaggttt gctggggctt gcaggcgcac gaacagcgtt     900
   agaacaagcg gtaacagctg ctcaaacggc gttagtaaaa gctagtgctg ctagtcaacg     960
   aggcatgggc ttgcttgcag cttttctaac gtattttaca gattaaggac ttaaatttag    1020
   atggaaaaag aacgagttga cgtattatta gtacaacaag gattatttga tacccgcgaa    1080
   aaggctaagc gtgccgtaat ggccggtgaa atcttaggtg tcaacgaaga acgcttagat    1140
   aagccgggca tgaagatacc agtggcaact gaattacatt tgaaaggcaa accgatgccc    1200
   tacgtttcac gtggcggtct caaacttgaa aaagcactca aaagttttga aattgacgtg    1260
   accgacaaaa cggtcctaga tattggctcg tcgactggtg gttttaccga cgtggtttta    1320
   caaaatgggg caaaaatgag ttacgccttg gatgtcggta gtaatcagtt ggtctggaag    1380
   ttacgccaag atc                                                       1393
<210> 17
   <211> 887
   <212> DNA
   <213> unknown
<220>
   <223> lp_2031
<220>
   <221> startcodon
   <222> (128)..(130)
   <223>
<400> 17
   tccagatgcg aacacgatgg ttgcgttagt ttaccaaaac agcctaaaat gtctatatag 60
   ttatcgaccg gacgaacagc ggtacaagcc gttcaaaatg tttgcggtca attaaggagt 120
   ttttgtcatg caggtgatta atttaaagta tcccgtggcg ttgagtcaga ttcccgaagg 180
   accaatcgta ttggccttag gattttttga cggcgttcac cgtggacatc agcaagtcgt 240
   tgcaacggca cgccaggccg ctcaggcgca acacgccaaa ctagcggtga tgacgtttga 300
   ccagcatccg tcagttgttt ttaaacacac tgatccgcag caagtccgtt atttaacgac 360
   aattgatcaa aaaacggcat tgatgagtga gctcggcgtt gacatcttat atgtacttca 420
   ctttgatgcc acggtcggtg ccatgccacc ccaaacgttt gtcgatcagt taattgttgg 480
   attacacgca caaacggtgg ttgctggttt tgattacacg tacgggccag ctgaaattgc 540
   aaatatgcag cgtttgaatg actatggtca taaccggttt gaaatcattg aagttcctaa 600
   ggcggaatta gatgctgaaa agatcagttc cacgcggatt cgccgcgctt tggacgcggg 660
   agatatcgat acggctaacc ggttactggg ctatcaatat gaaactgccg gcgaagtagt 720
   tcatggtgaa gcgcggggcc gaacgctcgg ctttccaacg gccaacgttg ctcacgggcc 780
   caatacccgg gttcccggaa ttggcattta cgcgacgatg gtgcagattg ggacacgctg 840
   ggttatggga atggcctcag ttggacgaaa tgtcacgttt ggtgatc 887
<210> 18
   <211> 1038
   <212> DNA
   <213> unknown
<220>
   <223> lp_1019
<220>
   <221> startcodon
   <222> (187)..(189)
   <223>
<400> 18
   gtcgtccaaa gtttatacga ggacgacgtc ttaaatcgtc gggaagcgca attgatttta 60
   gtggcgattg accacgagac gttaggatta acggaccggg acttagaaaa cagtctgcgg 120
   gcccggataa ttatcggaat cttgaaccac ttgcgttacg aaagctaaag aaagcgagga 180
   atcgcaatgg ataacttatt tacacctagt gccaaaagtg tgctggtact ggcacaggaa 240
   caggccaaat atttcaagca tcaggcagtt ggtacggagc atttgctatt agcattggcc     300
   atcgaaaaga atggaattgc taataaggtg ctacaacaat atgccgttag tgaagatgac     360
   attcgtgaag aaatcgaacg gttcactggc tatgggacgt tgagcaatgt tggcaaggat     420
   acctacttgc catattcacc aaaggccaaa gaaattttgt ccgttgccgg tgatgaagca     480
   aaacggctcg gtgccaacaa gattggaacg gaacatttgt tgttagcgat gctctctgat     540
   gaaagtatcc tttcctcgcg aattttaatg aatttaaact tagatctagg tcagacgcgt     600
   aaagtcgttc ttcgcaagct aggtgtcagt gacgccatga gtaagcgcaa gggtaacgcg     660
   gctaatcgcg gtggcaagaa gactgaagga acgccaacct tggattctct ggcgcgcgat     720
   ttgacccaat tagccagtga acaggaaatg gacccggtag taggtcgttc caaggaagtc     780
   aaacgggtca ttcagatttt gtcacggcgg acgaagaata atccggtctt gattggtgaa     840
   ccaggggttg ggaagactgc aatcgccgaa ggtctagccc aaaagatcgt ggccggtgat     900
   gtgccaactg atatggcaga taagcgcttg atgatgctcg atatgggatc attagtcgct     960
   gggacaaagt atcgtggtga attcgaagac cgcctgaaaa aagtgattga tgaaatttac    1020
   aacgatggcc atgtgatc                                                  1038
<210> 19
   <211> 1912
   <212> DNA
   <213> unknown
<220>
   <223> lp_3055, lp_3057 and lp_3058
<220>
   <221> startcodon
   <222> (983)..(985)
   <223> lp_3055 start codon
<220>
   <221> startcodon
   <222> (714)..(716)
   <223> lp_3057 start codon
<220>
   <221> startcodon
   <222> (324)..(326)
   <223> lp_3058 start codon
<400> 19
   agttttgaat gattaatata ggcaatcact gacgtttctt attaaacggt agtgattgcc 60
   ttttgtgata tcgtcgttgg tacttttgat gcggtcagtt gaccggtcga aatggcgact 120
   aataactgag caaaggaacg tacgcggcac tttttgtagt gacactaaat gtcagacata 180
   tgatagtagc aactgggtgg ggcgtttaaa aaattaaggg ttatgcctaa aaacaacatt 240
   tatcgttgac aattgtaaac gaaaagatta atatacacat atagattaca agtgtaaacg 300
   ataatgagaa agggacgaat aaaatgatga ttaaagtatt agtcttatta attggtgggt 360
   tagcaattgg gtttattctg tggtggttct tcgggaagca tgctacgaca acggtcaccg 420
   cgagtgcggt tgatgattta cagcaggttg atgtggaggt taagggtggc tattcgccag 480
   agcaaatcgt gttgaaaaaa ggggttccgg cggtacttaa ttttacgcgc aaagatgcat 540
   ctggttgtct ggatcacgtg gtgttcagtg atttcggtat caatcaggaa ttgccacaaa 600
   atcaagttga agcaattacg attgatacga gtaagtctgg cgaatatcaa tgggcctgtg 660
   ggatggacat gtttcatgga aaattaatca ttaaatagtg ggaggaaaat caaatgagcg 720
   cacgacaaca aatcaatatt acagtagacg gccattatga cccagcagaa gtaacgttac 780
   aacaaggtgt accggcccaa ttaacgttta cgcgcacgag tacgcagggg tgcttagagc 840
   aagtacattc aaccgacttg cactttacga ctgagttacc ccttgaccaa ccgcaaacgg 900
   tgacggtacc aactgatcga gccggtgaat tttactttag ttgtgggatg gacatggtga 960
   gtggcaaggt ggtgattgcg taatgaacac gacgaaacgg ttttggattt cattaatatt 1020
   ttccctgcca ctactggcta atatgatttt gatgcccttt ggctggatgc tcccgggtgg 1080
   tgattggacg cagttgattc tgacgaccat cattatggct gtttcggctc ggccgttctt 1140
   gcaaagtgcg tgggcgtcct ttactaagca ccatgtcaat atggatactt tggtggcgat 1200
   tggcactgcg acggcttatt tttacagctt atacgcaatg gcaacagggc aagcagtctt 1260
   ctttgaaagt gccgcacttg tgaccacttt cgttttgctc ggccaagtct ttgaagaacg 1320
   aatgcggcac aacgcgtcca gcgcggtcgc aaagctagtg ggcttacaag ccaaggaagc 1380
   ggaagtgtta cgcgatcaag aatttgtaaa agtgccgttg gatcaagtag tggtcggcga 1440
   cattattcgg gtcaagccag ggaaaaaaat tcccgttgat ggtgtaatta caactggccg 1500
   ctcaacggtt gatgagtcaa tggtgacggg agaaagtatg ccggttcaaa aaacggtggc 1560
   ggacgctgtc attggttcaa cgattaatag taatgggacc tttcagttca aagctagcaa 1620
   ggtcggtgac cagacaatgc tggctcaaat cgttgagctc gttaaaaaag cacaaaacag 1680
   tcacgcaccg attcaaaagc tgaccgataa agtttctgat atctttgtgc cgattgtgct 1740
   aattattgct atcgttacct tcttgacctg gtatgttttc ttgaatgcta gcctcgcgaa 1800
   tgccttgatt tttgcggtcg ctgtggtcgt gattgcctgc ccgtgtgcgc taggtttagc 1860
   tacgccgacc gcactaatgg tcggtaccgg ccgtggtgcc aagatgggga tc 1912
<210> 20
   <211> 651
   <212> DNA
   <213> unknown
<220>
   <223> lp_3288
<220>
   <221> startcodon
   <222> (484)..(486)
   <223>
<400> 20
   ctaattatcg gcttcttcag cgtgagctgc gacctgctga aaaatactaa tgatatgttt      60
   atcggccagc tggtagtgca cgttacgccc attgcgcgtt cccacaacta attttaattg     120
   tttgaggacc gttaactgat gagacacatt cgactgtgtc agggctaatc ccgtcgccag     180
   ttcacccaca ctcagcttag atacggccaa cgcgtagata atccgggcac gcgtcggatc     240
   accaagtgcc ttaaagattg cggtgatccg actcaactca tcagtcggcg gcatcagttc     300
   agccaaccgt ttgacgatca tttcatggtc catttcattt tccatcataa actccaattt     360
   ctaattgaga ttgtcatgta agcgtgttac aatacacatg aacagtcatt catatgtttg     420
   ttcattcata tttaaattat agccataaca aggataaatt caactaagaa gtaggtgatt     480
   catatggcac acagccatca aatacaacaa cagaccagcg ccttcaagat tggggtcgcc     540
   ctaaatactg ccttcattgt cttggaagct ggctacggtt ttgcgagcgg ctcactggcc     600
   ctagttgctg atgcaggaca caatctcagc gacgtcctgg gcttactgat c              651
<210> 21
   <211> 2053
   <212> DNA
   <213> unknown
<220>
   <223> lp_3303 and lp_3305
<220>
   <221> startcodon
   <222> (1022)..(1024)
   <223> lp_3303 start codon
<220>
   <221> startcodon
   <222> (394)..(396)
   <223> lp_3305 start codon
<400> 21
   ctcattgtac tgaataagtt tccgttgagt tgaatcgtta gcgattgcgc tcaatgctca      60
   tatttggggt agctggcagt gatgggggag ttgtcaaaac gagagacagt agcgctcgga     120
   ttttgagagt caggcgtaaa tggcgtacgt ttggttggtt agaaaaatcg aatgctagtt     180
   atcaatcgat aaggtgattc agctcagtct aaggtactag gtattggttt acgaacttgg     240
   ccattactaa ccgctaatac gggctcgtgg gcctaatgtc atttaatttg gacagtatcc     300
   gtcataacca tgtaaaaatc atgtaaattc gggagcaata attgttattc tagcagttac     360
   gtgctatgtt attatcatat tttatgaagt ggagtgatag ttatggctca gatttctgtg     420
   ggggatcggg ttaaatgtca aaagaacggc aataccgatt atgattttta tgctaaagtt     480
   gaaaaaattt acgagaactc cgccttcgtc acaattactc attatgattt gcgggatgat     540
   attaatgtgg cggaacttca atatcgggca gtgatcgcct taaagaagat gaaacccgct     600
   aaaccgactg ctggtgaagc gaagcagcta caggcagtta gtgcggccat gttagctgaa     660
   taaattaaga atatcgatgc aacatgtagg taatgggttg ttcaagaatt tgattcttga     720
   acaacccatt tttagtttgg ctgcaaagct tttcggtgtg atggagtgcc ctggttggga     780
   ctgattttat gatgattaga ggccaactga cttaatgaat ctgtccgtta gagctttaac     840
   agattggata tttaacaatc attcacgcgg gtgttgttac aagctaattg tgcacgtctc     900
   tttattcggt gtagcaaact tttttgttgc caatctgact tttagcctgt acatcattgg     960
   tttcaatggt acaatggata ttagtagttg ataactgttc taaagtaagg aggagttttg    1020
   aatgcagaca gctaatcaaa atgtaacgtt gacggggcgt cgacgcttct ggttcatttt    1080
   tacgctgttg cttgggactt ggaccatgtc gatcagtcag tcttccttgt cgacggtcta    1140
   cccaacattt atgcgcgact ttggcatctc ggcctcaacc gtgcaatggt taacgaccgg    1200
   ctttatgttg acaatggtcg tcatgatgcc aatcagtccg tggttgttga ataatattgg    1260
   gtttaagaaa ttattcttag ccgttttggt gcttttcgat attggttcaa tcattattta    1320
   tttcgcaccc agttttggtg tgatgatggt cggtcgccta attaaagcgg cggcagttgg    1380
   cgtgctgttt ccctcgtatc aatccatttt actgacgatt acgcccgagg aaaaacgggg    1440
   tagtacgatg ggtgtcgccg ggttggtcat gggatcagca ttagcatctg gcccgattat    1500
   ttccggtgtg gttttgaagt ttaccgattg gcacggattg tttgccgtct tcatcacggt 1560
   ggcaactttg ttgatcctaa ttagtttcac aacgatcaag gacgtgatgg tcaataagcc 1620
   ttcaaagctg gatttgctct caatcgtgac gttactgggc tttgctggga ttttatacgt 1680
   cgtgaatgag attggtaaac ccaatgtgaa ctggacgttc ggttggctgt tattagcggt 1740
   ttcagtacta gcggtggctt actttactta tcgccaattt cacttggaga cgccgttatt 1800
   ggaactacgg gtcttaaaaa catttaatta tgacttagcc attttcctaa cagcaatctc 1860
   gtacgtggcg ctgattgtcg taacaatcat cttcccactc tattaccagg gtgtcttgaa 1920
   ggtgtcgcca ttcgtttcag gcatgtcact ggtgcctggt gcggtgcttc taagtatttt 1980
   gaacccgctt actgggaaac tagcagagaa gattggatac aaacaaatca tgttacttgg 2040
   tatgagtatg atc 2053
<210> 22
   <211> 1313
   <212> DNA
   <213> unknown
<220>
   <223> lp_0800
<220>
   <221> startcodon
   <222> (797)..(799)
   <223>
<400> 22
   tcggcgggtc aacttaaagg atggtttcgt tcaagttcgc aacggtgaag cctggatgca 60
   aaacgttcac attagtccgt ttaaagaggg taaccgttat aacgttgacc cgttgcggtc 120
   ccggaaactt ttgttgcaca agaaagaaat cagtaagcta ggtgcagcaa cgatgactaa 180
   gggtgtgacg attattccac ttaagatgta tctcaagcat ggatttgcga aagtcttaat 240
   tggagtcgct cacggaaaac gggaatacga caaacggcaa gatatcaaga aacgcgaaca 300
   acagcgtcaa atcgaccggg ttatgaaaca ttactaggca tcgattacgt tagcgttgca 360
   tcgctgcacc atacaacgag tatggcagcg tcataaaaag gtcttccaag aattaattcg 420
   attcttggaa gacctttttt agtatgtgtt gttggtggtt ctgtgccgtg gacggacgac 480
   ggcaagcata tattttatat gtactgatgt agtgctcact gttataaatg ttctagatac 540
   tagcaaaatg gttatcaggc tataaatatt ttgcgaaagg tcgggtacaa gcacttgcga 600
   cttagctggt aattatgtta gcatataatt gtgttgatag ttaaacgcgt tatttattaa 660
   ttgattcaat aaatataatt gatactaaac tttggagaat gttattattc tgatattaat 720
   cagatttagt ataactcaaa ttgataataa gttaattttg atatgattta ttgcatggat 780
   tcttgggggg acatgtatga atagatttat aacgagcaag cagcattata aaatgtataa 840
   aaagggacgt ttctgggtat ttgctggtat taccgtggct acctttactc tgaatccact 900
   gataagtcgg gcagatacgg aaacgacgac cgcagccacc gcagctacga ccactgctgg 960
   ggcatcctca tcatccaact cacaggtttt aaggactact acgactagta caacaggggc 1020
   aacgactcag tctagtgcga cggcgattaa tgcggcaaca acaaatacta gtgctcaaaa 1080
   aaaacaggcg gttagtggca ccactactga ctcaaaggct gaacaaccgg ttacggcagt 1140
   aggagaaaat gaaaacgcaa ccagtaatct cagtacctct gattcggcat cagcaagcag 1200
   tcaggctaaa actggttctg gcaacagttt ggaccaaacg tcaaacagct cagtaagtgt 1260
   cgcatcgagt tcacagaaag tcacaacaca aaattctgat tatcaaaatg atc 1313
<210> 23
   <211> 815
   <212> DNA
   <213> unknown
<220>
   <223> lp_2940
<220>
   <221> startcodon
   <222> (44)..(46)
   <223>
<400> 23
   ggtcatggct gggcaatact ttttaaaagg ggattagttt agtatgtcaa aagcgcttaa 60
   gatagtgatg ggaatcacca tgttaacagg gggcatcatg gcccagaaaa tgacggtaca 120
   tgcagccgaa tctaacactc gaaccggtca ggcagtaagg atgaatggca cggtcagttt 180
   agcaagtcag gtcgaaaata acccagctgt taaggcggct cactaccagg taacacaagc 240
   ggtgcaagca ttaaccatgg ctactacggc agtcaaaacg gctatgagtg atctgcaagc 300
   ggcgcaaacc acgttagacg ctgccaacaa aacgttagcc aagaatcaaa agattcagac 360
   gcatatgggc gtacttaagc aagcagccac tgaccggcac gtcaaggcaa cgaaagcact 420
   agatgaacaa ttggcaacta aaaagacaag ccaaactgcg gtaacgacgg cgcaagcggc 480
   agtgactaag tcacaagctg ctgttcaagt agcccagagc aacttcgata aggataacag 540
   tgcggccaac aaagtcacgt tacaaactac tcaggcaaag ctgaaaacgg tgcaagagac 600
   gttaacagcg gcacaagcta accttgataa gactaatgag cacgtgatga tggcagaaga 660
   agaattagcc aacgccaaaa ttgaagtcag tggcacgagc cgcgacttcc aaatggcaca 720
   aagagattat gatattgttc agccccaagc agcagttaat caggcgaaag cagcggtgac 780
   agctaagttg cagcgcgttg cgggtactca agatc 815
<210> 24
   <211> 648
   <212> DNA
   <213> unknown
<220>
   <223> lp_0141
<220>
   <221> startcodon
   <222> (441)..(443)
   <223>
<400> 24
   cttgtaaaat caagttacgg acagtcggtt tcaagaaatc actgtcagcc tttaccgtta 60
   cgtgtgcgcg taataacgca ctaatttgtt ggagcgtggt ccgccgtcga gctcgttcac 120
   ttaacagaaa gaaacgattt tcagcggggg ttattaaagt tgttgctgcc ggcaatgccg 180
   cccacccctt tcagtaaaaa taccgttcta gcataccata actttcccat tcagtgcgaa 240
   cgttagttct aaaatctcgg tcactctcat taacccaacg gttactccca ccgcggttcg 300
   tttgttatca agcctttatt gattgcattc aaattcaccc actttttgat aattttattg 360
   atattacgat tgttgcaaag cagtcaaggt gctaaactat cttcgaatca aaaaagattc 420
   actttaaggg ggattttgaa atgaaaaaat taacatcagt cgttgcaaca cttgctttgg 480
   cattaccatt ggctgccagc ccgtttgcca tgaccaccac ggctaatgcc gccacggtaa 540
   ccccaaaagc cactacccat gcactcacta aagcgaccca cttccccgcc atcacaatgc 600
   acgcgaaggc cgccacgact ttgaatacaa ttcactttgc cgccgatc 648
<210> 25
   <211> 794
   <212> DNA
   <213> unknown
<220>
   <223> lp_1403
<220>
   <221> startcodon
   <222> (506)..(508)
   <223>
<400> 25
   tggatgatgt taaagcaacg gtactatcga tcttgcaaga tttaactggg gaagatgttt 60
   caaacaatat ggatgccaat ttatttgatg aaggtatctt agattccatg ggttccgtac 120
   aactgttact agaacttcaa aatcagcttg gtattgaagt gcccgtttcc gaatttgaac 180
   gttctgaatg ggaaacgcct gctaaaatcg tagctaaggt cgctagttta caataaataa 240
   aaattcaaat cgccgttaag tacgctgctt aatggcgatt ttttaattag ctggaaaaat 300
   tgtctaatta acaactgatt tactgcaaat aactttaatt tgccgatttc cgaattaccc 360
   gtggcaccag caataatggc tatcacaact cattttggaa aattttacga aaatgatatg 420
   caaagtttac acaattttga taaacaaacg ttcgctaact gagtaaacta aaatcactaa 480
   aatcattttt gtaagggatg gtattttgaa aatcagtgtt catcagctca tctgggtcac 540
   gattggcgca attagcttaa ctatttttac cggattcgga cacctatttg cccggcacca 600
   ccaggctatt gccagtaatc ggccaatcac taaagttagt cattcagcaa ctaaggtaac 660
   cacgccagtc ccggagaccg ttgactggca caagccctcc ctgtcacggc cgtatcccaa 720
   actggctaaa tacgatgaca tcaacattca ggtttcaatc aaaaaacagc gagtctactt 780
   aaaaaaccac gatc 794
<210> 26
   <211> 1358
   <212> DNA
   <213> unknown
<220>
   <223> lp_3176
<220>
   <221> startcodon
   <222> (770)..(772)
   <223>
<400> 26
   ttaccggcgg ccttgatttt aaagtcaaac gggttatagc tgactttatc tctaccgcta 60
   tttttcaatg ttacggtgac cgtataatat tgattgccag ttttaggctg aacatcattg 120
   agcgtcgttg cggttttaac attattaacc gttacttgca cgtgatcgat ggtggctgtc 180
   tgcccgactt tataaatcgt atttgccggg gtttccttag cggtggactt gctagcactg 240
   gtctttttgg tcgtcgctgc ttgtgagttt ttaccgtctt gtggagaatt gacgaggctg 300
   atgacaaaga agatgccaag gagaatcagg acccacgccc accatttttg ataccaagct 360
   ttcgtttttt gcatgagctt acctactttc ttagtgctaa gccgcccgca ataacaatga 420
   gcagaccgct tagaatcccg aagaagctga caaagacgaa gttaagaatc ccacaaagga 480
   tgatcaacca gcccattaaa acgtgattgc gattaatcat gacacttaaa ataatcgcga 540
   ggacagagac aataatgcag ccaacgccaa gattgctgac cccagccgat ccgctggttt 600
   ctaaagcact accgatgccg ccgatggtca tcgccagtaa gccggcaatg ataccgaaga 660
   taccgccaat aattcctaaa actaattcgg ctgttcgcga ctggtgtgcg actggtgtga 720
   cttcgttatt ctgatatttt gacattgtaa ttcctccaaa agtatggtta tgataaaagt 780
   tagtaatttt gataaaaagg ggggtgtcac ggtgttaccg attgttcaag caatgcaatt 840
   gttaagtacg gctggttatg aaccactagg accattagtt gcgcaacgcg agtttccgtt 900
   actggttaag aagcaacagg ttgtttcggt agctaaacta gtaaatcctg ataatgttcc 960
   tgttctacag cgtttgaagc agcagccaat tccccgaatg ccccggatcg aagcattatt 1020
   aacggcgact gattgcattg taagtgttga gaccttaatt aatggtcaaa cactagtgga 1080
   tcgattggcc acacaaggac cgttttcccc cgaaatgacg gtgcgcgtcg cacaaggatt 1140
   gttgataacg ctacaggcgt tggggcaaca acaaattgtg catcgggacc ttaaactaag 1200
   taacgtgatg tgttatcggg atcactacta tttgattgat gtcaacgcag cgcgacaata 1260
   tcatgctgag caagacagtg atactcgttt attaggcacg agcggttttg ccgcacctga 1320
   aaactatggc ttcgcccaaa cggatcaacg gagcgatc 1358
<210> 27
   <211> 1201
   <212> DNA
   <213> unknown
<220>
   <223> lp_3514
<220>
   <221> startcodon
   <222> (538)..(540)
   <223>
<400> 27
   gccttgctgg cacgagaggc ggccggaaat caattagtat taaaccatgt ttcgttgccg      60
   cacgcctcca cggaattgag tgatgcatat aaagtctttg cgattacttt tgatggaata     120
   atgaaaattg atggtaaccc catttactta attctaatag tacttgctaa cgaaaaacgt     180
   gcagatagtg ggcaggtatt cagttattta tacactaggc ttaaacagct cagtaatgtg     240
   cagctacaaa atgttgatac gtatgaaacg ttaattaaat atttgaagta gttgaaatgc     300
   aattagtgat atctgacagc aatatatcat tgctgagatg atagaaattg acatttaacg     360
   cttgcaatcg ttttccgaaa ccgctatact agctaattgt aaataaggat tgttaccgct     420
   aacattagct agtgggcaaa acctaattaa tcgattgtat tcgtgattga tagcacggat     480
   gcagtgggtt attaggtttt tttctttatt taggtatgag agtcgaggag cgtgcgcatg     540
   cgaatcaaaa aggtttttaa taataacgtc ctattggctg aaaaaaatgg acgggaagtt     600
   gtcttaattg gtaaaggaat cgggttcaaa aagcatactg gtgacgatgt tgagcaacaa     660
   cagattacta agatttatac gccagaagga gataattgga ttagtaattt ccaaagcttg     720
   atggcagata ttgaaccgga atattttgaa gttgcttcgc aaattattga gttggcagaa     780
   caacaattgc aaacgacttt taatgtttat ttgttgattt cattgactga ccatattcac     840
   tttgctgtct atcggcacca ccatcagatg gatattcata atgaaatctt gtgggaaact     900
   aagcgaattt atcaccgtga gtttcaagtc ggtatgcaag cattggcact gattaaaaag     960
   cattttgatg tgacgttgtt ggaagatgaa gctggattta ttgcaatgaa gtttgttgag    1020
   aatagtatgg ctgactctaa cactgatcaa acacttgcaa tgactaagtt gattaatgat    1080
   attttgaata ttgttaagta tcagctgtca ttaacgatgc cagatgagag tgttagtttg    1140
   caacgatttt tagttcatct acgctttttt gcggagcgtt taacgctgaa acgacctgat    1200
   c                                                                    1201
<210> 28
   <211> 1191
   <212> DNA
   <213> unknown
<220>
   <223> lp_3646
<220>
   <221> startcodon
   <222> (740)..(742)
   <223>
<400> 28
   cctgaaatgt tttcttcaac catggcattc aagtgggcga agctctgacg gatgtcaaag      60
   aagacggtgt gtgcttcggt actcatgcgt gtcgttaacc aggcgataat gggtgttagg     120
   ataattagtg cgagcattaa ttgccagttg agccaaccca tgacaatcac ggcagttaag     180
   aaccatagga cacattcggc ggtgttatag gtaacccagc atagaaagtg tcggattgca     240
   tcagtatcac cagttaaccg agccataata tcgccgttag tgacagaatt gaagaagggg     300
   taatctaagg attgcaattt cgtgaataaa tcatggcgaa tgtcatacaa tgaattttga     360
   ccgacgcgtt cccagaggat aatgtagcag taacgcatca cggttctaat taacgtgata     420
   ccgaccatcc ctgctaataa cggcagaagc aagttgagat gccgttgatt gatgaccacg     480
   tcaacaatga tgccacccaa taatggtact gctactacac caagtgcatt gacgataatt     540
   aagagactag ctagtgtgat ttgccagcgg ctgtgccggg cgtattgcca gacccactga     600
   taaggattca tgattaacct cccgtagtaa ccagctaaag ctggctaact tgacaaggct     660
   aagtatgatg atattggagg gatcattgaa tagattattt tgactagttt aatgtctttt     720
   tttgtaagga gggatcacaa tgttgcaatt aagtgaattt gaaccggaag ttctttacgc     780
   ctttccgttt cgaaatgaac atccaggaac ctacgtctat catcaacatg attttctaga     840
   gctctcaatt atgttggaag ggtattcaga ctacaatgtg gaaggacaat ggcgtcgagt     900
   tgttgccggt caggcactgc tgtttaaccc tggcattcat catcaggaga cccagccccc     960
   gcatacccaa agccttcaac tgcacattgg ctttcggcat atcgcgctac ctggtcaggt    1020
   gcctaaccac ttgcccttta aagatgcgtt aattagtctt gatgattatc gggcagaatt    1080
   catggcaaat gcccaacgaa ttgtagcgga aagtcagcgc cccactgcat ttgggcacgc    1140
   cttgttaatt caagcgatgg tgattgaaca gctttgctgg ttattgcgat c             1191
<210> 29
   <211> 1027
   <212> DNA
   <213> unl<nown
<220>
   <223> lp_0237
<220>
   <221> startcodon
   <222> (575)..(577)
   <223>
<400> 29
   tgtccggccg caattttgcg gatatgtaac ggggttgcct cgttgatgac ccgagtcccc      60
   ttcaggtgta gccaaacttg tgcggcttcc accttagctg ccggaaaatc actaagttgt     120
   ggctgacgga ttgccttaaa cgttgctgcg gtactacgct gcagccgacg ttgatgatag     180
   gtccggcgct ggcttgtatg ccgttcaagc tgcacttgtt cccacaccaa acaagcacca     240
   aagaggacga taccgacccg taatgcccaa atcaattttt tcaatgatta gccccccact    300
   ttaccataat tacgctactt aatttaacat ggtcttagct aattatattg gcaaagccgg     360
   taataattta attattaata gggcagtcgc accaactcgc catttctagt tgcgcttccc     420
   cttaaactat taacgatttc taacttgaca gctaaactga tttattgtca aaatcactat     480
   ttaaatattt tattttatga tggcaataca aacattttga ttatcgcgat atacttagac     540
   gagcattagc tttttcaata aggaggcact actgatgaac tcattcaaga acccacttat     600
   gacaacaccc tatctgctag ccataatcgc cacactgacg atgctagtta gtccactgac     660
   aacggttgtt atatcagtac tgatatggtt gtcgggaatt acgctggtgt ggttacaccg     720
   ccagcaacga tggctccgct tcttctacgc acccgtgttt ttgtacactt ttttactaga     780
   acaaaggtat ttctccccat caacccagcc actgacagaa acgattctca ttaacctcgc     840
   aattcttggc attgccattt tcagcacatg ggcagcttat caattacgtc ataacacgta     900
   aattactcac caagaaaaat aagtccccca gatgcggtaa ccgtttctgg gggacttatt     960
   gattgcttca acagctaccg ttaaataacg ctcgttaggc aggcttcact tcggctagtt    1020
   tttgatc                                                              1027
<210> 30
   <211> 991
   <212> DNA
   <213> unknown
<220>
   <223> lp_0299
<220>
   <221> startcodon
   <222> (604)..(606)
   <223>
<400> 30
   tgatagcagt taccaaacgg ttaagagttc gatgaataac gttaaatcat ttgcggataa      60
   gattgtctgg ctcgttgcga ttgctggaac gattatctta gctttaatcg ttatcttgat     120
   gattcgtgaa cgccgctatg aaattggtgt cttgttatca ttaggtgaga gccgttggaa 180
   gattgtcgct caattctttg ctgaaatggt gatggtcttg atcgtctcag ttgtcttagc 240
   cggattcggt ggtaagtttg ttggtaataa gctgggtgac cagttagttt cacaacaaac 300
   gacgactgca acgactacga cgagttcaac tagtcagcag ggtcagcctg gtggcggtgg 360
   cggcgcccct agtggtaaca ctggtggtag caagccaagc ggcaatatgc ctagtggcgg 420
   cggtcagact ggtaatgcca gtgccacgac cgacacgaac ttagatatta atgtcacggt 480
   aatggattta gttgaactcg gcggctttgg cttagccatc atgttcctgt cgattatgct 540
   ggcttctggt ggcatactac gactacagcc gaagaaagtt ttaattgatt aggaggggcg 600
   gcaatgttaa aagtaaatga tcttggttac tggtatgacc gccaagaaaa tagtctattt 660
   gagaatgtaa accttgaatt taagtcgggg acttcgtatg ccattgttgg tcaaagtggt 720
   tccggtaaga cgaccttcct ctcactgctg gcgggtctag ataaaccgcg ggctgggcaa 780
   attgaactca atggtgaacc aatcaataag attggtctga cgaactatcg gaagtccaaa 840
   gtggcaatcg tttttcaatc atataatttg ttgacgtata tgtcaccatt gaataatctg 900
   atgactgcca tggcaattac gaactcggaa caccgtggtg acaaggatta tgcgatgaag 960
   atgttacgac ggttaggtat tacagatgat c 991
<210> 31
   <211> 537
   <212> DNA
   <213> unknown
<220>
   <223> lp_0305
<220>
   <221> startcodon
   <222> (532)..(534)
   <223>
<400> 31
   cccgtggcaa ccccagcgca cgttgcggac tatcaagaaa taactgacgc ctattttgcc 60
   cccgctaact ggcaccaaac accggttggc tttgatggtt ttggcgatta ctcactcatg 120
   ggccgaccct attttgacca cggtatgcca tcacgcgcaa ttgcattaca tcttatttat 180
   gttacggtta acggtgacct gcgcatccat cattttgttt cggacagcaa tgccaatatg 240
   cgtggtcaaa agcaaaagtt ttttgaagca ttaacaaagc tcaccgactg ggcacccgca 300
   cacctgacgg gactcaatcg cacccccgcg cttgccaccc tattagccta cgctgagggc 360
   gataaattcc cagggttagg aataattaag aaattagcaa tcatgcacca ctttcaattg 420
   atgcaccgac tactagcact gaattgagga gaaatgatgg caaaacaaga atctgcttgg 480
   aaacaaatga tccatttcta tcgtcatgat tacgaagaac agcatgctcc gatgtta 537
<210> 32
   <211> 1710
   <212> DNA
   <213> unknown
<220>
   <223> lp_0393
<220>
   <221> startcodon
   <222> (1150)..(1152)
   <223>
<400> 32
   cggttgacca ccaccatcag tattcttagc ctctccactg taacaggcat cactaccgtt 60
   attacaggaa tcattcccca gctcaaacgg acctttccca ccgtcccgac tacgctgatt 120
   gaatggctcg tcaccatcgc caactgtagc gcattgatta ctttgttatt caacccccga 180
   ttaacaagcc ggtggggatt acggccaatc gtgattagtg gactcttgat cagcgccatc 240
   atgggactga ttcccgctat cacgactaac tttaccgtaa tcatggtcag tcggctcgga 300
   ttaggattag gcgtcggcct cttttcacca catgccatca gtttgttgac ccacagcttt 360
   acaggtgatt tacgggcccg actgttaggt taccagacgg gcctctcagc cctaggtaac 420
   gcgatattgc taggattagc aggcctgctg atcagtctca gttggcatgc cgtcttttgg 480
   ctatacgggc tactggtgat tgttgctggg ggcgtggcgt ggttcgtacc ggaacccact 540
   gttccatcaa caacgactaa tcacgcacgc actgaaaaga cacaactacc acggcaccag 600
   tggacgttac taggacttac ctttatcacc tatctactca tctggggcgt tcaattaaag 660
   ctgccgagtt actttagcgc ccgtcatttt ggtaacgctg ccactatcaa cttaacctta 720
   gcagccatga acatcggtgg cttgctggct ggtctcacct ttggctatct ataccgctat 780
   ttacaccggt tcaccttaac cttaggctat gccggagcag ctatttcagt cttagtatta 840
   tggttggcat ccaatgcaac tgtggctatt ggcgctgccg tatttttcaa ctttatttac 900
   tcgtacaccg ggccatattt ggtcttcact agcaatactg gcctggatac cattcaagtc 960
   aacgtcctga gtagctacct gacgattgcc accattatca gcgctttctt tgcgccactg 1020
   gtctggaaca gtctcggcca acttgggccg cagacgctca ccgccaacgt tctcatttgg 1080
   attatgctca ttttaggtgg cttagccttg attaccggca gccatcatcc acgaaaggaa   1140
   gtttaattaa tgaccaataa taacgatcgt cttcatactg gcgaactcta cttacctaac   1200
   gaccctgaac ttgaaaaacg ccaatttggc tatctcgatc agctctatga tttcaaccaa   1260
   actcggccca gcgaactaac caagcgccaa attctactta gcaagatgtt cgcagaaatc   1320
   ggcccgaagt gttacatcga accaccgttt cacagtaact ttggtgggca ccatgtccac   1380
   tttggcaagg gggtttacgc caattttaac ctaacactgg tagatgatac ccatatttac   1440
   gttggcgact atacgatgtt cggccccaac gtgacaattg caaccgcggg ccatccgatt   1500
   ttgccatcat tgcgcgaaca agcttatcag tacaatatgc cagttcatat cggccgtaac   1560
   tgttggttcg gcgcaggtgc catcgtacta cccggtatta cgattggcga caatgtcgtc   1620
   gttggcgctg gtagcatcgt gaccaaagac ttacctgata acgtcgtcgc cgtggggaat   1680
   cccgcccata tcttgcgaca cattaatgat                                    1710
<210> 33
   <211> 1180
   <212> DNA
   <213> unknown
<220>
   <223> lp_0419
<220>
   <221> startcodon
   <222> (266)..(268)
   <223>
<400> 33
   caacaattat gaaaaaattt ctagttttgc gtgaccgtga attaaatgct atttcaggtg     60
   gcgttttcca tgcctatagc gcgcgtggcg ttcggaataa ttataaaagt gctgttgggc    120
   ctgccgattg ggtcattagc gctgtccgag gattcatcca cggatagttc aagccatcaa    180
   gtttaagcac tataagaaag cactcgattt atgactgggc ctgcagtgct cagccttttt    240
   agtttatatg gggggaattt taactatgcc attaataaaa gtaataatca cagctattta    300
   ctacctaata gtgctctttt taatgaatcc tttgactgat attatgggaa taaaagacgg    360
   accctttcaa ttcatattaa ctgaatctat tatattaatc gcgataatca ttctgaatag    420
   gcgatatgtt aagcaaccta tccactggct acccgttaat atcatgtcaa tattaaagaa    480
   gaatagtctg cctttgagcc tagcaataat atttctactt attttctttc gaaaccacat    540
   gtatcaattt ttaatctcat tattactttc gttgattgta gcgataactg aagaatacgc    600
   ttttcgtggc atgatattca gaacgctttt agcactaaac cttaaaaaat ttgccacatt 660
   gcaggccacc atcgcttcga tgatgactgc ttcgcttatt tttgcggcta tgcatttggt 720
   caacctttta tcacagcccg tatggtcagt attgtgtcag gttctctatg ttattgggtt 780
   aggtatttta cttgcagcaa tctatttaaa aaccggaagc ttactggctg ccattagtgt 840
   ccattggttg atagattttt ctagtttcta ttctcaaggt attgacccaa ctcaatcacc 900
   cattaacggg ccaatggaag ctcttctaaa ggggctcttt ctaaatattt tattcattgg 960
   cattgctacg tttatcctat cctctaagca ttggaagtta ttgagtatcc taaatattga 1020
   agataaaata gacgagtaat catccattct gaattcctat aaattattgt ttgcagcact 1080
   acaatttcaa ttatctctga agttttgggt tacgctaaga gctagtcata tcgctaacta 1140
   aatttcacgt cttaacgcta tgctaatata ctttacgatc 1180
<210> 34
   <211> 578
   <212> DNA
   <213> unknown
<220>
   <223> lp_0489
<220>
   <221> startcodon
   <222> (443)..(445)
   <223>
<400> 34
   cctaaaggac tttcctcaat tcaggatatt agccatagta agtctgataa tcaaactgaa 60
   ttacttctaa acttcagagt aagtaggtat gtaaaatccg gttatgttga ggcaataaag 120
   aaacaaacag ataagaatat ttccttggaa gtgttaagag atacgccagc gtctacttca 180
   aagcgtagta atcagttagc taatgctttt gtctcgggat ttaaagtggt acagaaaatg 240
   attcctgaat atattgtaaa gactggtact agtgatatga atacattagc aacccaatgg 300
   actgttccaa tggtggcata tggtccaggt gactccacac tcgatcatac actgcaagaa 360
   gaagtgccgt ttgctgatta tcatctaggt attcagacat tacaagaggc catccaaaca 420
   tacttaggca aggtgacaga taatgacaaa taaacagttg tcagtgacac aacaaagaaa 480
   gttattgtta gactccttta aaattcgaga agaaattatt gatattgctg ccactctaac 540
   cggttgtcat attggtggga gcttatcaat cacagatc 578
<210> 35
   <211> 667
   <212> DNA
   <213> unknown
<220>
   <223> lp_0698
<220>
   <221> startcodon
   <222> (115)..(117)
   <223>
<400> 35
   gatcgttagt ggaagcagca caaccaggaa gctaactgtt caggacgttt aaacaatggc      60
   ttataataag taatgataac caaagtatga ctgatgtaag aaaggaagaa tcgcatgtat     120
   cgggcactat atcgggtttg gcgtccccaa cgctttgatg agattgtcgg gcaacagatg     180
   attacccaga cactaaaaaa tgccattatg acccaccaga cgagtcatgc gtatctgttt     240
   accggtcccc ggggaactgg gaaaacgtcg gcagcaaaga tttttgccaa ggcaattaac     300
   tgtcaccatc ttgttgatgg ggaaccttgc aatgaatgtg agacttgtac ggcgattacc     360
   aaggggcaac taaatgatgt catcgaaatt gatgcggctt ctaataatgg tgtggaagag     420
   attcgtgata ttcgggacaa agcgaagtat gctccaacgc aagcggacta taaagtctac     480
   attattgatg aagttcatat gttatcgact ggcgcgttta atgcgttgct caaaacgcta     540
   gaagaacccc cggctaacgt tatatttatc ttagcgacaa cggagcccca taagattcca     600
   ttaacgatta tttcacggac gcaacggttt gattttcggc ggattacagc taaggatagc     660
   tacgatc                                                               667
<210> 36
   <211> 1262
   <212> DNA
   <213> unknown
<220>
   <223> lp_0740 and lp_0741
<220>
   <221> startcodon
   <222> (256)..(258)
   <223> lp_0740 start codon
<220>
   <221> startcodon
   <222> (299)..(301)
   <223> lp_0741 start codon
<400> 36
   atcggtttac ggggctatgg tcaattgaat ccgttagttg aatatcaacg agacggttac      60
   cagatgtttg aagaaatggt tgcggatatt gattatgata cgacgcgact cttcatgaag     120
   tctgaaattc gccaaaatat tcaacgttaa aatttaacgt tagtgctagc gagtctaaga     180
   ctaaggtctt aggctcgttt tacagttaaa ccagatatta aataattaat aaataagttg     240
   aaagaagggt cctttgtgga attaagtgaa tataaacacc tgattgaaga aatgcaaagt     300
   gctgtcgacg actttagggg gtcgctttga cttagatgct ttgaacgaaa gcattcaaga     360
   aaatgaagct cgaatggccg aacccggttt ttgggacgac caggctgccg cccagaaagt     420
   catcgatgag aataacgtgt tgaaaggaaa gtatgatacg tttaagcaat tagctgatga     480
   agtcggtgac ttggcagtcg cttatgagtt actgagtgag gaacccgatg ctgagatgca     540
   ggctgagttt gaaacggact ttcagcacgc tgaacatgat ttacaacagt accggctcaa     600
   cttacttttg gacggtccgt acgaccgcaa taacgctatt ttagaaatcc atcctggagc     660
   tggtgggacc gaatcccaag attggggtgc gatgctacta agaatgtaca cgcgctgggc     720
   tgcgagtcac aatttcaaag tcgagactgt tgactaccaa gctggtgatg aggcaggcat     780
   taagagtgtg actttattaa ttagcggcca caatgcttac ggttatttgc ggtcagaaaa     840
   aggggttcat cgtcttgttc ggatttcgcc gttcgatgcg gctggtcggc gccacacatc     900
   ttttgcgagt gttgatgtga tgcctgagtt agatgatacc gtggatgttg atattcgacc     960
   tgaagatttg aagattgacg tttatcgagc cagtggggcc ggtggacaac atgtcaataa    1020
   gacttcttca gcggttcgga ttacccacgt gccaacggga attgtcgttg ccagtcaagc    1080
   gcaacggtca caattacaga accgacaaac agcattgaac atgttacgag cgaagttgta    1140
   cgaacgtgaa gaagagaaga aggctaagga acgggcggcc atccaaggtg agcaaatgga    1200
   cattgggtgg ggctcacaaa ttcgctccta cgtttttcat ccgtatacga tggtcaagga    1260
   tc                                                                   1262
<210> 37
   <211> 1614
   <212> DNA
   <213> unknown
<220>
   <223> lp_1847
<220>
   <221> startcodon
   <222> (1149)..(1151)
   <223>
<400> 37
   acaccacgct gtcccagctg gtggtgcgct agccgttgat cgggatacgt tctctgcaga 60
   aatcacggct gcaatcaccg cgttacctaa tgttgaaatt attaatgaag aaattactag 120
   tctcccagat ggcattacgg tcgtagcgac tggaccatta acggcggcgt ccttagcgaa 180
   atcgattcaa gcgtttaacg acgaagatga cttacacttt tttgatgcgg ccgcaccgat 240
   tctaacgaaa gattcgatcg acatggataa ggtgtatctc aaatcacgct atgaccgtgg 300
   tgaagcggcc tatctgaact gcccaatgac ggaagccgaa tttgacgttt tctatgacgc 360
   gttgattcac gccgaaatgg ccgaagcgca tgattttgaa aattcagatg tctttgaagg 420
   ctgcatgccg atcgaagtga tggcacaacg tgggcgccaa acgatgctat ttggcccact 480
   taaaccagtg ggccttgaag atccgaagac gggcaaacaa ccctttgcgg tcgttcagtt 540
   gcgccaagat gatgccgctg gtgatctata taatattgtc ggcttccaaa ctcatcttaa 600
   gtggggcgaa caaaaacggg tcttttcact gattccgggt ctggagaacg ttgaatttgt 660
   ccgttatggc gtcatgcatc gtaatacttt tatgaagtca ccgaaactat taacgccaac 720
   ttatcagaca cagcaacggc ccgatttgtt ttttgcgggc caaatgacgg gcgtcgaagg 780
   ctatattgaa agtgcagcga gtggcatcgt tgctggaacc aacgcagcgc gcttagcact 840
   tggtttagaa ccggtcgttt ttccaacgga cacgatgatg ggggcgatgg cccactatat 900
   cacgcatacg agcgctagca acttccagcc catgaacgcc aactttggca ttatgccaaa 960
   actcaaacag cgcattcggg ataagcgtga gcgtaatacg gcgatttctg aacgggcctt 1020
   ggctgacctg acgactttta aagatgaaac attgaccgtt aacaactaaa atatcgcccc 1080
   attgacaggg tgcataattg tgttttcctc catactttgc taaagtagca acgtatggag 1140
   gtttttgcat ggaaacggat tatttaaccc tctttttaaa gtacattcgg gttgaacgac 1200
   agtattcgga agagaccgct gctgcttatc gggaagacat tcaagctttt aatgatttct 1260
   taacggcaaa tggcggcgcg aaagcgtata ctgaagttga ccggttggac gttaacgtct 1320
   atttaagtca tttatatgac cggcacttga cccgcaattc aattgctcgg aaagtttcaa 1380
   gcttgcggtc gttctataac tttttggtta agaatgattt agctaaattg aatcccttcg 1440
   tgtacgtcca actcaaaaaa cacgcggcgc gcttaccccg ttttttctat caaaaggagc 1500
   tggacgtatt atttcaaacc gtctatgcgg atgatagcgt gatgggggcc cggaacgcgg 1560
   cgttattaga agtcctgtat ggtactggga ttcggctgag tgagtgtgtc gatc 1614
<210> 38
   <211> 815
   <212> DNA
   <213> unknown
<220>
   <223> lp_3051
<220>
   <221> startcodon
   <222> (275)..(277)
   <223>
<400> 38
   cactaaaaag acgactaatt actaagattg tgaatctttg taattagtcg tctttttgta      60
   ggcagtaata tcaaatagca tgaggactat agttcaccgt catttaacac ttggttacaa     120
   caggtatgcc gtgattgccg caccttttct aaacgatatt gtgaaaaata taattaatta     180
   gccaaaaagt attgcaaatt gtaattatta cgactataat ttgattcgta aattagtaat     240
   gatagcgatt acaatataaa aagaggttat taatatgact gaacgtaatt ttgatttctt     300
   aatgcctagc gttaactttt tcggtcctgg tgtcattagt aaaatcggcg agcgtgccaa     360
   aactcttggt atgcacaagc cagtcatcgt caccgacaaa tttcttgaaa gtctgccaga     420
   cggcgccgtt gctcaggtgc ggcggtcact ggatgctgcc gggattgact acgtaattta     480
   taatcaagtt gagcccaacc ctaaaattca taacattcaa gctgttaaag ccttatatca     540
   agccaatcaa gcggactccc tgattactat tggcggtggt tctgcccacg ataccggaaa     600
   aggtgccggc atcatcatga ctaacggcga cgatatcact aagttagcgg ggatcgaaac     660
   actcaagaat gccctaccac cactgattgc ggtcaacact accgctggga ctggttctga     720
   attgacccgt cactgtgtca ttacgaatga agaaacgcat tataagttcg tcgttgcttc     780
   atggcgtaac atgccactcg tgtcctttaa cgatc                                815
<210> 39
   <211> 587
   <212> DNA
   <213> unknown
<220>
   <223> lp_3082
<220>
   <221> startcodon
   <222> (432)..(434)
   <223>
<400> 39
   atattatcct caccccacac gcacagctca tgtaagacaa cggacaaact ccggccataa 60
   tcacttagtc cgtattccac gcgcggtggc acttgttggt acactttgcg cgtcacaata 120
   ttagcggctt ccaactctct aagttgttgt actaacattt tctgcgagat ttgtggaatc 180
   gcgcgttcca attcgctcgt ccgcatcgtc tgagtacgca gatggcacag aataattgat 240
   ttccacttac ccccaataat ttccattgtg gcctcgaccc caatatgata cgttttggtt 300
   tccaataatt gtccctccat tccatactta ctttttagtg ggtatcccac tttaaagtcg 360
   gtacttgtct aaaagtcact tgctagtata atcattattt atcaacttag caatagcagg 420
   aggcttatct tatgacaaaa cagtttaatc ccagttggac actttttgca ctcgcaatca 480
   gtgccttcgc cattgggtca acggaattta ttagtgttgg tttaatgcca ctactcgtta 540
   gcaactttca catcaacttg gcgcaagctg ggctgaccgt ctcgatc 587
<210> 40
   <211> 1736
   <212> DNA
   <213> unknown
<220>
   <223> lp_3281
<220>
   <221> startcodon
   <222> (1107)..(1109)
   <223>
<400> 40
   cttagtgtag gccgcgtggt cagcttcggg atcattgagt ttgctaatta agaaagccca 60
   cgtcgtatcg tctaactgaa tataacgaca cccaaggtca taaaaatgtt gaatggtatc 120
   gtgataagct tgggcgagat catttaaata ggcatcccag ctgtcataga atttggtcca 180
   attgtcactc cgattatcac ggaataacat agttggtgat ggaatcgttt gtttagcgag 240
   tacgccagcg ggaacgattg attgtaagta ttcaaaggca gcaaagaacg gatggtcggg 300
   attatacgcg actttgccaa taagttcagc gttatccgtt cgagtatggt cccccttgaa 360
   cttatagctt tgatggtagt cgtacttgcc aacaccagtt aagccccaaa ggaaatctaa 420
   gtgccaccag ctgcgtgaaa attcgccatc ggtcacggct ttgagcccga gcttaacttg 480
   ttcagcgacc actcgtttga tttcagtgtg ttggatttca agttcttgat cacgcgtaat 540
   ggtaccggcg gctaattgtt catgggcctt tttcaaacta gcggggcgca ataggctgcc 600
   gacgacgtca aaacggaatg ggacggttaa agttgtagtt gtcattaaat atcaaactcc 660
   ttaaatttta ttgggaacga aaaaacgtcg tcctgaataa tattaatcat tattcaggac 720
   gacgttgggc cgtggtacca cctgagttct aggctagtga actagtctac tcgatacagc 780
   tgccaaatga ttggcaattg ctaacgcggg taacggttgt taaaccggct ttagttaatg 840
   ctggttactt tgttgatcac cagacactca ccaaaacgaa ctagggagaa gaccatcttc 900
   attggttgtg ccgataccgt ttcaccaacc cgatactcgc tgaacagcga caccaattac 960
   tcatctttta atcgttcttt aattattgac acctatcata ctggtaatta ttttaaatgt 1020
   caaatttaat ttaaggccgg aatcggtggg cgggattaat ccatagtcat gctataatgc 1080
   aagtaacatg cgattggggg caaattatgg atagaatcat tcggtttttt aagcgggatg 1140
   acgttgactt gtacttaacg ttggcgttat taatcatcat tatttattta atgcgtgggt 1200
   ttgcgacagt tgttctatta acgacgattt ttgcgtattt gggcattaag cttagtcggt 1260
   ggttaaacct ccgaacgcac ttaccttatt ggattgctgt cattgtggtc tacgtcggcg 1320
   tgattgcgtt gtttatcggt gcgctatcgt acgcggcgcc taccctggtc gatcagttga 1380
   aggtcattcc ggatatgctg gctaaagcaa tcaccaatca cccggtattg aataagaata 1440
   ttgataaatg ggtcaaccaa gccattcata gtagtgaatt gattcaaaac gggaaggcgt 1500
   tactcgtcac cggtattaaa ggactgagtg aggttggaac tggctttacg cacgtcttaa 1560
   tggcaatctt tttgagtttt atctttgcag tttcgcgggg acgaatgatg gtctttggcc 1620
   gccagttctt gcagtcgaag tttaaaaagt tcttcacgaa cgtttactat ttaacgcata 1680
   aattcgttat gattctgggg cggattattg aaacgcaact ggtgatttgt acgatc 1736
<210> 41
   <211> 749
   <212> DNA
   <213> unknown
<220>
   <223> lp_3500
<220>
   <221> startcodon
   <222> (137)..(139)
   <223>
<400> 41
   atacatggct aagggtattg attacgttgc ggattacacc tataagcaga ctgtgtctgt      60
   tttaaaggaa gcgtggcctg aaattttaaa agacccagtt caagcttaga tatttgatta     120
   taaggagtga ttattaatga ataataattg gttaggatta acggataaag ttgttgcaat     180
   tacgggtgct gttggtggta tgggtaccaa gttctgtgaa gagtttgcta agcagggtgc     240
   caaaattgtc ttgattgata tgattgcgga taaaaccgaa gcgtacgcaa agaaattgac     300
   agaaaagtat ggcgttgaaa cgttggccgt taagtgcaat acgactaacg aagaggaagt     360
   tgatgctgct gttaaggccg tgactgataa gtttggtgaa gtcgatgtgc tagttaatac     420
   ggctgctatt ttacgcttct caccacttga agacttgcgt ttggatgaat ggcaagctgc     480
   cttgaatgtt aacttaacgg gttacttctt aatgtcacaa cggtttggtc gcgtcatgat     540
   tcaacagaaa catggtgcga tggtacacat ctcaaccgta gcgtcacgtt atcctgaaac     600
   atatagtggg gcctatagta caactaaagc agcggttaat atgatgtcta agcaaatggc     660
   tgctgaatgg ggacagtatg gtgttcggag taactgtgtc ttaccttgtc tggttaagac     720
   gccgttatct gagaactttt acagtgatc                                       749
<210> 42
   <211> 619
   <212> DNA
   <213> unknown
<220>
   <223> lp_3505
<220>
   <221> startcodon
   <222> (26)..(28)
   <223>
<400> 42
   tgcataaaga gaggagtggc acacaatgtc aattcatagc aataactttt attccaacgt      60
   gttacgacag tcagtgcaag tccaagttat cttgccggaa ccagtgaacg cggtcggaca     120
   aattttacca gactacagta gtggtgaaca gcaacttcca actgtgtggc tgttgcatgg     180
   cttaggtggg gacgcaacgt cgtggttacg gcgaacggaa atcgaactac tggcaacgca 240
   ataccgagta gcggtcgtca tgccggaaac ggggcgggga ttttatacga atatggtcca 300
   tggtccacgc tattgggatt acttgaccca cgagttacca acacgaatgc gttatatctt 360
   cccgttgtcg gcacgaccgg aagacaatta tttgttgggg aactccatgg gtggctatgg 420
   ggcattacgt tatgccttta cctatccgca gcggtttgcg gctgtcgcgg cactatcacc 480
   ggtgacggat ctcaaaaggt ttcacgtgga acaatcggca attatgccag accttgagct 540
   ggctttctca ccagcacaga tgcaagggac gccagcggat ttgtcttact tggtacagca 600
   accgacaaac tgggggacg 619
<210> 43
   <211> 1503
   <212> DNA
   <213> unknown
<220>
   <223> lp_3662
<220>
   <221> startcodon
   <222> (218)..(220)
   <223>
<400> 43
   gaggagcttg cggttgtttt tgactgctat tgttcattaa tgaacttaac aatcgaattt 60
   ttttattggc tataaatact gctaaacatg agtttataag tgtactgttt ggatgatatt 120
   aaataatttt tcaaattctt gttgcattat gagcaaggat ggctataata aagtttgtaa 180
   attaattcac aaaggaattg aacggaggaa gcacttcatg attaagacag aaaaaaatca 240
   aacaagcaaa gttactgacg aagttgatca attagttcaa cgttctaaga aagcattagc 300
   tattttgaag tcatataccc aagcacaaat cgatgattta tgtgaaaagg ttgcggttgc 360
   cgcactggac aatcatatga agttagccaa gttggcggtt gaggaaaccg gtcggggtgt 420
   tgttgaagat aaggcgatta agaacatcta cgccagcgaa tatatctgga acagcatgcg 480
   ccatgataag acagtcggcg tcattaaaga agacgatgaa gagcaactaa tggaaattgc 540
   tgaaccagtt ggtatcgtag ccggggttac acccgtaacg aatccaactt caacaacggt 600
   tttcaagacg ttgatctcat taaaggggcg taacacgatc gtctttggtt tccatcctca 660
   agctcagaaa tgtagttctg cggctgctga tgtgatgcgg gaagcgatta aagctgctgg 720
   tggtccagct gatgctgttt tatacatcga acacccaagc attgaagcaa ccgacgcctt 780
   gatgcaccat acggatgttg cgacgatctt ggctaccggt ggtccaagta tggtgacagc 840
   cgcttactca tcaggtaaac cagcactggg tgttggtcct ggtaacggcc caacttatgt 900
   tgaaaaaaca gctgacatta agcaggcggt taacgacatt gttttatcga agacgtttga 960
   taatgggatg atttgtgctt ctgaaaatag tgccattatc gacaaggaaa tttatgctga 1020
   agttaaagct gaatttattc gtttgggttg ctactacgtt aaacctaaag atgtgcaagc 1080
   cttgagtgat gctgtcattg atcctaaccg gcacacagtt cgtggtccag ttgctggtaa 1140
   aacagcttat caaatcgcac aaatggctgg cttgaaagat gtcgctaagg actgccgggt 1200
   cttgattgcg gagattaatg gtgtcggtat caagtaccca ttatccggcg aaaaattatc 1260
   accagttctg accgtttaca aagcggattc gcatgaagcg gcgttcaaac gggctaatga 1320
   attacttcac tatggtggct taggccatac tgctgggatt catacgaccg atgatgcatt 1380
   ggtcaaagaa tttggcttac aaatgccagc ttgccggatc ttagttaaca cgccatcttc 1440
   agttggtggg ttaggtaaca tctacaataa catggcgcct tcattaactc tgggcacggg 1500
   atc 1503
<210> 44
   <211> 922
   <212> DNA
   <213> unknown
<220>
   <223> lp_0026
<220>
   <221> startcodon
   <222> (478)..(480)
   <223>
<400> 44
   tgtgactact gagccgcgaa ccatgacgag tcacgattta aagttgagtg gtgtgcccat 60
   aaaattagca caagcgattc aatgctgcct tgtgacacag acgctgggtg gtgaacagtt 120
   gcgccttatt gaaaacttta cctaaatttt acaggaactt gatgattgtt agtacggttc 180
   agccgtatag tataattgta ctttttaata gcaccccgct attgattatg tattacgaag 240
   tactcatcga tccttaatga gacgtaggca cattagtgcg tggctttcct tgattggcca 300
   cgcatttttg tgcgcttata agcagaatgt ctgcgttgtt cagacgaggt gcttataata 360
   aaggtaaaca ctaaagcacc ttttcggatg gttttaaata gacgtcatca cgaaggtaat 420
   tagtgtattg gtggcgctgg ttattggcca gtgactgatt aattaggggg taattggttg 480
   taccaagcag tagtgttttt tgatttggat ggaacgttaa tgcaggacga taagacggtt 540
   gcggccagca gtgtgcgggc aatccagcaa ttacgagcta accacgtcct acccgtcatt 600
   gccacgggtc gcaatgtatt cgaagttcaa gatgtgatga caacgacggg gatcgattca 660
   atcgtcagtg cgaatgggag ttacgtcgcg tatcaagggc agtttttgag tgcgcacgaa 720
   ctggataagg agcttttgac cgatattact cattttgcca atcgccaaca gaatgcgcta 780
   gcttattata ataacgccgg ttttgcgtta agccatcgtg atgcattgac aaccagtaat 840
   tatcgggcac tcagacagac ggcccgggtc gcaccgcact tttatgaaca tcaacatatt 900
   aatttcttac tgacgtttga tc 922
<210> 45
   <211> 801
   <212> DNA
   <213> unknown
<220>
   <223> lp_0139
<220>
   <221> startcodon
   <222> (343)..(345)
   <223>
<400> 45
   ctgccgatga ttttcaatat acaggaacta ttcgttaatt tgaacggtta gttaaaataa 60
   aaagtgagcc gtctgcattt gtcaaggctc gctttttgca tacataaatg gcagaacttg 120
   tggtgggaat gagccgcttg aaaaaatgta atatttgtta aaggcaatgt gtttaattgg 180
   agaaaagtca tcaaatggtg ttacgctatg ggcagaaatt ggttgactag cgcaaatatt 240
   aagaaagcgg caagaaaaca aaaaagttca gttcaaacta gacaatcact atccaagttg 300
   tataatgtac ttgtaagcgt tatcagaacc taattaccta ttttgggggc gttatttatg 360
   gttaaaagta aagcaattat gattggtgcc gggctatcaa atatggctgc ggcggtctac 420
   ttgattcaag atggtcattg ggatggtaag gacatcacat tctatggtgt tgatatgcac 480
   ggtgccaatg atggtggtgc cacgactgat tttaccaatg agtattggaa taagaatcat 540
   ccgatggcta acacgactgg gtatgttgcc cggggtggtc ggatgctgaa ttaccggacg 600
   tacgttgact taatggattt attggaccgg attccatcgg taactgaacc ggggatgacg 660
   gcggctgaag atacgcgtga ttttgatgcg aaacatcgga cgtatgatat tgcccgcttg 720
   atgcagggtg gtaaaggcat tattaatgct ggtaagttag gattcaataa taaggatcgg 780
   actttgctga ctaagttgat c 801
<210> 46
   <211> 865
   <212> DNA
   <213> unknown
<220>
   <223> lp_0190
<220>
   <221> startcodon
   <222> (103)..(105)
   <223>
<400> 46
   atctaacaat tcgtcaattg atactgcatg atttatttac taataagtca catcccggca     60
   ccacgtcaac taacgaactt agatatgagg agggggatta caatgcttgc atacgggtat    120
   caaaaatttg ggggacctac cgtatttgaa gaactaccac aatcacagct gattccaaca    180
   gataatcaat tattaatcaa aactttagcc gttaacctaa ccgaggaaga ccgcttagaa    240
   cggctaggta ctatccaaag cgtcgcacta ccgcatattc ctgggcatga tgtcattggg    300
   aaaatcattc aagttggcgc cgctgtcact gacttggacg ttgggacagt ggtggctgcc    360
   cgcacgaacc atacttacgc ggaccaggtt tgtttagata gcgatctagc ggtgaccatg    420
   ccaactaatc ttgatccggt caccgccgtc agcttagtca ctccagggat tgtcgcttac    480
   aaaacggtgc gctactttgc ggacatccaa gccgaccaaa cggtgattgt caaaggcgct    540
   ggcagcccag tcggcttgtt aattattcag ttagccaaac gattaggtgc ccacgttatc    600
   gcgattgcta gtgaccgtta tgctaacttg attgcgacac tgggagtcga ccaattcgtc    660
   gactatcagc atcaaaatcc agttcacgtg ttagccgacc gcggtgacgt cgtaatcaat    720
   gtcgctatcg atggcgttgg tggcaaagat gacttagcaa tggccaaatt tgatgcgacc    780
   atcgcttcgt ttgcccgtca cttgccagcc accagtaaat caattcgttt ccaagcaatc    840
   caaccgaccg aagtgatttc tgatc                                          865
<210> 47
   <211> 1551
   <212> DNA
   <213> unknown
<220>
   <223> lp_0291
<220>
   <221> startcodon
   <222> (738)..(740)
   <223>
<400> 47
   cacgatttgt ttgagaccgc ccatattgat agttgcatag taatcgatag gcacgttgaa      60
   tagcttgcta acactagcct tggccatctt ggagccacca atattgtaag cggcgttgag     120
   tttttgaaca tttgtgtcct gatcaccgac catttctgct aatgaatcgc gtggaatgct     180
   ggtaatgagc gttttcttgg tctgcggatt gatcgtaacg accataatgg tgtcagaatt     240
   tcccttatca atacgaccgt ctgccccggt gtcaacacct agtagtaaga tgctgagtgg     300
   cttagtcgta tcgattttag tactgctgtt tcccgtatag atcgtcgatt ggtagcgcgc     360
   ctgtgcctgg cgaataatta agccaatcgc cacgaatgcg ataacgataa tgatgccgac     420
   cgcaattaac actggccgta attttgaaga tgctgaatgc cgcatgttca aatcctccct     480
   taattaaagg cgttaagatg gttccccgga taacgtcttt gtcttgaaat cactgtttct     540
   agcatacccg gtcagtacga tcccggaaaa tacttatttt gagtgtactt aatttaaaaa     600
   atagatacca cctaaaagaa ttagattgga aactgagcaa attaggagcg gtcacttacg     660
   gacagtatta cggcagaaaa cggattcttg gcttatactg acagtgaact taaatatatg     720
   aagtgaggtt tggtaatttg acagtctttc gcgcattttc taaccaagaa gttttagatt     780
   atttagcaac gcaagtcact gacgggcagg tcttcactga cgaggagacg ctgaaaaaat     840
   actctttcag tccgcggttg accgatgacg atagtggcct tgccttagcc tatgttgaag     900
   cacatacgac caaggacatt caaggaacgt tgaaaacggc ccgtaaatac cacatcgcag     960
   tgattcccca agaccaaaca acgagcactg ttatcggtgc ggacgggtta acggacagtc    1020
   tgattctttc aaccgccaag atgaaccgaa tcatcgaaat tagcaaggct gattccatcg    1080
   ccgtggttga acccggcgtc gttaacggtg atctggataa agaagcacgc aagcagggta    1140
   tgttctacgc gccggaccct ggttctaagc ccatctctgg gattggcggt aatgtggcga    1200
   caaacgccgg cgggatgagt acggtcaagt atggggctac taaggacaat gtcttaggcg    1260
   ttaaggtcgt tttagccgat ggtcgcgaag ttaagttagg cggtcggacg ctaaagcagg    1320
   cctttggtta tgatttaacg caattgatga ttggttcgga aggcacgtta ggaatcatta    1380
   ccgaagtaat cgttaaactg ttgccgattc cgctagggac cccgtgatgg ggcgtcgcct      1440
   ttttcgataa tatgacagcc ttggctaaag cagtcacggc gattcgaatc tccggtgttt    1500
   atccaacgat gctagaggca ttggatggca acacggttgc ggcactcgat c              1551
<210> 48
   <211> 1400
   <212> DNA
   <213> unknown
<220>
   <223> lp_0476 and lp_0477
<220>
   <221> startcodon
   <222> (1115)..(1117)
   <223> lp_0476 start codon
<220>
   <221> startcodon
   <222> (168)..(170)
   <223> lp_0477 start codon
<400> 48
   ctgctgggaa aagttcgaac ccgcgtaaac tttttgagtc ggctgtgttc caacaattgt      60
   ccctcagccc cggaggtcga aataggccgc acgttgctga cgaacaaaaa accaactaac     120
   ttgcacgttt cattatccta tgatggtatg ctacagatta agttgatttg tctaagccaa     180
   tgcaacggta ccatttttct agtataatta gtgatgatac ggacaacggt ggcggtttca     240
   tcatgagaca tccctcgtgc ccgataatag cggataacgg ggtggctttc ttgacggaat     300
   caacaattgc aactttgagt caaaaatacg aaccggcagc gatgctggat agttttgcgt     360
   atacgaacgc cttgctgtgg ttaacggcgg ctcgtcagca accaatcgtt catttctggc     420
   agttacagcc aaccgtcatt ctcggcttat tagaccaacg cttgccacgg ctaactgctg     480
   gcttagcaga tctacacgct gccggttatc agatcatgct ccgtaattca ggtgggctag     540
   ccgtagttgc tgaccctggt gtactaaacg tttccttatt tcttccagca actcgtgaac     600
   gctatccaat tacagcggca tatcaattga tggtcgacta cgttcaggca gtttggccac     660
   aattaaatat cacgactggt gagattacac gctcctattg tcctggtgat tatgatttga     720
   gtattgccgg tcgtaaaatt gccggaatgt cgcaacggcg cactgccgaa gcgctggtca     780
   tcatgctgta tgtcagcgtt gatggcgatc agatggcccg tagtcaactc attcagcgtt     840
   tttaccaagt cagcttagcc ggtcaccatg acgcgcggtt tccagatgtt gatccacaag     900
   tcatgacgac cgttgctgac gctttggcag cgccgactga cactaccgct acacaagctc     960
   gttttatagc catcctagct caacatggga cggtcgacac aacaagtttg ccgttagtca    1020
   ccgaagaacc tgaatttcaa gcacacttaa atcaagccta tcaacaaatg caacgccgac    1080
   aacaacgctt gcatcactga aagaaggaat taatttggcc tatcgccagc aactattacc    1140
   catggagaaa gtctttcgtg acccggtcca tgattacatt tatatccaac atcaagtcat    1200
   tctcgatttg atcaacactt cggaatttca acgactgcgc cgtattaagc aactcggtac    1260
   gacctcgctg accttccacg gtgcagaaca ctcccgcttt ggacattgcc tcggtgttta    1320
   tgagattaca cgccgtattt gcgataactt cgaacgtaac tacccgaccc aggcccctgg    1380
   tgacggtggt tgggatgatc                                                 1400
<210> 49
   <211> 657
   <212> DNA
   <213> unknown
<220>
   <223> lp_0907
<220>
   <221> startcodon
   <222> (106)..(108)
   <223>
<400> 49
   atgtaaattt gtttgaacat tggtctatca aagttacgat tgtgttaaac tgaaaagaat     60
   gtaatcggtt acatctgttt ttcaacccgc agacgcttat aataaatgca acttaaactg    120
   gaggtggcac cgttggtcaa tttaataacc attttaaccc atttagaaca aagtatcgtc    180
   ccactattaa ctacgcttgg tagttggtct tacatcttat tattcgccgt tatctttctt    240
   gaaacgggca tggtcgtttt cccatttctt cccggcgaat cattgatctt tctcgccagc    300
   acgatggctg caactcacag cgacgccctc agtattcaga ttctggtgcc agtattcttt    360
   gtggctgctg taattggcga cactgtcaat ttcgaaattg gccgccatct gattcgctta    420
   ccgtggttgc aacgccacct cccccaatcc aagcaacaac gcgcacaaca attttttgct     480
   aagtatggcg cgaaagctgt catgtttggc cgctttgttc cactcgtgcg gacctttgtc    540
   ccgctgagtg ccggggccgc taaaatgcca caaacgacct tcgctatctg gaacttagtt    600
   ggcgttggta tctgggtcag tgtcggctgt tcactcggtt acttctttgg ctcgatc       657
<210> 50
   <211> 491
   <212> DNA
   <213> unknown
<220>
   <223> lp_1969
<220>
   <221> startcodon
   <222> (413)..(415)
   <223>
<400> 50
   tccgtacctg cggccaattt atgatgcgct gtacgatatt tatggcgctg aacataccca     60
   gcgcttaatg gatcgtggta tcatcgaaat cgcaccgctg gcttatatgc gggggcggac    120
   gcttgaccat gcgttcgtca tcttggatga agcacagaat acgacgaatg cccagatgaa    180
   gatgtttttg acgcggctag gtttcggtgc caagatgatt gttaatggtg atatctcaca    240
   aattgattta ccacaccata ctaagagtgg cttgattcaa gccgaacaga ttttgggcgg    300
   cattagcaat gtcgcgttcg ttaagtttac ggcggatgat gtggtcagaa atccggtcgt    360
   tgccaagatt attacggcgt acgatcgtgt ggaacaacgc tagggggcac ttatggattt    420
   agaattatac gaccaaacaa caacgggagc gcagccagaa cagttgcaat tgattcggga    480
   tttaattgat c                                                         491
<210> 51
   <211> 1688
   <212> DNA
   <213> unknown
<220>
   <223> lp_2337
<220>
   <221> startcodon
   <222> (957)..(959)
   <223>
<400> 51
   ggtattcaag ataatagacc ccctgccggg ccatcatagc tagctgccca ttactccgaa 60
   gccgaactgg cccgtcaaaa tcattgacag acaacaactg ctgttgacga acagccgccg 120
   tcaacttggc cacgtagcga tcaattgacg tatagtctcc cgtcacttga ctgtagcgac 180
   catcgggatg ttgaacacta ctgcgctgtg gacgactcgt agtatccgta acggctaggc 240
   gtggtgaagc gccaaataaa tactgaatca gccagttcat gcgcaccaat ccctgagcga 300
   ctttcagata aattgcattg cgaaagtcaa cataactgtg atactgctga tggaaagtct 360
   cagtatataa acgggtgaat aaagcttcat tcaagctcat attcacgtga ctaccagtcg 420
   tcattaatcg ctgtaactca tacttacgag ctaagtcgcg acgacgctga tagctgactt 480
   gatcaacatc agccagtggt acgttcgtta gatcatccgg caacacaggc gtactcgaca 540
   gtggccaaag tcgttcgtcc gcatcgagtg accgccgtgc tgcggtattc aagcccttca 600
   gatacgccat caatgcagga atgtcacgac gcaccggcgt agtaaacttt aaagcacttg 660
   cggaaaagcc cggacgtaac tgatgatttt gttgcgctga tccaaacgct tgcgggtgtg 720
   ctgtcgtcgc taagtgacca tgggcgtcca cccggtgcat ggtgacctct agtcctaaat 780
   tagcctgatg aacgagtggg actaagtgat actgtacaat tgccttacca acggcatcta 840
   attccataat gatcaatcct ttataattat tttgttcgtt aaactgatta ttcggtgtcc 900
   ttcattgtaa atgctataat agttgggact agttttgcaa ggagggattt caaacgatgc 960
   aacaaccatt agcctaccgg atgcgaccaa aaacgctgaa agacgttgtc ggtcagcaac 1020
   aattggtcgg cccaggcaag atcatcgccc ggatggtcaa agccaaacga ctatcatcaa 1080
   tgattctata cggcccacca ggtactggca agactagtat cgcgagcgcc attgccggtt 1140
   ccaccaagta tgcttttcga atgctgaatg cagctaccga cagtaaaaaa caacttcaaa 1200
   tcgtagccga agaagccaaa atgagcggga cggtcattct tctgctggat gaaatccacc 1260
   ggttggataa aactaaacaa gactttttat tgcctcactt agaaagtggc cgcatcattt 1320
   tgattggtgc tacgaccgaa aatccatata ttagcattaa tccagccatc cgtagtcgaa 1380
   cacagatctt tcaagttttc ccgctcactg acgatgatat tcagcacgcg gtggaacgag 1440
   ccctcgctga cgaaaaaaac ggtcttggca gttaccacgt tgatttagca cccgcggcca 1500
   tgcaccacct gtgcaccgct accaacgggg atttgcgcag tgccctcaat gggttagaat 1560
   tggccgtatt atcatcgtct ccggccgatg acggcaccat cgccatttca caacaaacaa 1620
   ttgaagaatg cttgcaaaag aaagccttat ccgcagataa agatggtgac gcccactacg 1680
   acgtgatc 1688
<210> 52
   <211> 758
   <212> DNA
   <213> unknown
<220>
   <223> lp_2507 and lp_2508
<220>
   <221> startcodon
   <222> (615)..(617)
   <223> lp_2507 start codon
<220>
   <221> startcodon
   <222> (204)..(206)
   <223> lp_2508 start codon
<400> 52
   tgcggacctg cgttagccac attgcggtta gatgcggatt attgatgaag agggtactcg      60
   cagccacccc tgagataccc cacagaatac aggcactact agcgagtaag acgccccgta     120
   aatgattttg ttgtgtcaaa ttacgactcc tttctgaaaa taataagcaa ttttcattat     180
   agcaaaggtg cgtctgggaa cctatgttta tgaggaatct cttattattc tcatgggtta     240
   cttcatcaaa agtaacgggt gcgtttcaga agaactcatg ctatgatatg aacaatcaaa     300
   cgcgaataat gatgcgtcta acggaggcta taaagatgaa agcaattatt accgttgtgg     360
   gacaggacca agtgggaatt gtcgcaaaag tagcgaacga gttagcacgt ttaaaaatca     420
   atattgtcga tatttcgcaa accttgatgg atcataattt taccatgatg ctatcggcgg     480
   aatgggatga ccagcaatta agttttgcgg cagccaaagc ggcgttagaa tcgctcggtg     540
   aggcatcaga gttaacgatt cgaattcaac ggcaagcggt ttttgacgca attcagaagt     600
   tataggaggc taccatggaa agtcgttcaa ttttagaaac aattcagatg gtggccgagg     660
   aaaatcttga tattcgtaca atcacaatgg gcatttcgct ctttgattgt gttgatagtg     720
   acggtgagcg ggcccggcaa aaaatctatg ataagatc                             758
<210> 53
   <211> 1226
   <212> DNA
   <213> unknown
<220>
   <223> lp_2713
<220>
   <221> startcodon
   <222> (938)..(940)
   <223>
<400> 53
   ttccttagag agctgattcc agaattggag gttcaggtcc agccagacgg atgggtcttc      60
   cttgcgaaat gagcgcattg agcatttgag cgtgtcgata tcttttaaaa gccactgaat     120
   gtttttaaga ttggctagac ttgcaccgac gttatcgatt ccaaactgca tgccgtaacc     180
   gcgggcttca cgaattcgtc gccgaaatag gcgtttattg atacgacgcg gttggtattg     240
   aggtgtgtac tcaattgcca agtgcatcgg ttcgatgttc gcaattgccc agcgaacaaa     300
   ataacggaat tcgggactga tgatttgttc gtattctaaa ttgattgata acgtaatcgc     360
   ttcatcaggt agcgccttaa aggtatcttc aagcaggaag atcacccgct gaagtggaag     420
   tgagtcaagt tgtggtggta atgaccacga accatcagta ttttgttgac gcaggagaca     480
   ttcatagccg gtagtgcggc cacgaaaatc gacttgtttt tgaataaagt agcgtagatt     540
   aaaatcatca ttttcaaggt aattgttgga acgtttgcgg gagtaccaat aataagtacc     600
   aatcgtcagg atgaagatga ccgcaattga gcctgtcaac cacagtaatc ctatttctaa     660
   ttgtgctaga ctcatctaat ataatctccg taagaaaaac ttatggtaac aggaaagacg     720
   actaaccgtc gtagttgaca gtgtcaaaca ttttgacatt ataggcctaa ttttgcacag     780
   tttcaacaaa aattatctct tgataaatag aaatgatttt tgattaacgt taatcagtcc     840
   tgtcttatga acgaatcacg caaccgcaca tcaacactgt ttagccaact aaactaccag     900
   tgattgacgg ggttttagtg gtcgcttata atcgataatg actttgacgg ataatttaat     960
   aagtatagaa gagggttatc ataaaatgaa aattgtttta acgggttcaa tcggtcgtat    1020
   taaccagacc ttggtaccta agctgaccca tgctggtcat gaagtgacgg tcatcagtca    1080
   tcaagcaact cgcagtagtt tgataaaggt ttttcgcgca acccctgcca ttggtcgact    1140
   tgatgacgaa gcgtttttga ccaagacgtt catgggcgct gacgtggtat acctcatggt    1200
   aactggtata gcggcgagtg atgatc                                         1226
<210> 54
   <211> 710
   <212> DNA
   <213> unknown
<220>
   <223> lp_2718
<220>
   <221> startcodon
   <222> (225)..(227)
   <223>
<400> 54
   ccgaactgat attggcgcta aggggatgac ggcgacccac gattagcgct tgacattcgg      60
   tgagcaccat tctatgctgg aaatattact taaattcgtc ataaaagaat aatggttggt     120
   gttggcagtt aagggctgac ccaaacgttg aaatcgtcct ttttaagcag taggggacga     180
   tttttatatt ggcagaatgc aattaatttg gaggctaaac gggcatggaa tttggcacat     240
   tgatgatgat ggttaggcac cgtgtacatg gttggcaaat ggcaatgcgg tctcaaaaag     300
   ggttgcatcg gggcacaaca acgttgaata ttaacgcggc gggacttaaa cggatggagg     360
   acgctgcttt tatgcatcat attcggaaac cggttgataa gtcgcataat ctaaattggt     420
   ggcactttaa ctttttctgg gggctacaag gcatcaccca atccgggata ggtttggtgc     480
   cggcgctcgc gttgaccggt aaaatcagtg gcttcaacca tcctgccgtt gcgcattttg     540
   aattcatgcg caatcaaatc agtggtggaa ctgtggataa acaaattttg gccgctcccg     600
   agctgtgctt ggcagaactc aaacggtccg ataatgcgcg cgcacttaat cgagtctatc     660
   aatcagatac ggaaatggtg catgacttgg ccgcagccta ccaccagatc                710
<210> 55
   <211> 1145
   <212> DNA
   <213> unknown
<220>
   <223> lp_3312
<220>
   <221> startcodon
   <222> (1062)..(1064)
   <223>
<400> 55
   ataacatcat cagtcaattc accatcacca atgtgggcaa agtcattagc tttgtcttcc      60
   attaaacgat cgatcccata aacagcgacc cgtggaagat caggaatgag acggccacgt     120
   gcgtatgcat ctggtagtcc ggtgatgatc ttgtagtgac gtgctttccg catgtcagga     180
   gtataaacat cgaagacgcc ttggttatga gtcttgcgat attcagtgaa aatcttgtcg     240
   ttttcttcat caggggtata accgtatgat ttcaaagcgt catcagccat tcgaatacca     300
   ccaaatggca tgaaggcccg cttcaaaggc ttgtcagtct ggagaccaac aatcttttcg 360
   agatcttttt gaatataacc agggccgtgt gaagtaatcg ttgcaacgac tttagtatca 420
   gcatccaaca caccaccagc ggcacgttct tgtttcttta attctaagac tttgtcattc 480
   aaggtcttag tagcggctgt tggtccggcc aggaagcttt cgtcgccatt gtactgtgtg 540
   aagttatttt gaataaaatc acgaatgttg atttcttcct gccagtgccc gcctttgaaa 600
   cctttccaag ctgctggttt tgttgtcttt tctgatgtaa tcattttctc gcctccataa 660
   tttgaatcca atcaaatgat tgataaaatt ctatatttga atcataacat tttttgctgg 720
   atttgtaacc gctttcaggt gatttatttc acgtttgggt tatttttttg caatctatta 780
   ttgaaaagtt gactattaac gttgatataa cagtacttac atagctagat aggcattggt 840
   aattcactta attatattta gaaaacggct aaaaacaaga ctcgttttga tatataagtg 900
   gaatattgtc cataatgtaa caagtgttta actagttaat taataaaaag tgcaacgagg 960
   tgtggagggt gttaaaagcg tttgccgttg attttatgag gaagttcgtt atcataaaga 1020
   agataagcag aaactagtga atgtagtgaa aggtgggcgt aatgcggatg ctcgaaaaaa 1080
   ttgaacaata tattgacagt tgtattgaag ctggcgacat ttatggggcg tcgtttagct 1140
   tgatc 1145
<210> 56
   <211> 744
   <212> DNA
   <213> unknown
<220>
   <223> lp_0118
<220>
   <221> startcodon
   <222> (342)..(344)
   <223>
<400> 56
   ctacagttgg gtgtccagtg gcatccggaa atgatgcaac aggtaaactc catgcaggcg 60
   cggttatttg cggcgtttgt gcgggcttgt gtggcgtaga tagcattgac tagcaaatta 120
   ataaaatagg cgtggttatt ctgaattgta ggataaccac gtttttaatg tgcagccgca 180
   aaactaatga atgtttcacg tgaaacatct gaaatggtgt tggcaactag ataactaatc 240
   caagatattg taagctgacg tgtaaaacaa ttcgtaaagc tgttaatact ggcaacgctt 300
   tcgtataatg gaataaagtt attgttaagg gggtgaccgc tatggaacga attgaagctg 360
   gcatgagcgt aatgacgttg aagaagcaga cctatcgggt tatcgctgtg attaatcagg 420
   ggcaagaact gttagttcgc gcccacgaac cagaaacagc tgacattgaa ataattccag 480
   tgaccacagt tgttcaagta actgaacaaa attcaacagg cgatgcagcc gtcgcattgg 540
   atacagtcac ggtcgttggc gaacggtatg tcgatgatat tgtggcaacg ttaacgacgc 600
   tcagagtggg tatggcggtg ttgctccagc gagaaagtgg caatcaatac gatgataatg 660
   ccatctcagt gtggacgttg caacatgcta agttaggcta cattgcccgg tatcagaacc 720
   agccgtacgc gacactgatg gatc 744
<210> 57
   <211> 832
   <212> DNA
   <213> unknown
<220>
   <223> lp_0292
<220>
   <221> startcodon
   <222> (685)..(687)
   <223>
<400> 57
   gatctttaag cttgataatg gtggtcagga tagtatggca gtcgttaaga agatactggc 60
   cgaacagcaa gccaacgatg ttaagattac gaccgagcct aaggaacagg cagcgctaga 120
   acaattacgg cgcgatatgc tgccggcagt ttttgctggt cagaatcata ttatggaaga 180
   tatggccgtg ccattatcgc aattagcccc attaatggac tatattcaag aactatccaa 240
   gcggttagac gtcgaaatct atacggctgg acatgctggc gatggtaatg tgcacccgac 300
   cttagtgtgg ccaacgtcgg taaccgaagt gcctgaaaaa atcattattg cgctacaaga 360
   aatgtttaag aagacgctgg cacttggtgg gacgatttcg ggtgaacacg cagtcggaat 420
   gttgaaaaat caatggaaca atgccgaatt aggcgaagat gttgatatgt tgcaacatca 480
   gatcaaagca ctctttgacc cgatgaatat tttgaacccg aaacgtaaaa tcaattaacc 540
   caacgattac aaagcgctta agttttgata gcagttgttg gtcagatatt gcgtaacttg 600
   gtcatcgtgt cttattattg agatagtccc aaaaatgagt tgttggttaa tgagtcacta 660
   agaatgtgca gtgtggatta accgatgcga aggaggcccc aagtgatacc taagagtgca 720
   cgaatccgaa ttgaatttga tgccgaagta ctccgaaacg agatttttga tgtgcatgat 780
   ttcgccagtt ttcaaagctt ctggcagcga tttgagaatt tcttggccga tc 832
<210> 58
   <211> 234
   <212> DNA
   <213> unknown
<220>
   <223> lp_1788
<220>
   <221> startcodon
   <222> (34)..(36)
   <223>
<400> 58
   tactggcgca caactactaa ggaggactga accatgcaac cagtcgaaaa acatcgctta      60
   caacaaaaat acaaacttgc cccaacttat tacggcgtca ttcaaattac caacgaacgc     120
   aatcataaaa tttggattga cacagtgccc aatttacaca accgctgggc cttttatcaa     180
   ctaaatctta ataaaaactt ttaccggtcc aacccgttac aggctgattg gaat           234
<210> 59
   <211> 974
   <212> DNA
   <213> unknown
<220>
   <223> lp_1872
<220>
   <221> startcodon
   <222> (885)..(887)
   <223>
<400> 59
   gatctgtttg acgggttagc agatatggaa caaggaatct tacgcgccgt tggagtggct      60
   gaagaacggt ttcacgaaga tgcgttgcgg atgatgcgtg ccgttcggtt tgcaagtcag     120
   ttaggcttta ccattgaatc gcagaccgaa caagcgattg ccgataatgc ggcgttgttg     180
   gcgaaaattg ccgtggaacg gacgcgtgtc gagtgggaga agctgctgat gggtcagcac     240
   ccagttgctg gtttgaccgg cttgctgacc actgacttgt atcgttatat gcccgcaatg 300
   gctgatcaag aagcgatgct gcggcagtta atggcacttc cagcttggca tttgccgagt 360
   attgaaagta cttggacctt gcttagctgg accatgcaac ggactgacga agcggctgtt 420
   cgccagttgt taaagacgtg gaagacgagc aatgagctga ttaatcacgt gacggccgct 480
   attaaagcgc tgatggcgtt aaaacacaac ggtcagttaa ctgcgcagga gaacttttat 540
   acgggcttag aggcgctgaa aacggctaat caggtggcta cgattttagg ctttggccag 600
   gaccaagcac aattggtgca atcttatgct agcttaccaa tccatgacaa gcatgaattg 660
   gccattaacg gtggcgattt attgaaagct aaattggtga cgcctgggcc aatgatgggt 720
   caaattctag cggcttgctt acaggccgtg gtgatgaaac aggtcccaaa tcagcaggat 780
   gctttgcttg attttgcaag gatggtagcg gattcaaaaa accattgata ttttctatga 840
   atctgttacg attaaagtga aactaattca atgaggtgat ttgaatggaa cgagcattag 900
   aagcattgaa atttcatttt cggaatggtg acacttggac agttcatcat caagatatct 960
   ctgacctgtg gatc 974
<210> 60
   <211> 711
   <212> DNA
   <213> unknown
<220>
   <223> lp_2112
<220>
   <221> startcodon
   <222> (243)..(245)
   <223>
<400> 60
   caataatggc taaaaaccac ccaaatcttt ttttcatagg tattctcctc gttttgtaat 60
   ttacaagaat aaagtataac cgcatatgat tatttatgca agtatttttg aaaatttctt 120
   tattttatgc ataaataagt cgtttataca cgttttagcc cattgattat gcataaacga 180
   cgaataactg acaaattgtc ggaaatttgc tatgataggc gttaatcgaa aggaatgatt 240
   ttatgagttt aaaagtacag cgtcaagata accttgataa gctattcgac aagtttgcca 300
   ttggtcctga cgataaggat aaactcacca ctaaagcggt tcaggatact attgacgaac 360
   atgctaaacg caaagaaaat aaggcagctg ataaaaagga cgcccataaa tagcagtcaa 420
   aaaaaacgca atggcagtta gccatcgcgt ttttatttga ataactattt agcaaatttt 480
   agtcccgatt gtttgcatct tttcgcgccg ctctgcttcc gtttctggtg aaacatcgcg 540
   ccaatcaaca acagccaaag caccgtcgag catcccgcta tcatccttga gcgctaaagc 600
   attatgccag cgagaaatgt tgaggccgtt ccccaagtag gcttcatcag ccggtgcaac 660
   gtgcatcaca taaccagcat tattttctaa tggcgtggta tagtcgggat c 711
<210> 61
   <211> 1660
   <212> DNA
   <213> unknown
<220>
   <223> lp_2584
<220>
   <221> startcodon
   <222> (1208)..(1210)
   <223>
<400> 61
   atcaagggcc gtgaactggt ggcacaactt gaaaagcaac tcacacaatt tccaattgac 60
   attcaattgg cgagcccggt gacggatgtc acgggagcga tgggtgattt cacgattacc 120
   acggcgagtg gacagcagtc acattccaaa gcgattatcg tagcgactgg gagtggggct 180
   tttgaacccc ggcgcttagc agtcgataat gcggctgaat ttgagaataa gcaattattc 240
   taccacattc caagtgtcaa gcaatttgcg gatcgaacgg tactagttgc gggtggtggc 300
   gactcagcca tcgatatggc cttgatgctt gaacccgttg ctaagcacgt ttatatcatg 360
   catcggcgtg atcggttccg tgggatggaa cacaatgtgg acttgttaaa agcgtcttcc 420
   gttgaaatca agacgccatt tttgattaag caacttgcag agacggctac cggacagtta 480
   cagctgacga tgaaagaggt tcgcggcaca actgaagaaa cattggccgt tgatgatttg 540
   attgttaact atggctttat cgcggataat aaagtcatcc gaaactggca cgtgacccca 600
   accatggccc accgactcat caccgttgat acggagatga ataccgacgt acctgggatt 660
   gcggctattg gtgatacggt gacttacgcg ggcaagttag gcttgattgc cagtggcttc 720
   ggggaagctc ctaacgcggt caatcagtta atgatgacct tgtatcccga acgacggagt 780
   ccactgcata gtacgaccgt gtttgaaaaa atgtaagttt aaaagagctg cgatataact 840
   ggggctatac aagcaaatag gacgagaaac cgaatcttgg cttctcgtcc tatttgcttg 900
   ttcctttaag aacccgcttt attggcggcc tgttgtacca tgaagtggta gaaaggcaaa 960
   tgtttttttc aatctagata gttttggtgt gtcttaaaat aaaaacacaa ccggtcgctc 1020
   cggctaaact tttacaatgt ataagtttgt acaaaaaaat acacaaaatt tactgaaaat 1080
   tgataacgat aaagaaacgc tgttgttaag ataaattcgt aacatactgc ctgttgttaa 1140
   cgtagaattt gagaggatga ttctgtttat ggcaaaaggc tccattattg atggtgtgtt 1200
   agtttagatg attagatttt atatgtcact aagtgttgtg aagcatacga tactttattt 1260
   gtgttttatt tttctattac tgtatttaaa aaagcgttat ccagacaatg taattatcgg 1320
   ttttttttta aaaatacggt ggtgttttat taatagtatg cttcatttac attttatcgc 1380
   aatttggatt gccggttacc gcaatcgatg ttccaatagt tcttggaatt ttgggtattt 1440
   cggttgttgt gttgagacac aagcaaaagt aggacgaact gctaccaatc tgttgcttac 1500
   ggagtcatga taactcatgg ctctttctgg tttttatcta atattgacgt ccctgataac 1560
   gtactgtcat caaaggactt catggttcag accctgtttc tgggtcattc catatgagca 1620
   cctaattggc ggtaaaagta ttaaaaatca gtggctgatc 1660
<210> 62
   <211> 1395
   <212> DNA
   <213> unknown
<220>
   <223> lp_3246
<220>
   <221> startcodon
   <222> (1001)..(1003)
   <223>
<400> 62
   cgacttagga ataatcgggc aaaaggccgg attgaacggg gcctatatgg ctgatttctc 60
   tgacaagtca cagttctatg gcattctgga agaagtccca caacattaat accggccagg 120
   gggttgaggg ctaccggtcg tgtaattgct gattggtatt ccgaaagcgt cctccgattg 180
   tgatgctgat taaagcgtaa gtaagaccag cttatcgtat aaattaaaag gaacacctca 240
   ggctgtgata gtccacttaa ggttgacagg gactaataca ggaaaactga aagtgttcct 300
   tttgttttag gataaccagc gcgctaaata gccggttgcc attgaactgg ttctattcac 360
   tgtgcgtcaa tttcaatcgc tttaaatcag ctgttttagt tacattgagt agtttctggc 420
   agtctgggca taatccatag acttccatat tgtgacccgt aaccaagtac ccagagagtt 480
   ccgccgcctt ggcttcgaca ctgcggaggt cgggttcgaa aacgtcagta attttaccac 540
   aattctcaca aacgacgtga taatgtggtc gaccgaaaaa gtcatagtgg gtgccagcat 600
   cgccattttg taattcaatt acgattccta agtcgacaaa aagttttaac gtattgtaaa    660
   ccgttgccaa gctcaggtta ggtaattgag ttgctaattc ctgatagatg gtttcgacgg    720
   atggatgatt atggtgagta atcaaataat taagaatgat ttgccgttgc ggcgttacac    780
   ggattttatg gtcctttaat tgctgcaagg cttgggtcaa catatcttct gccacgacgc    840
   ggttcctcct tttatcatat tcacccttat taaaccacac tttttagaag gattccaatt    900
   tagaaacatt actagataaa acttttgaca aaattaatta acgaaatagc tgaaaaggct    960
   ttactggggc cgaagacgtg agtaaactaa cgataattaa gtgcgtgttg atgggggtgg   1020
   caaaatgaca caattaattg taccagcatt accggttgag cgggtccaat acgaacgcgc   1080
   atatgtactt ggccttgaag caatgatgaa agttatccgg caacttacga ctacggaaaa   1140
   aggacgacag gtacccacta atcagttccc agtatttgcg gcgagctatg atgttcccca   1200
   agcgttggcc caagttgcag tcctaaaagc cgctactact cggttgaagg ttcgaccgat   1260
   tcagttgacc gtgacccggc agtacgtgac gttacgattc caattacaag cagccgctgg   1320
   acaaccagtc tgggtgccgt ccgccggggc actatggcgc ctatatgatg gctttttaag   1380
   taatttagtg gggat                                                    1395

## Description

### Field of the invention

The present invention relates to the site-specific production of biologically active substances by recombinant micro-organisms in the body of a human or animal subject. The invention provides transcription regulatory sequences, which are specifically induced in the gastrointestinal tract (GI-tract) of subjects and methods for their use for *in vivo* site-specific expression of nucleic acid sequences encoding biologically active substances.

### Background of the invention

Harmless or even beneficial micro-organisms such as lactobacilli, enterococci, or other common bacteria, such as *E. coli* commonly populate human and animal body cavities. For example, many strains of *Lactobacillus,* a genus with a longstanding tradition in food industry, are present in large numbers on gastrointestinal (GI) mucosa together with other intestinal organisms (Mitsuolca, 1992 in The Lactic Acid Bacteria in Health and Disease, Volume I., E.B.J.B.Wood, Elsevier Science Publishing Ltd. pp 69-114). These beneficial micro-organisms are believed to play an important role in human and animal health by interfering with the invasion and colonisation of potential pathogens of the GI-tract. One of the mechanisms by which interference is believed to be achieved is competitive exclusion of pathogens by the beneficial micro-organisms through occupation of common receptors on the mucosal surfaces of the GI-tract and other body cavities such as mouth, nasopharynx, vagina and rectum.

The natural occurrence of these non-pathogenic micro-organisms in the various body cavities opens the possibility of using them as delivery vehicles for biologically active substances to those areas of the body. To this end the beneficial micro-organisms could be genetically engineered to produce a desired biologically active substance, such as a protein, an enzyme or reaction products thereof (Elmer et al., 1996, JAMA 275: 870-876; Steidler et al. 2000, Science. 289:1352-5). The engineered micro-organism is then introduced into the desired body cavity where it will produce and deliver the desired biologically active substance(s).

Indeed, for some years now lactic acid bacteria are being developed as delivery vehicles for vaccines, as reviewed by Wells et al. (1996 Antonie Van Leeuwenhoek 70: 317-330) and Pouwels et al. (1998 Int. J. Food Microbiol. 41: 155-167). The ubiquity of lactic acid bacteria in the mammalian GI-tract and their capability to adhere to squamous epithelial cells and mucus makes them useful as vehicles for vaccine delivery.

WO 99/47657 discloses the use of a transformed *Lactobacillus* species, in particular *Lactobacillus reuteri,* as vaccine delivery vehicles. A gene encoding a factor involved in bacterial aggregation, referred to as agg, is disclosed in WO 99/47657, as well as a gene encoding a factor involved in the adhesion of *L*. *reuteri* to mucous membranes, referred to as muc. WO 99/47657 further discloses fusions of agg- and/or muc-genes with genes encoding exogenous antigens to be used for vaccination of animals. Lactobacilli transformed with such fusion genes are said to be capable of provoking a desired immune response to the exogenous antigen and, therefore, useful as oral vaccines.

US 5,821,081 suggests using lactobacilli transformed with sequences encoding antiviral proteins referred to as cyanovirins, to deliver the antiviral proteins in the vagina for protection against viruses like HIV.

US 6,605,286 and US 2002019043 describe the use of *Lactobacillus lactis* for delivery of IL-10, IL-2, IL-6 and other biologically active peptides to the GI-tract.

However, none of the art teaches transcription regulatory sequences that are capable of driving efficient gene expression in lactobacilli or other non-pathogenic or enteric bacteria under conditions that occur while these bacteria are passing the vertebrate's GI-tract. In particular, transcription regulatory sequences which are specifically induced during passage of a micro-organism through the GI-tract, while not being active under non-inducing conditions such as outside the subject's body, are not available in the art. Such transcription regulatory sequences are extremely valuable tools for GI-tract specific expression of nucleic acid sequences and are one embodiment of the invention. One species of lactobacilli, *Lactobacillus plantarum,* has a long tradition of utilization in industrial and artisanal fermentations and is 'generally regarded as safe' (GRAS; Adams and Marteau 1995, Int. J. Food Microbiol. 27:263-364). Next to the occurrence of *L. plantarum* in human and animal diets, this microorganism is frequently encountered as a natural inhabitant of the GI-tract (Ahrne et al. 1998 J. Appl. Microbiol. 85, 88-94). The complete 3.3 Mbp genome sequence of *L*. *plantarum* WCFS 1 has been determined (Kleerebezem, M. et al. 2003 Proc. Natl. Acad. Sci. USA 100, 1990-1995) and is available to the skilled person. Strain WCFS1 has been deposited under the Budapest Treaty at the CBS, Baarn, The Netherlands, on 27 June 2003 and was given accession number CBS 113118. The strain WCFS1 is a single colony isolate of strain NCIMB8826 (available at the National Collection of Industrial and Marine Bacteria, Aberdeen, UK) which reaches the ileum in high numbers and in an active form compared to other lactic acid bacteria strains and is detectable in the colon (Vesa, T. et al. 2000, Aliment. Phannacol. Ther. 14, 823-8). Intriguingly, genome sequence comparison revealed that the closest relatives of *L. plantarum* include *Listeria inocua* and *monocytogenes* (Kleerebezem *et al.* supra), which also naturally inhabit and persist the human GI-tract (Glaser, P. et al. 2001, Science 294, 849-52). Despite the availability of the complete genome sequence of *L*. *plantarum* it is not known which genes are specifically induced during passage of the micro-organism through the GI-tract. Also, it is not known whether traditional methods for identifying genes that are highly expressed *in vivo* as compared to laboratory conditions can be used to identify *in vivo* expressed genes in food-grade bacteria such as *L. plantarum,* as these methods have so far mostly been applied to pathogenic bacteria, such as *S*. *typhimurium, Listeria monocytogenes, Pseudomonas aeruginosa* and *Histoplasma capsulatum,* in order to identify genes involved in pathogencitiy.

The three main strategies developed and used in pathogenic micro-organisms for the identification of genes that are highly expressed *in vivo* during infection as compared to laboratory conditions are selective capture of transcribed sequences (SCOTS, see Dozois, et al. 2003 PNAS 100, 247-52; Hou et al. 2002 Infect. Immun. 70, 3714-26; Daigle, et al. 2001 Mol. Microbiol. 41, 1211-22 and Liu, S. et al. 2002 Appl. Environ. Microbiol. 68, 1697-705), signature tagged mutagenesis (STM, for reviews see Mecsas, J. 2002 Curr. Opin. Microbiol. 5, 33-7 and West, N. P. et al. 2003 Trends Microbiol. 11, 338-44) and *in vivo* expression technology (IVET). The original IVET technology involves a tandem set of promoter-less reporter genes which were used to identify promoters that are specifically switched on in S. *typhimurium* during infection (Mahan, M. J. et al. 1993 Science 259, 686-8). This set-up has been modified in several ways and auxotrophic markers, antibiotic resistance genes and induction of site-specific recombinases have been used to trap promoters specifically activated during infection of several pathogens (for reviews see Angelichio, M. J. & Camilli, A. 2002 Infect. Immun. 70, 6518-23 and Mahan, M. J. et al. 2000 Annu. Rev. Genet. 34,139-164).

A recent study describes the use of IVET to identify *Lactobacillus reuteri* genes specifically induced in the GI-tract of mouse treated with the antibiotic lincomycin (Walter et al. 2003 Appl. Environ. Microbiol. 69, 2044-51). This study identified three *Lactobacillus reuteri* genes, which are induced during colonization of mice on an antibiotic-containing diet. However, continuous antibiotic administration is required during the animal experiment and will dramatically disturb the intestinal microflora natively present in these mice. Moreover, these experiments were performed in nonconventional, *Lactobacillus* free mice. These factors indicate that the GI-tract conditions encountered by *L. reuteri* in these experiments differ significantly from those in a conventional mouse. Thus, although in this study a food-grade bacterium was used, the artificial model applied and the method used were not suitable for identifying transcription regulatory sequences, which are induced in a conventional, undisturbed GI-tract.

A modification of IVET is the so-called resolvase-based IVET (R-IVET) strategy (Angelichio and Camilli 2002 supra), which makes use of a promoterless resolvase gene, such as *tnpR.* A major advantage of R-IVET over IVET is that no selection pressure is exerted on the micro-organism. R-IVET has also to date only been used in pathogenic micro-organism, and it is not known if it can be adapted to identify *in vivo* induced genes from food-grade bacteria.

The present inventors have for the first time used an R-IVET strategy in a non-pathogenic, food-grade lactic acid bacterium, *L. plantarum* strain WCFS1, to identify genes expressed *in vivo* during passage of the bacterium through the GI-tract. 71 transcription regulatory sequences have been identified that are specifically induced *in vivo* during passage of *L. plantarum* through the GI-tract of conventional mice.

### Description of the invention

### Definitions

The term "LPXTG-like anchoring motif" refers to a cell wall anchoring region commonly found in surface-anchored proteins of Gram-positive bacteria. These motifs can be used to attach proteins or hybrid proteins (e.g. covalently) to the cell surface of Gram-positive bacteria, as described by Leenhouts et al. (Antonie van Leeuwenhoek 1999, 76(1-4):367-76). The definition as used herein encompasses the LPQTNE motif found in *L. plantarum* surface anchored proteins. The *L. plantarum srtA* gene encodes a sortase, which is involved in maturation and anchoring of proteins comprising an LPXTG-like anchoring motif to the cell wall (Navarre and Schneewind, 1999, Microbial Mol. Biol. Rev. 63:174-229).

The term "gene" means a DNA fragment comprising a region (transcribed region), which is transcribed into an RNA molecule (e.g. an mRNA) in a cell, operably linked to suitable regulatory regions (e.g. a promoter). A gene may thus comprise several operably linked fragments, such as a promoter, a 5' leader sequence, a coding region and a 3'nontranslated sequence comprising a polyadenylation site. "Expression of a gene" refers to the process wherein a DNA region which is operably linked to appropriate regulatory regions, particularly a promoter, is transcribed into an RNA, which is biologically active, i.e. which is capable of being translated into a biologically active protein or peptide. In one embodiment the coding sequence is preferably preceded by a nucleic acid sequence encoding a secretion signal, so that the encoded protein or peptide is secreted out of the cell. The coding sequence is preferably in sense-orientation and encodes a desired, biologically active protein or peptide.

A "chimeric" (or recombinant) gene refers to any gene, which is not normally found in nature in a species, in particular a gene in which different parts of the nucleic acid region are not associated in nature with each other. For example the promoter is not associated in nature with part or all of the transcribed region or with another regulatory region. The term "chimeric gene" is understood to include expression constructs in which a promoter or transcription regulatory sequence is operably linked to one or more coding sequences.

A "transcription regulatory sequence" is herein defined as a nucleic acid sequence that is capable of regulating the rate of transcription of a (coding) sequence operably linked to the transcription regulatory sequence. A transcription regulatory sequence as herein defined will thus comprise all of the sequence elements necessary for initiation of transcription (promoter elements), for maintaining and for regulating transcription, including e.g. attenuators or enhancers. Although mostly the upstream (5') transcription regulatory sequences of a coding sequence are referred to, regulatory sequences found downstream (3') of a coding sequence are also encompassed by this definition.

As used herein, the term "operably linked" refers to a linkage of polynucleotide elements in a functional relationship. A nucleic acid is "operably linked" when it is placed into a functional relationship with another nucleic acid sequence. For instance, a promoter, or rather a transcription regulatory sequence, is operably linked to a coding sequence if it affects the transcription of the coding sequence. Operably linked means that the DNA sequences being linked are typically contiguous and, where necessary to join two protein encoding regions, contiguous and in reading frame.

The terms "signal sequence", "signal peptide" and "secretory leader" are used interchangeably and refer to a short (usually about 15-60 amino acids), continuous stretch of amino acids at the amino-terminus of secreted and membrane-bound polypeptides, which directs their delivery to various locations outside the cytosol. Signal sequences usually contain a hydrophobic core of about 4-15 amino acids, which is often immediately preceded by a basic amino acid. At the carboxyl-terminal end of the signal peptide there are a pair of small, uncharged amino acids separated by a single intervening amino acid that defines the signal peptide cleavage site (von Heijne, 1990). Despite their overall structural and functional similarities, native signal peptides do not have a consensus sequence.

The term "induced", "specifically induced" or "specifically expressed" as used herein refers to the induction of activity of a transcription regulatory sequence, specifically of a promoter. In particular, an initiation or increase in mRNA transcript production of a DNA sequence operably linked to the transcription regulatory sequence is referred to. Induction preferably refers to a change from no mRNA transcription to mRNA transcription, preferably to high mRNA transcription. In some instances "induction" may also refer to a change from a low basal transcription level (e.g. low basal expression levels present *ex vivo*) to a higher transcription level (e.g. induction *in vivo*). Induction can be triggered by physiological, developmental or environmental changes. In particular, an increase of at least 5%, 10%, 20%, 30%, 50%, or more, of mRNA transcription after induction is encompassed in this definition.

"Site-specific induction" or "site-specific expression" refers to the induction of transcription in a specific body cavity, such as in the GI-tract of a subject. Site specific induction may also comprise de-repression of the transcription regulatory activity. Preferably transcription remains long enough active after induction for a sufficient amount of a biologically active substance (encoded by a coding sequence operably linked to the transcription regulatory sequence) to be produced. A sufficient amount in this respect is the minimum amount necessary for the substance to exert its biological effect. The time required will vary, depending on the nature of the biologically active substance. Transcription may, therefore, in one embodiment remain active for an extended period of time, e.g. until the host containing the transcription regulatory sequence has passed through the subject, while in another embodiment it may only be active for a short period following induction. In one particular embodiment, the transcription regulatory sequence is only active in the body cavity in which it is induced. The site-specific induction may occur in any cavity of the GI-tract, such as the stomach, the large intestine or a part thereof (e.g. the cecum, colon or rectum) or the small intestine or a part thereof (e.g. the duodenum, jejumun or ileum).

"GI-tract-induced transcription regulatory sequences" (or "GI-tract-inducible transcription regulatory sequences") refers to transcription regulatory sequences, especially promoters, which are induced *in vivo* in the GI-tract of a conventional subject or any part or parts thereof. GI-tract-induced transcription regulatory sequences may, for example, be only transiently active in the GI-tract.

*"In vivo"* refers to the presence or activity within a body cavity of a subject, in particular within the GI-tract of the subject. Vice versa, *"ex vivo"* refers to the presence or activity outside the body of a subject, e.g. in the laboratory.

The term "body cavity of a subject" is understood to mean a cavity in the body of a subject, in particular a vertebrate, that is externally accessible and that is usually lined with a mucosal surface. Examples of such cavities include the mouth, the nasopharynx, the upper and lower GI-tract, the rectum, the vagina and the non-mammalian counterparts of these mammalian cavities.

When referring herein to "conventional subjects", such as for example "conventional mice", subjects whose GI-tract has not been disturbed by the administration of e.g. drugs or other pharmaceutical substances, such as antibiotics. The GI-tract is thus in a natural, undisturbed condition.

A "nucleic acid construct" is herein understood to mean a man-made nucleic acid molecule resulting from the use of recombinant DNA technology. The term "nucleic acid construct" therefore does not include naturally occurring nucleic acid molecules although a nucleic acid construct may comprise (parts of) naturally occurring nucleic acid molecules. A chimeric gene as defined above is an example of a nucleic acid construct, in particular it is an example of an expression construct. The term "vector" as used herein refers to a nucleic acid construct used for introducing exogenous DNA into host cells, such as for example bacterial cells. A plasmid vector thus at least comprises a replicon and a DNA segment that may be used for insertion of the exogenous DNA into the vector by recombinant techniques, preferably without interfering with the plasmids capability to replicate. Vectors usually comprise further genetic elements to facilitate their use in molecular cloning, such as e.g. selectable markers, multiple cloning sites and the like (see below). An "expression vector" as used herein refers to any plasmid-based cloning vector, in which a promoter and other regulatory elements are present to enable transcription of inserted exogenous DNA (e.g. one or more chimeric genes) when the expression vector is present in a suitable environment (e.g. a suitable host cell and/or body cavity). A "shuttle vector" refers to a plasmid vector that is capable of replication and stable maintenance in at least two different host organisms, e.g. two organisms of different species or different genera. For this capability the shuttle vector may rely on a single broad host-range replicon but usually a shuttle vector will comprise different replicons for different host-organisms or different groups of host-organisms.

A "recombinant micro-organism", a "host" or a "recombinant host" are terms referring to a (micro)-organism comprising a (man made) nucleic acid construct within its cell(s), in particular one or more chimeric genes. The recombinant micro-organism preferably contains the nucleic acid construct as an episomally replicating molecule, or alternatively and more preferably, integrated into its genome. The latter has the advantage of greater genetic stability of the introduced DNA. It is immaterial by what method the nucleic acid construct is introduced into the micro-organism. Suitable transformation methods for introducing nucleic acid constructs into cells of micro-organisms, such as e.g. electroporation, are available to a skilled person. It is noted that sometimes the recombinant micro-organism itself is also referred to as "vector" in the art, as it can be used as a vehicle to deliver nucleic acid and protein molecules to desired locations.

"Food-grade" micro-organisms are in particular organisms, which are considered as not harmful, when ingested by a human or animal subject.

A "subject" refers herein to a human or non-human animal, in particular a vertebrate, such as but not limited to domestic animals.

The term "selectable marker" is a term familiar to one of ordinary skill in the art and is used herein to describe any genetic entity which, when expressed, can be used to select for a cell or cells containing the selectable marker. Selectable marker gene products confer for example antibiotic resistance. Genes conferring resistance to antibiotics such as kanamycin, rifampicin, erythromycin, actinomycin, chloramphenicol, tetracyclines, nisin and lactacin F are generally known in the art. The term "reporter" may be used interchangeably with marker, although it is mainly used to refer to visible markers, such as green fluorescent protein (GFP). Selectable markers may be dominant or recessive or bidirectional.

The term "homologous" when used to indicate the relation between a given (recombinant) nucleic acid or polypeptide molecule and a given host organism or host cell, is understood to mean that in nature the nucleic acid or polypeptide molecule is produced by a host cell or organisms of the same species, preferably of the same variety or strain. If homologous to a host cell, a nucleic acid sequence encoding a polypeptide will typically (but not necessarily) be operably linked to another (heterologous) promoter sequence or, if applicable, another (heterologous) secretory signal sequence and/or terminator sequence than in its natural environment. Such regulatory sequences may also be homologous to the host cell. In this context, the use of only "homologous" sequence elements allows the construction of "self-cloned" genetically modified organisms (GMO's) (self-cloning is defined herein as in European Directive 98/81/EC Annex II; see also below herein). When used to indicate the relatedness of two nucleic acid sequences the term "homologous" means that one single-stranded nucleic acid sequence may hybridize to a complementary single-stranded nucleic acid sequence. The degree of hybridization may depend on a number of factors including the amount of identity between the sequences and the hybridization conditions such as temperature and salt concentration as discussed later.

"Stringent hybridization conditions" can also be used to identify nucleotide sequences, which are substantially identical to a given nucleotide sequence. Stringent conditions are sequence dependent and will be different in different circumstances. Generally, stringent conditions are selected to be about 5°C lower than the thermal melting point (Tm) for the specific sequences at a defined ionic strength and pH. The Tm is the temperature (under defined ionic strength and pH) at which 50% of the target sequence hybridizes to a perfectly matched probe. Typically stringent conditions will be chosen in which the salt concentration is about 0.02 molar at pH 7 and the temperature is at least 60°C. Lowering the salt concentration and/or increasing the temperature increases stringency. Stringent conditions for RNA-DNA hybridizations (Northern blots using a probe of e.g. 100nt) are for example those which include at least one wash in 0.2X SSC at 63°C for 20min, or equivalent conditions. Stringent conditions for DNA-DNA hybridization (Southern blots using a probe of e.g. 100nt) are for example those which include at least one wash (usually 2) in 0.2X SSC at a temperature of at least 50°C, usually about 55°C, for 20 min, or equivalent conditions.

The term "substantially identical", "substantial identity" or "essentially similar" or "essential similarity" means that two peptide or two nucleotide sequences, when optimally aligned, such as by the programs GAP or BESTFIT using default parameters, share at least about 75 percent, preferably at least about 80 percent sequence identity, preferably at least about 90 percent sequence identity, more preferably at least 95 percent sequence identity or more (e.g., 99 percent sequence identity). GAP uses the Needleman and Wunsch global alignment algorithm to align two sequences over their entire length, maximizing the number of matches and minimizes the number of gaps. Generally, the GAP default parameters are used, with a gap creation penalty = 50 (nucleotides) / 8 (proteins) and gap extension penalty = 3 (nucleotides) / 2 (proteins).

For nucleotides the default scoring matrix used is nwsgapdna and for proteins the default scoring matrix is Blosum62 (Henikoff & Henikoff, 1992). It is clear than when RNA sequences are said to be essentially similar or have a certain degree of sequence identity with DNA sequences, thymine (T) in the DNA sequence is considered equal to uracil (U) in the RNA sequence.

The term "comprising" is to be interpreted as specifying the presence of the stated parts, steps or components, but does not exclude the presence of one or more additional parts, steps or components. A nucleic acid sequence comprising region X, may thus comprise additional regions, i.e. region X may be embedded in a larger nucleic acid region.

The present invention is based on the finding that Cre recombinase (also referred to as resolvase) functions correctly and efficiently in *L. plantarum* and that the R-IVET method can successfully be used to identify *L. plantarum* transcription regulatory sequences, which are specifically induced *in vivo,* i.e. during the passage of *L*. *plantarum* through the GI-tract of a conventional subject. Abremski et al., 1983 (Cell, 32: 1301-1311) discovered this site-specific recombination system of bacteriophage P1, consisting of a recombination site designated loxP and a recombinase designated Cre. Since then the Cre/loxP recombination system has been used for example in *Saccharomyces cerevisiae* (Sauer, Molecular and Cellular Biology, 7: 2087-2096, 1987), mouse (Sauer and Henderson, Nucl. Acids Res., 17: 147-161, 1989) and plants (US 5,658,772 and US 4,959,317).

### Screening method of the invention

In one embodiment of the invention a method is provided for identifying transcription regulatory sequences of lactic acid bacteria (preferably food-grade), which are specifically induced *in vivo,* in the GI-tract of a subject, while preferably displaying very low or preferably no activity *ex vivo.* "Lactic acid bacteria" are bacteria, which produce lactic acid as an end product of fermentation, such as bacteria of the genus Lactobacillus, Streptococcus and Bifidobacterium. In one embodiment the method of the invention is used for the identification *of L. plantarum* genes and transcription regulatory sequences, which are specifically induced *in vivo* when *L. plantarum* passes through the GI-tract of a conventional subject. The method comprises the insertion of a selectable marker gene flanked by loxP sequences into the genome of a lactic acid bacterium from which *in vivo* induced regulatory sequences are to be isolated, constructing a chromosomal library of the lactic acid bacterium in a vector comprising a promoterless *cre* gene (wherein the chromosomal fragments are inserted upstream of the *cre* gene, introducing the vectors into the loxP-selectable marker-loxP comprising strain, counter-selecting against activity under *ex vivo* conditions by growing the cells for several generations *ex vivo* in the presence of the selectable marker, (orally) administering marker positive colonies to the subject, collecting an plating faecal samples and identifying marker negative strains. The transcription regulatory element trapped in the vector of these cells is then sequenced. The method is further described in the non-limiting Examples.

### Transcription regulatory sequences of the invention

The present invention is further based on the surprising discovery that a large number of transcription regulatory sequence of *L. plantarum* are specifically induced in a body cavity of a subject, more preferably in the GI-tract of a subject and most preferably in the GI-tract of a conventional subject *in vivo.* In one embodiment of the invention transcription regulatory sequences, which are specifically induced in a body cavity or in the GI-tract, or one or more parts of the GI-tract, of subjects are provided (transcription regulatory sequences of SEQ ID NO: 1 to SEQ ID NO: 62). The present invention relates to the practical application of such transcription regulatory sequences, in particular to the use of these regulatory sequences for the *in vivo,* site-specific production of biologically active substances in the GI-tract of a subject. As the whole genome of *L. plantarum* has been sequenced and is available in the art (EMBL database Accession number AL935263 and Kleerebezem *et al*. 2003 supra), SEQ ID NO's 1-62 are available to a skilled person. However, the present invention provides a previously unknown use for these 71 transcription regulatory sequences and variants thereof, such as active fragments thereof, or longer transcription regulatory sequences comprising any of the transcription regulatory sequences of SEQ ID NO: 1-62, or sequences, which are substantially identical to the transcription regulatory sequences of SEQ ID NO's 1-62 and have the capacity to be GI-tract-induced.

SEQ ID NO's 1-62 refers to the GI-tract-induced transcription regulatory sequences normally associated with the following *L. plantarum* genes, as listed in Table 1 below. It is noted that in eight instances one SEQ ID NO comprises more than one transcription regulatory sequence, which is why the 71 GI-tract induced transcription regulatory sequences identified are comprised within only 62 SEQ ID NO's.

| SEQ ID NO: | GI-tract-induced genes (ORFs) and product or function: the translation start codon (bases within the SEQ ID NO) | |
|---|---|---|
| *Functional class of gene: nutrient acquisition and synthesis* | | |
| 1 | Lp_0185 (*pts1BCA*)^{a} (553-555) | sucrose PTS EIIBCA |
| 2 | Lp_1164 (*pts14C*) (431-433) | cellobiose PTS, EIIC |
| 3 | Lp_2647 (*pts19A*) (509-511) | N-acetylglucosamine/galactosamine PTS, EIIA |
| 4 | Lp_3473 (*ram2*) (1010-1012) | alpha-L-rhamnosidase |
| 5 | Lp_3522 (*pts32BC*) (519-521) | sucrose PTS, EIIBC |
| 6 | Lp_3526 (*pbg10*)^{a} (553-555) | 6-phospho-beta-glucosidase |
| 7 | Lp_3618² (*pts37A*) (356-358) | sorbitol PTS EIIA |
| 8 | Lp_3659³ (*rbsD*) (1276-1278) | ribose transport protein |
| 8 | Lp_3660³ (*rbsK3*) (353-355) | ribokinase |
| 9 | Lp_0017 (*proA*) (68-70) | glutamate-5-semialdehyde dehydrogenase |
| 10 | Lp_0228 (*pepD1*) (916-918) | dipeptidase |
| 11 | Lp_0696 (417-419) | cytosine/adenosine deaminase |
| 12 | Lp_0775 (*argG*) (707-709) | argininosuccinate synthase |
| 13 | Lp_0854 (*birA2*)^{a} (643-645) | biotin--[acetyl-CoA-carboxylase] ligase and biotin operon repressor |
| 14 | Lp_1058 (*adk*) (673-675) | adenylate kinase |
| 15 | Lp_1319 (*rsuA*) (391-393) | pseudouridylate synthase |
| 16 | Lp_1602⁷ (116-118) | geranyltranstransferase / farnesyl di P transferase |
| 17 | Lp_2031 (*ribC2*) (128-130) | bifunctional protein: riboflavin kinase and FMN adenylyltransferase |
| *Funtional class of genes: stress* | | |
| 18 | Lp_1019 (*clpC*) (187-189) | ATP-dependent Clp protease |
| 19 | Lp_3055⁴ (*copA*) (983-985) | copper transporting ATPase |
| 20 | Lp_3288 (484-486) | cation efflux protein |
| 21 | Lp_3303⁵ (1022-1024) | putative multidrug transport protein |
| *Functional class of gene: extracellular* | | |
| 22 | Lp_0800 (797-799) | cell surface protein precursor, LPQTNE motif |
| 23 | Lp_2940 (44-46) | cell surface protein precursor, LPQTNE motif |
| 24 | Lip_0141 (441-443) | extracellular protein |
| 25 | Lp_1403^{a} (506-508) | cell surface protein |
| *Functional class of gene: regulators* | | |
| 26 | Lp_3176 (*pkn2*) (770-772) | serine/threonine protein kinase |
| 27 | Lp_3514 (*bglG4*) (538-540) | transcription antitermination |
| 28 | Lp_3646^{a} (740-742) | transcription regulator |
| *Functional class of gene: other* | | |
| 29 | Lp_0237 (575-577) | integral membrane protein |
| 30 | Lp_0299 (604-606) | ABC transporter, ATP-binding protein |
| 31 | Lp_0305 (*gcsH1*) (532-534) | glycine cleavage system, H protein |
| 32 | *Lp_0393* (*thgA1*)(1150-1152) | galactoside O-acetyltransferase |
| 33 | Lp_0419 (*plnl*)^{a} (266-268) | immunity protein |
| 34 | Lp_0489 (*tktl-N*) (443-445) | transketolase |
| 35 | Lp_0698 (*dnaX*)^{a} (115-117) | DNA-directed DNA polymerase III, gamma/tau subunit |
| 36 | Lp_0740⁶ (*prfB-N*) (256-258) | peptide chain release factor 2, N-terminal fragment |
| 36 | Lp_0741⁶ (*prfB-C*) (299-301) | peptide chain release factor 2, C-terminal fragment |
| 16 | Lp_1603⁷ (*ispA*) (1021-1023) | hemolysin homologue |
| 37 | Lp_1847 (*codV*) (1149-1151) | integrase/recombinase |
| 38 | Lp_3051 (*dhaT*) (275-277) | 1,3-propanediol dehydrogenase |
| 39 | Lp_3082 (432-434) | transport protein |
| 40 | Lp_3281 (1107-1109) | transport protein |
| 41 | Lp_3500 (137-139) | short-chain dehydrogenase/oxidoreductase |
| 42 | Lp_3505 (*est2*) (26-28) | acetylesterase |
| 7 | Lp_3617² (*tal3*) (756-758) | transaldolase |
| 43 | Lp_3662 (*adhE*) (218-220) | bifunctional protein: alcohol and acetaldehyde dehydrogenase |
| *hypothetical* | | |
| 44, 45, | Lp_0026 (478-480), Lp_0139 (343-345), | |
| 46, 47, | Lp_0190 (103-105), Lp_0291^{a} (738-740), | |
| 48, 48, | Lp_0476⁸ (1115-1117), Lp_0477⁸ (168-170), | |
| 49, 50, | Lp_0907 (106-108), Lp_1969 (413-415), | |
| 51, 52, | Lp_2337 (957-959), Lp_2507⁹ (615-617), | |
| 52, 53, | Lp_2508⁹ (204-206), Lp_2713 (938-940), | |
| 54, 19, | Lp_2718 (225-227), Lp_3057⁴ (714-716), | |
| 19, 55, | Lp_3058⁴ (324-326), Lp_3312 (1062-1064) | |
| 56, 57, | Lp_0118^{a} (342-344), Lp_0292 (685-687), | |
| 58, 59, | Lp_1788 (34-36), Lp_1872 (885-887), | |
| 60, 61, | Lp_2112 (243-245), Lp_2584 (1208-1210), | |
| 62, 21 | Lp_3246 (1001-1003), Lp_3305⁵ (394-396) | |

| | | |
|---|---|---|
| ^{a} = identified twice during R-IVET screen; ²⁻⁹ genes with the same tag originate from one clone. The gene-annotation corresponds to that of Kleerebezem *et al*. 2003 (supra). | | |

Although SEQ ID NO's: 1-62 provide the complete insert nucleic acid sequence of the clones identified in the R-IVET screen, it is understood that preferably only the transcription regulatory sequence is referred to, when referring to a specific SEQ ID NO. The transcription regulatory sequence is in particular the nucleic acid sequence (or an active part thereof) found upstream (5') of the prokaryotic translation start codon (ATG, GTG or TTG) of the nucleic acid sequence or its complement. One can also refer to the "lp_XXXX transcription regulatory sequence", which is understood to mean the transcription regulatory sequence normally driving the transcription of the nucleic acid comprising the lp_XXXX open reading frame (ORF) in *L. plantarum* (wherein X refers to the numbers as indicated in Table 1). This wording encompasses the whole or any active fragment of the naturally found transcription regulatory sequence, or active variants thereof.

The transcription regulatory sequences present in SEQ ID NO's 1-62 can be operably linked to nucleotide sequences encoding a variety of biologically active proteins or peptides, as described further herein below. Also, active fragments of SEQ ID.NO's 1-62 may be used. Fragments which retain the ability to be specifically induced in the GI-tract of a conventional subject can be easily generated by a skilled person by, for example, deleting one or more nucleotides at either or both ends of the nucleic acid molecule and subsequently testing the activity and specificity of the modified transcription regulatory sequence. Similarly, single nucleotide insertions, replacements or deletions can be carried out and the specific activity of the modified nucleotide sequence can be tested.

As mentioned above, the skilled person will recognise that the sequences in SEQ ID NO's 1-62 at least comprise a functional part of the transcription regulatory sequences that are specifically induced in the GI-tract, but that these nucleic acid sequences may in addition comprise (parts of) coding sequences that are naturally linked to the transcription regulatory sequences as present in the *L. plantarum* genome. Such endogenous coding sequences may be removed from the transcription regulatory sequences for use in the present invention, preferably at or near the translation initiation codon (or 'start codon', as indicated in Tabel 1). Alternatively, a coding sequence, encoding a biologically active protein, may be linked in frame to the present coding sequence (or part thereof), so that a fusion protein is generated upon transcription and translation (as long as the biological activity of the protein or peptide is not reduced significantly by the amino acids fused to it upstream). Similarly, the transcription regulatory sequences present within SEQ ID NO's 1-62 may comprise only a part of the transcription regulatory sequences that are specifically induced in the GI-tract. Although the transcription regulatory fragment is active, it may function suboptimally. In such instances the skilled person will recognise that the transcription regulatory sequences comprised in SEQ ID NO's 1-62 may be extended with upstream sequence (relative to the direction of transcription) to complement the transcription regulatory sequences of the invention, using the genomic sequence information as available for *L*. *plantarum.* A skilled person can easily generate such sequences varying in length and identify whether the whole transcription regulatory sequence (as found endogenously in *L. plantarum*) or shorter fragments confer optimal GI-tract induction.

Transcription regulatory sequences of the invention are thus selected from:
(a) a sequence comprising any of the sequences of SEQ ID NO: 1-62;
(b) a sequence comprising a fragment of any of the sequences of SEQ ID NO: 1-62 whereby the fragment retains the ability to be specifically induced in a body cavity of a subject; or,
(c) a sequence which is substantially identical to any of the sequences of (a) or (b) and
which retains the ability to be specifically induced in a body cavity of a subject. The ability of a fragment or of a modified regulatory sequence to be GI-tract-induced can be tested by, for example, generating an expression vector comprising the (modified) regulatory sequence operably linked to a coding sequence, transforming *L*. *plantarum* with this vector and analysing spatial-temporal induction of the transcript during passage of the recombinant *L. plantarum* through the GI-tract of a subject. The assay used to analyse spatial-temporal expression depends on the nature of the operably linked coding sequence. This sequence may for example encode a light-emitting reporter (such as GFPuv, a Green Fluorescent Protein protein variant optimized for bacterial expression, see Geoffrey et al. 2000, Applied and Environmental Microbiology Vol. 66(1), 383-391) or an enzyme whose activity can be tested. Alternatively, quantitative reverse transcriptase PCR (RT-PCR) can be used to quantify the transcript levels produced. Relative transcript levels are determined by comparing steady state levels of the transcript(s) regulated by the transcription regulatory sequence *in vivo* and *ex vivo* or, if more convenient, indirectly by comparing the steady state levels or enzymatic activities of the protein(s) or enzyme(s) respectively encoded by these transcripts.

Nucleotide sequences which are substantially identical to SEQ ID NO's 1-62, or parts thereof, and in particular to the sequences upstream of the translation start codon(s) comprised in SEQ ID NO's 1-62, and which retain the ability to be GI-tract-induced are also an embodiment of the invention. Such sequences can either be generated by modification of SEQ ID NO's 1-62 (or parts thereof) as described above (nucleotide deletions, insertions, replacements), by *de novo* synthesis of sequences which are substantially similar to SEQ ID NO's 1-62 (or parts thereof) or by isolation of substantially similar sequences from other strains of *L. plantarum* or other organisms, such as but not limited to other food-grade lactic acid bacteria. This can be done using, for example, hybridization methods known in the art, wherein all or part of SEQ ID NO's 1-62 is used as a probe. Preferably stringent hybridization conditions are used to isolate substantially identical sequences.

It is understood that substantially identical GI-tract-induced regulatory sequences can also be obtained by other known methods, such as e.g. those employing (degenerate) primers or probes. For example PCR primers can be synthesized, which correspond to parts of any one of nucleotide sequences SEQ ID NO: 1-62 or to parts of the coding sequence which is in nature associated with the particular regulatory sequence and a PCR reaction can be carried out using genomic DNA of another strain or organism as template DNA. Substantially identical sequences can also be identified *in silico,* by using publicly available algorithms and databases such as BLAST, FASTA and the like, using any one of SEQ ID No's 1-62 (or fragments or variants thereof) as input sequence, or any of the coding sequence listed in Table 1. Sequences identified in this way can then either be cloned using standard molecular biology techniques or synthesised chemically using for example an oligonucleotide synthesizer as supplied e.g. by Applied Biosystems Inc. (Fosters, CA, USA). In all cases, the ability of the resulting nucleotide sequence to be GI-tract-induced and to drive expression in the GI-tract of a subject need to be tested as described above.

Thus, the use of transcription regulatory sequence which have at least about 75%, more preferably at least about 80%, 85%, 90%, 95% or 99% substantial identity to any one of the transcription regulatory sequences of SEQ ID NO's 1-62 (or fragments thereof) and which are GI-tract induced in a conventional subject are one embodiment of the invention. It is understood that identical sequences obtained from other organisms or other bacterial strains and which are GI-tract-induced are also part of the invention. The transcription regulatory sequences according to the invention are preferably specifically induced in at least one part of the GI-tract, although they may also be induced in several GI-tract parts or the whole of the GI-tract. They may be active transiently (e.g. only for a certain time) or only in a certain part(s) of the GI-tract of the subject. For delivery of a biologically active protein or peptide to a specific body cavity of the GI-tract, it is preferred that the transcription regulatory sequences are specifically induced in only this specific cavity and that the transcription regulatory sequences only remain active in this body cavity, so that the protein or peptide encoded by the operably linked nucleotide sequence is exclusively produced in this cavity. Preferably, the transcription regulatory sequences provide a high level of transcription of an operably linked coding sequence after induction, although this depends on the properties of the encoded biologically active substance. It may, for example, be preferable to express a coding sequence encoding a substance which is very active, but which has negative side effects, for only a short period and/or at a low level. A skilled person can analyse the relative transcription strength, the GI-tract part(s) where induction occurs, the time period during which transcription remains active or the induction cues (such as osmolarity, pH, temperature, activators and the like) using known methods. Using this information the transcription regulatory sequence most suitable for the *in vivo* production of a biologically active substance can be selected. For example, it may be desirable to specifically induce protein production in e.g. the duodenum, the stomach or the colon. For specific delivery of a biologically active substance to the duodenum the transcription regulatory sequences SEQ ID NO: 29 or SEQ ID NO: 12 (or active fragments or variants thereof) may be particularly suitable, while for specific delivery to the stomach for example SEQ ID NO: 18 may be suited and for specific delivery to the colon SEQ ID NO: 4 or SEQ ID NO: 9 may be suited.

### Vectors

In another embodiment of the invention vectors comprising one or more of the transcription regulatory sequences of the invention (as described above) are provided. Usually the nucleic acid vectors of the invention will consist of DNA. These nucleic acid vectors may be provided using techniques known per se, for which reference is made to standard handbooks such as e.g. Sambrook *et al*. (1989) and Sambrook and Russell (2001). In particular expression vectors comprising one or more of the GI-tract-induced regulatory sequences of the invention are provided. An expression vector comprises at least one transcription regulatory sequence according to the invention and one or more coding sequence(s), whereby the transcription regulatory sequence is operably linked to the coding sequence. Suitable coding sequences are those encoding biologically active substances as described elsewhere herein.

The 'empty' vector, only comprising one or more of the transcription regulatory sequences of the invention, but lacking a suitable coding sequence is also an embodiment of the invention. Ideally the empty vector comprises one or more restriction sites downstream of the regulatory sequence, which can be used to easily insert a coding sequence downstream of the regulatory sequence, so that the coding sequence is operably linked to the regulatory sequence.

A vector according to the invention may comprise more than one chimeric gene, each of which may comprise a GI-tract-induced regulatory sequence. The GI-tract-induced regulatory sequences may be different or identical. In this way the timing, location and expression levels of the coding sequences *in vivo* can be regulated. For example, nucleotide sequences encoding two biologically active peptides may be expressed concomitantly or consecutively in the GI-tract of a subject. Similarly, one coding sequence may be expressed at a high level, while the other may be expressed at a low level in a particular part of the GI-tract by using two different transcription regulatory sequences of the invention.

Further, a single regulatory sequence may drive the expression of two or more coding sequences. For example, nucleotide sequences encoding different proteins (e.g. proteins having a synergistic effect) may be linked by IRES (internal ribosome entry sites) elements, providing bicistronic or multicistronic transcripts under control of a single regulatory sequence. Suitable IRES elements are described in e.g. Hsieh et al. (1995, Biochemical Biophys. Res. Commun. 214:910-917). The arrangement of several coding sequences in a single transcription unit allows for the co-ordinate expression of several different polypeptides. This may be advantageous for the expression of multisubunit enzymes or in metabolic pathway engineering when several enzymes of a biosynthetic pathway need to be co-ordinately expressed.

The one or more coding sequences operably linked to the regulatory sequence are preferably each provided with the appropriate signals for initiation of translation such as e.g. a Shine-Dalgamo sequence and a translation initiation codon. Optionally a coding sequence in the expression construct comprises a signal sequence encoding a signal peptide causing translocation of the polypeptide encoded by the coding sequence across the cell membrane, and preferably secretion of the polypeptide from the host cell. Signal sequences are well known to the skilled person and a variety of suitable signal sequences is available in the art. Usually the skilled person will select a signal sequence known to effect secretion of polypeptides from a given host cell of choice.

Preferably, the signal sequence to be used in the invention will be homologous to the host organism or will originate from an organism closely related to the host organism. Signal sequences that are preferably used in the context of the present invention include signal sequences from lactic acid bacteria of which preferably *Lactobacillus.* Examples of such signal sequences include e.g. signal sequences from α-amylase, *slpA,* and *prtP*. Further suitable signal sequence s may be identified using dedicated genetic screening methods as described by Poquet et al. (J. Bacteriol. 1998, 180: 1904-1912.) and Hols et al. (Gene, 1992, 118: 21-30). In the expression construct, the transcription unit comprising the one or more coding sequences linked to the transcription regulatory sequence is preferably terminated by a suitable 3'nontranslated region (comprising a transcription terminator), such e.g. *tldH* or *tslP.* Suitable bacterial 3' nontranslated sequences may be those naturally associated with the coding sequence or may be identified as described by Ermolaeva et al. (J. Mol. Biol. 2000, 301: 27-33.).

The nucleic acid constructs of the invention may further comprise additional sequence elements for various functions known in the art *per se.* Such sequence element include e.g.: autonomously replicating sequences or origins of replication for episomal multiplication and maintenance of the vector; sequences for promoting integration of the vector or chimeric gene into the host's genome; sequences for homologous recombination; sequences for site-specific integration and/or recombination; selection marker genes; reporter or indicator genes; sequences for promoting conjugation or other means of transfer of genetic material etc.

### Hosts

In a further aspect of the invention a host organism, preferably a microbial and/or recombinant host organism, comprising one or more transcription regulatory sequences of the invention, and in particular comprising one or more chimeric genes which comprise at least one regulatory sequence of the invention, is provided. The chimeric gene may be integrated into the genome of the host organism or may be present as an episomal entity, for example on an expression vector, as described above. Integration has the advantage of greater genetic stability. The "host" refers to the organism comprising the chimeric gene(s). The host or host cells may be any micro-organism (such as a bacteria, fungi such as yeasts, viruses) or any plant or animal. A host or host cells may also be a mutant, generated by classical (X-ray or chemical) mutagenesis or the host may already have been transformed previously. However, the preferred host is a bacterium, preferably one of the following: A food-grade bacterium, a commensal bacterium or a (attenuated) pathogenic bacterium. In one embodiment the host is a food-grade bacterium, particularly a gram-positive bacterium, and more preferably a lactic acid bacterium. Other preferred hosts are food-grade fungi and yeasts, such as bakers or brewers yeast, such as species of the genus Saccharomyces, Pichia, or Hansenula. The host may also be a probiotic bacterium, which in itself has a beneficial effect when ingested by a subject.

A preferred host is a bacterium that belongs to a genus selected from the group consisting of *Lactobacillus, Lactococcus, Leuconostoc, Carnobacterium, Streptococcus, Bifidobacterium, Bacteroides, Eubacterium, Clostridium, Fusobacterium, Propionibacterium, Enterococcus, Staphylococcus, Peptostreptococcus,* and *Escherichia:* A further preferred host is a bacterium that is a *Lactobacillus* or *Bifidobacterium* species selected from the group consisting of *L*. *reuteri, L. fermentum, L. acidophilus, L. crispatus, L. gasseri, L. johnsonii, L. plantarum, L. paracasei, L. murinus, L. jensenii, L. salivarius, L. minutis, L. brevis, L. gallinarum, L*. *amylovorus, B. bifidum, B. longum, B. infantis, B. breve, B. adolescent, B. animalis, B. gallinarum, B. magnum,* and *B. thermophilum.*

Preferred hosts or host cells are (1) capable of initiating transcription, and preferably also regulating transcription, from the transcription regulatory sequence of the invention; (2) capable of surviving passage through a subject's GI-tract and preferably capable of colonizing a mucosal surface lining a subjects GI-tract; and (3) preferably, not pathogenic to the subject in which the host or host cells are to be employed. With respect to the third aspect it is to be understood that the invention comprises the use of hosts that may normally be pathogenic to the subject (e.g. *Listeria* spp., *Salmonella* spp. or *Campylobacter* spp.) but that have been modified such that they are no longer pathogenic or virulent. Thus, a preferred microbial host will usually be a non-pathogenic bacterium capable of colonizing mucosal surfaces of vertebrates, which bacterium is transformed with a nucleic acid construct or vector described herein above. In a further embodiment of the invention, the host or host cell may be modified as to its capability to adhere to mucosal surfaces. A range of bacterial proteins are known to be involved in adherence to mucosal surfaces. The genes encoding these may be modified. A number of *L*. *plantarum* genes also display significant sequence homology to such genes identified in other bacterial species and may thus be modified with a reasonable expectation of successfully modifying the capability *of L. plantarum* to adhere to mucosal surfaces. These include genes involved in mucus-binding, fibronectin binding, aggregation promotion, intercellular adhesion, cell clumping, etc., such as for example: lp_0304: aggregation promoting protein [*Lactobacillus gasseri*], lp_0520: autoaggregation-mediating protein [*Lactobacillus reuteri*], lp_1229: mucus binding protein precursor; Mub [*Lactobacillus reuteri*], lp_1643: mucus binding protein precursor; Mub [*Lactobacillus reuteri*], lp_1793: fibronectin-binding protein-like protein A [*Streptococcus gordonii*], lp_1873: aggregation-relevant adhesin [*Streptococcus gordonii*], lp_2278: autoaggregation-mediating protein [*Lactobacillus reuteri*], lp_2845: aggregation promoting protein [*Lactobacillus gasseri*], lp_2847: aggregation promoting protein [*Lactobacillus gasseri*], lp_3050: aggregation promoting protein [*Lactobacillus gasseri*], lp_3059: mucus binding protein precursor; Mub [*Lactobacillus reuteri*], lp_3114: mucus binding protein precursor; Mub [*Lactobacillus reuteri*], lp-3209: mucus adhesion promoting protein [*Lactobacillus reuteri*], lp-3214: collagen binding protein [*Lactobacillus reuteri*] / mucus adhesion promoting protein [*Lactobacillus reuteri*]. Similarly, the expressing of mucosal adhesion proteins MapA may be altered in the host. Such alterations may include changing the expression of endogenous mucosal adhesion proteins, adding the capability to express an exogenous, i.e. heterologous mucosal adhesion proteins, or changing the mucus binding properties of a mucosal adhesion protein. The alterations may be effected in various ways: e.g. endogenous genes encoding mucosal adhesion proteins may be added or deleted, exogenous of genes encoding mucosal adhesion protein may be introduced, mucus binding properties of mucosal adhesion proteins may be modified, the regulation of expression of the mucosal adhesion proteins may be altered, e.g. by linking a non-native or modified transcription regulatory region to a sequence coding for a mucosal adhesion protein. Some non-limiting specific example include e.g. the inactivation of the endogenous *mapA* gene of *L. reuteri,* or the expression of the *L. reuteri mapA* gene from a promoter other than its native promoter, such as a constitutive or differently regulated promoter.

Methods for transforming bacterial cells are known in the art and are for example described in "Genetics and Biotechnology of Lactic Acid Bacteria", Gasson and de Vos, eds., Chapman and Hall, 1994. Similarly, methods for introducing recombinant DNA into fungi, plants and animals are well known, such as particle gun transformation, protoplast transformation, electroporation, *Agrobacterium* mediated transfonnation and the like.

In case a host of the invention is constructed through genetic engineering such that the resulting recombinant host comprises only sequences derived from the same species as the host is, albeit in recombined form, the host is said to be obtained through "self-cloning". Hosts obtained through self-cloning have the advantage that there application in food (or pharmaceuticals) is more readily accepted by the public and regulatory authorities as compared hosts comprising foreign (i.e. heterologous) nucleic acid sequences. The present invention thus allows the construction of self-cloned *L*. *plantarum* and other lactobacillus hosts for food, pharmaceutical or nutraceutical applications (see also de Vos, 1999, Int. Dairy J. 9: 3-10) and such self-cloned hosts are one further aspect of the invention.

### Method for in vivo site-specific delivery to the GI-tract

Another aspect of the invention relates to a method for producing a polypeptide or a biologically active substance in a body cavity or in several body cavities of a subject, wherein the method comprises the step of administering to the subject a recombinant host or recombinant host cells (preferably recombinant bacterial cells) capable of producing the biologically active substance. In one preferred embodiment the body cavity is the GI-tract, or one or more parts thereof. For this purpose the host comprises at least one chimeric gene, which comprises a transcription regulatory sequence of the invention, so that transcription of the coding sequence is specifically induced when the host is present *in vivo* in the body cavity (e.g. in the GI-tract, or a part thereof) of a conventional subject. The coding sequence that is operably linked to the transcription regulatory sequence preferably encodes a polypeptide that is (1) the biologically active substance, (2) a precursor thereto, or (3) a polypeptide involved in the biosynthesis of the biologically active substance.

A "biologically active substance" as used herein refers to a substance, which has biological activity when present in at least part of the GI-tract of a subject. Suitably nucleotide sequences encoding biologically active proteins or polypeptides are available to a skilled person. The type of coding sequence that is linked to the transcription regulatory sequence depends on the biologically active substance to be produced and delivered site-specifically by the recombinant host to the GI-tract (or parts thereof) of the subject. The coding sequence is not natively associated with the transcription regulatory sequence. The coding sequence may be homologous but preferably is heterologous to *Lactobacillus.* The coding sequence may encodes a polypeptide selected from the group consisting of an antigen from a human or animal pathogen, such as e.g. tetanus toxin fragment C (TTFC) or a protective antigen of *Bacillus anthracis;* an enzyme, such e.g. a β-galactosidase, an amylase, a protease, a pancreatic (phospho-)lipase or a superoxidedismutase; a biologically active peptide, such as e.g. oxyntomodulin, oryzatensin, prosomatostatin-derived peptides, exorphin A5, bioactive antinutritional peptides derived from cereal prolamins and others as reviewed by Dziuba et al. (Nahrung, 1999, 43: 190-195), gastrointestinal (peptide) hormones, such as somatostatin and others as reviewed by Rehfeld (Physiol. Rev., 1998, 78: 1087-1108), therapeutic proteins such as e.g. interleukin-10, TGF-β, lactoferrin. The coding sequence may also encode an antibody (raised against any desired antigen), a hybrid antibody, a altered antibody or an Fab fragment or any part which exhibits the immunological binding properties of the parent antibody molecule. In one embodiment of the invention the coding sequence, which is operably linked to the regulatory sequence(s) according to the invention, encodes a peptide or protein which is able to cause lysis of the bacterial host cell. Such a host is herein referred to as a "GI-tract lytic strain", as the cell auto-lyses *in vivo* upon induction of the transcription regulatory sequence. The coding sequence may for example comprise a bacteriophage-derived lytic coding sequence or cassette, an autolysin encoding sequence (e.g. *acmA*), a sequence encoding any other bacteriolytic enzyme or toxin, or a sequence encoding an activator of cell autolysis. The GI-tract lytic strain is preferably "pre-loaded" with a biologically active compound, which is released into the GI-tract lumen upon cell lysis. Pre-loading can for example be achieved by recombinant expression of a biologically active substance under laboratory conditions (e.g. using a constitutive promoter), or at least prior to arrival of the GI-tract lytic strain in the cavity in which lysis is induced. The biologically active substance is preferably present in significant amounts in the cytoplasm of the lytic strain prior to cell lysis. Controlled expression of lytic coding sequences has been described by de Ruyter et al. 1997(Nat. Biotechnol. 15:976-9) and Buist G et al. 1997(Appl Environ Microbiol. 63(7):2722-8),

Preferably the bioactive polypeptides originate from the subject species in who's GI-tract the polypeptides are to be produced, e.g. human sequences coding for interleukin-10, TGF-β, or lactoferrin are preferably used for production in man. Alternatively, the coding sequence(s) may encode one or more enzyme(s) involved in the biosynthesis, transport or release of a biologically active substance. Examples of such biologically active substances are vitamins like folic acid and other B-vitamins; organic acids or alcohols such as lactate, acetate, ethanol or butanediol; antioxidants like thiols; amino acids; non-digestible oligo- and polysaccharides such as those synthesized by Gram-positive bacteria; or antimicrobial agents such as e.g. bacteriocins or lantibiotics. Generally secretion of the biologically active substance into the GI-tract is desired and can be achieved by operably linking a nucleic acid sequence encoding a suitable signal peptide to the nucleic acid sequence encoding the biologically active substance (as described above). Likewise, it may be sufficient that only part of the biologically active protein or peptide is presented externally on the host's cell(s).

A further aspect of the invention relates to compositions comprising host cells as described herein above, as well as to methods for the production of such compositions. Host cells of the invention, preferably bacteria, are cultured under appropriate conditions, optionally recovered from the culture medium and optionally formulated into a composition suitable for the intended use. Methods for the preparation of such compositions are known *per se.* Compositions for enteral or oral administration may be either food compositions or pharmaceutical compositions whereas compositions for nasal, vaginal or rectal administration will usually be pharmaceutical compositions.

Pharmaceutical compositions will usually comprise a pharmaceutical carrier in addition to the host cells of the invention. The preferred form depends on the intended mode of administration and (therapeutic) application. The pharmaceutical carrier can be any compatible, nontoxic substance suitable to deliver the host cells to the body cavity, e.g. the GI-tract of a subject. E.g. sterile water, or inert solids may be used as the carrier usually complemented with pharmaceutically acceptable adjuvants, buffering agents, dispersing agents, and the like. Compositions will either be in liquid, e.g. a stabilized suspension of the host cells, or in solid forms, e.g. a powder of lyophilized host cells. E.g. for oral administration, the host cells can be administered in solid dosage forms, such as capsules, tablets, and powders, or in liquid dosage forms, such as elixirs, syrups, and suspensions. The host cells can be encapsulated in gelatin capsules together with inactive ingredients and powdered carriers, such as e.g. glucose, lactose, sucrose, mannitol, starch, cellulose or cellulose derivatives, magnesium stearate, stearic acid, sodium saccharin, talcum, magnesium carbonate and the like.

A preferred composition according to the invention is suitable for consumption by a subject, preferably a human or an animal. Such compositions may be in the form of a food supplement or a food or food composition, which besides the host cells of the invention also contains a suitable food base. A food or food composition is herein understood to include liquids for human or animal consumption, i.e. a drink or beverage. The food or food composition may be a solid, semi-solid and/or liquid food or food composition, and in particular may be a dairy product, such as a fermented dairy product, including but not limited to a yoghurt, a yoghurt-based drink or buttermilk. Such foods or food compositions may be prepared in a manner known per se, e.g. by adding the recombinant host cells of the invention to a suitable food or food base, in a suitable amount. In a further preferred embodiment, the host cells are micro-organisms that are used in or for the preparation of a food or food composition, e.g. by fermentation. Examples of such micro-organisms include baker's or brewer's yeast and lactic acid bacteria, such as probiotic lactic acid strains. In doing so, the host cells of the invention may be used in a manner known per se for the preparation of such fermented foods or food compositions, e.g. in a manner known per se for the preparation of fermented dairy products using lactic acid bacteria. In such methods, the host cells of the invention may be used in addition to the micro-organism usually used, and/or may replace one or more or part of the micro-organism usually used. For example, in the preparation of fermented dairy products such as yoghurt or yoghurt-based drinks, a food grade lactic acid bacterium of the invention may be added to or used as part of a starter culture or may be suitably added during such a fermentation. Preferably, the above compositions will contain the host cells in amounts that allow for convenient (oral) administration of the host cells, e.g. as or in one or more doses per day or per week. In particular, the preparations may contain a unit dose of the host cells.

In another aspect the invention relates to a method for (site-specific) production of a polypeptide or a biologically active substance at a mucosal surface of a subject. The method comprises the step of administering to the subject a composition comprising host cells, preferably bacteria as described above. In a preferred embodiment, the polypeptide is an antigen from a human or animal pathogen. The *in situ* production of the pathogen's antigen(s) allows for the vaccination of the human or animal to which the composition is administered. In another preferred embodiment the polypeptide or the biologically active substance is a health-promoting factor.

Another aspect of the invention relates to the use of a host cell according to the invention in the preparation of a medicament for preventing or treating an infection by pathogenic micro-organisms of a mucosal surface in a subject.

The following non-limiting Examples describe the use of R-IVET in *L. plantarum* and the identification of GI-tract-inducible transcription regulatory sequences. Unless stated otherwise, the practice of the invention will employ standard conventional methods of molecular biology, virology, microbiology or biochemistry. Such techniques are described in Sambrook et al. (1959) Molecular Cloning, A Laboratory Manual (2nd edition), Cold Spring Harbor Laboratory, Cold Spring Harbor Laboratory Press; in Sambrook and Russell (2001) Molecular Cloning: A Laboratory Manual, Third Edition, Cold Spring Harbor Laboratory Press, NY; in Volumes 1 and 2 of Ausubel et al. (1994) Current Protocols in Molecular Biology, Current Protocols, USA; and in Volumes I and II of Brown (1998) Molecular Biology LabFax, Second Edition, Academic Press (UK); Oligonucleotide Synthesis (N. Gait editor); Nucleic Acid Hybridization (Hames and Higgins, eds.).

### Description of the figures

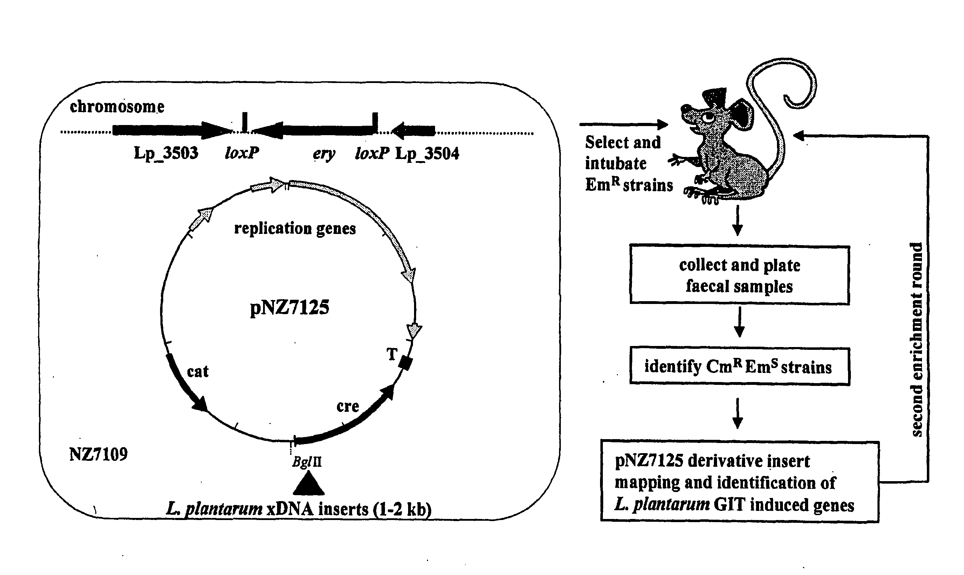

Figure 1: Basic principles of R-IVET. *L. plantarum* strain NZ7109 harbors a chromosomally located *loxP-ery-loxP* cassette and pNZ7125. Chromosomal loci of the *L. plantarum* genome (black triangle) are cloned upstream of *cre* in pNZ7125 and promoter activities in the resulting library can be trapped by monitoring the erythromycin phenotype, since *cre* expression will lead to excision of the erythromycin marker from the chromosome by a homologous recombination event between the two *loxP* sites. The colonies in the library appearing as erythromycin resistant in the laboratory (no active promoters under laboratory conditions) are intragastrically incubated in mice and changes in the erythromycin phenotype (promoter activations) can be monitored in faecal samples.

### Examples

### Example 1 - Materials and methods

### 1.1 Bacterial strains and plasmids

*Escherichia coli* strains MC1061 (Casadaban and Cohen 1980, J. Mol. Biol. 138, 179-207) was used as cloning host during construction of pNZ7109 (see below) and was grown aerobically in TY medium (Sambrook *et al.* 1989, supra). *Lactococcus lactis* MG1363 (Gasson, 1983, J. Bacteriol. 154, 1-9) was used as a cloning host during construction of pNZ7125, pNZ7126 and the *Lactobacillus plantarum* promoter library (see below). *Lactococcus lactis* was grown without aeration at 30 °C in M17 medium (Merck, Darmstadt, Germany), supplemented with 0.5% (wt/vol) glucose (GM17). *L*. *plantarum* WCFS 1 (Kleerebezem *et al.* 2003, supra) and its *loxP-ery-loxP* derivative NZ7109 (see below) were grown at 37 °C in MRS medium (Difco, Surrey, U.K.) without aeration. When appropriate, antibiotics were added to the media; for E. *coli* ampicillin (50 µg/ml); for *Lactococcus lactis* chloramphenicol (5 µg/ml); for *L*. *plantarum* chloramphenicol (5 µg/ml), erythromycin (5 or 30 µg/ml, for selection after transformation or replica-plating, respectively), lincomycin (10 µg/ml) and rifampicin (50 µg/ml).

### 1.2 DNA techniques and sequence analysis

Plasmid DNA was isolated from *E. coli* on a small scale using the alkaline lysis method (Sambrook et al. 1989 and Birnboim and Doly 1979, Nucleic Acid Res. 7, 1513-1523). Large scale plasmid DNA isolations were performed using Jetstar columns according to the manufacturer's instructions (Genomed GmbH, Bad Oberhausen, Germany). DNA isolation and transformation in *Lactococcus lactis* and *L. plantarum* were performed as described previously (de Vos et al. 1989, Gene 85, 169-176; Ferain et al. 1994, J. Bacteriol. 176, 596-601; Josson et al. 1989, Plasmid 21, 9-20). Standard procedure were applied for DNA manipulations in *E. coli* (Sambrook *et al.* 1989). Restriction endonucleases, *Taq* and *Pwo* polymerase, T4 DNA ligase and calf intestine alakaline phosphatase (CIAP) were used following the recommendations of the manufacturer (Promega, Leiden, the Netherlands and Boehringer, Mannheim, Germany). Primers were purchased from Pharmacia Biotech (Roosendaal, the Netherlands). The sequences of the inserts present in the pNZ7125 derivatives (see below), were amplified by PCR using the vector-based primers cre-R2 (5'-GTCCATCAGGTTCTTGCG-3') and BglII-*cre* (5'-ATAGTTTACCCCGTCAGC-3'), followed by amplicon purification using Sephadex-G50 and multiscreen HV 96-well plates (Millipore, Amsterdam, The Netherlands). Partial insert sequences were determined using either primer *cre*-R2 or BgIII-cre, approximately 100 ng of the purified amplicons and the ABI Prism^{®} BigDye (tm) Terminator Cycle Sequencing Ready Reaction Kit protocol provided by the manufacturer (Applied Biosystems, Nieuwekerk a/d IJsel, The Netherlands). Sequence reaction products were analyzed using an ABI-prism 3700 DNA analyzer. The determined insert sequences were assigned to *L. plantarum* WCFS 1 chromosomal loci using Blast-N (Kleerebeezem *et al.* 2003).

### 1.3 Construction of L. plantarum NZ7109

The intergenic locus flanked by the convergent genes Lp_3503 and Lp_3504 (Fig. 1) was chosen for the integration of a *loxP-ery-loxP* cassette (the *ery* gene confers erythromycin resistance) into the chromosome of *L*. *plantarum* WCFS 1 using a two step double cross-over strategy (Ferain *et al*. 1994, supra). For this purpose pNZ7109, a pUC18 (Yanisch-Perron et al. 1985, Gene 33, 103-119) derivative which harbours a *loxP-ery-loxP* cassette flanked by PCR amplified 5'- truncated fragments of Lp_3503 (using primers 5'-TGCTTTCCAAGAGCAAGCTG-3' and 5'-ACGTCTGCAGTCAGGTGTGAAGTTGGCACT-3') and Lp_3504 (using primers 5'-ACGTCTGCAGCCACTCACCGACTAACAGTC-3'and 5'-CGTAAGCTTGACCCACGAGTTACCAACACG-5') (Kleerebezem *et al.* 2003) was constructed and integrated into the chromosome of *L*. *plantarum WCFS1* (Fig 1). One integrant possessing the anticipated genotype, as analyzed by PCR and Southern blotting, was cultured with increasing concentrations of rifampicin (maximum concentration 50 µg/ml) and the resulting rifampicin resistant strain was designated NZ7109. The rifampicin and the plasmid-derived chloramphenicol resistant phenotype, were used for selective recovery of NZ7109 from faecal samples (see below).

### 1.4 pNZ7125, pNZ7126 and R-IVET library construction

To implement R-IVET in *L. plantarum* the low-copy vector pNZ7125, a pJIM2246 (Renault et al. 1996, Gene 183, 175-182) derivative, was constructed. This vector harbors the *prtP* ribosome binding site originating from pUC-*Nco*I (Fernandez et al. 2002, J. Bacteriolo. 184, 82-90) and a promoterless copy of the *cre* gene followed by the *pepN* terminator, both originating from pNZ8048-Cre (Campo et al. 2002, Appl. Env. Microbiol. 68, 2359-67). To confirm correct chromosomal *l*o*xP-ery-*/*oxP* resolution (Ambreski et al. 1983, Cell 32, 1301-11) in strain NZ7109 upon *cre* expression, a pNZ7125 derivative containing the *L. plantarum ldhL1* promoter (Ferain *et al*. 1994, supra) upstream of the *cre* gene was constructed (pNZ7126). Moreover, a *L. plantarum* WCFS 1 chromosomal R-IVET library was constructed in pNZ7125. Chromosomal DNA was partially digested with Sau3AI and size-fractionated on 1% agarose gels. Fragments ranging from 1-2 kb were purified using Sephaglas™ Bandprep (Pharmacia Biotech, Roosendaal, The Netherlands). These purified fragments were cloned into *Bgl*II digested and CLAP dephosphorylated pNZ7125 (Fig 1). Ligation mixtures were transformed to *Lactococcus lactis* MG1363 (Gasson *et al*. 1983, supra) and approximately 50,000 of the obtained colonies were collectively resuspended in GM17. Plasmid DNA was isolated from these pooled cells and was introduced into NZ7109 cells. Approximately 37,000 colonies were obtained on MRS plates containing 5 µg/ml chloramphenicol, collectively resuspended in MRS containing 15% glycerol, and stored in aliquots at -80 °C.

### 1.5 R-IVET animal experiments

To counter select against clones in the R-IVET library that harbour pNZ7125 derivatives containing a promoter element that is active under the laboratory conditions applied, the collective library was grown for approximately 20 generations in MRS containing 5 µg/ml chloramphenicol, 30 µg/ml erythromycin and 50 µg/ml rifampicin. Subsequent animal experiments were performed in an accredited establishment (N° A59107) according to guidelines N°86/609/CEE of the French government. Seven weeks-old female Balb/c mice were purchased from Iffa Credo (St Germain sur l'Arbresle, France) and had free access to tap water and standard mice chow during the experiments. Four mice received an oral dose of freshly prepared bacterial suspensions (1 x 10⁹ CFU in 100 µl) by intragastric administration at 2 consecutive days, and 24h after the last administration faecal samples were collected and resuspended in MRS medium at a final concentration of 100 mg/ml (wet weight). After extensive homogenization, the complete R-IVET library was recovered from the faecal samples by plating appropriate dilutions of the suspensions on MRS plates containing 5 µg/ml chloramphenicol and 50 µg/ml rifampicin. After 72h full grown colonies were replica-plated to plates containing 5 µg/ml chloramphenicol and 50 µg/ml rifampicin, with or without 30 µg/ml erythromycin. Another 24h later the plates were compared, leading to the identification of cells displaying an erythromycin sensitive phenotype. The inserts present in the corresponding pNZ7125 derivatives harboured by these resolved clones were amplified by PCR and from the resulting amplicons the DNA sequences were analyzed.

To verify the primary R-IVET results 132 of the selected clones were divided in four groups of 33 clones which were used for collective plasmid DNA isolation. These mixtures of pNZ7125 derivatives were reintroduced into *L. plantarum* NZ7109 and from all four groups several hundreds (minimum of 200) colonies were collectively stored at -80 °C. These four groups were separately subjected to a second round passage using 2 mice per group, following the same procedure as applied in the first round passage, including recovery from faecal samples and analysis of the erythromycin phenotype of the clones.

### Example 2 - Results

### 2.1 Implementation and functionality of R-IVET in L. plantarum

Previously R-IVET has been used exclusively for the identification of genes in pathogens which are induced during infection of host tissues (for reviews see Angelichio and Camilli 2002; Mahan *et al*. 2000, supra). To evaluate the functionality of the *cre-loxP* system in *L. plantarum,* a *L. plantarum* WCFS 1 derivative strain that harbors a chromosomally located *loxP-ery-loxP* cassette, was constructed and designated NZ7109 (Fig. 1). The growth rate of NZ7109 did not differ from that observed for wild-type *L. plantarum* WCFS1, and after 50 generations of growth without antibiotic selection pressure, replica-plating revealed that all NZ7109 cells were erythromycin resistant. Moreover, PCR analysis revealed that the chromosomal *loxP-ery-loxP* insertion could be amplified from all colonies, indicating that the chromosomal insertion is stable. Both pNZ7125, a low-copy vector encoding a promoterless copy of *cre,* and a derivative containing the *cre* gene under control of the *L. plantarum* WCFS1 *ldhL1* promoter (pNZ7126) were introduced in NZ7109 cells and plated on MRS medium with chloramphenicol. Replica-plating of the transformants revealed that all NZ7109 colonies harbouring pNZ7125 were erythromycin resistant, while all NZ7109 colonies harbouring pNZ7126 were erythromycin sensitive. In addition, a PCR analysis confirmed that the *loxP-ery-loxP* cassette could only be amplified from erythromycin resistant colonies. These results establish the functional implementation of the *cre-loxP* resolution system *in L. plantarum* WCFS 1 and confirm the suitability of pNZ7125 as an R-IVET vector.

### 2.2 Construction of a L. plantarum R-IVET library in pNZ7125

A genomic library of *L. plantarum* WCFS 1 was constructed in pNZ7125, using *Lactococcus lactis* MG1363 as an intermediate cloning host. Approximately 37,000 colonies were obtained in *L. plantarum* NZ7109 and the quality of the library was assessed in several ways. First, 100 colonies were randomly picked from MRS plates and their plasmids were used as template for insert amplification by PCR, demonstrating that over 95% of the investigated clones contains an insert, of which the average size was estimated to be 1.3 kb. To assess insert redundancy, all amplicons were digested with *Sau*3AI and the resulting fragments were separated by 2% agarose gel electrophoresis. No common restriction profiles were detected among these 100 amplicons, indicating that redundancy in the R-IVET library is low. Moreover, 28 of these amplicons were used for partial sequence analysis, which revealed no apparent over- or underrepresentation of a specific region of the *L. plantarum* genome. These results support the randomness of the library and genome coverage was calculated to be approximately 98%. The R-IVET library was replica-plated to plates with and without erythromycin which indicated that 10% of the R-IVET clones contain a pNZ7125 derivative harbouring a properly oriented promoter element that is active under the laboratory conditions applied during the experiments. Moreover, the resulting *cre* expression level in these clones is sufficient to result in excision of the *loxP-ery-loxP* cassette from the chromosome of NZ7109.

### 2.3 R-IVET screen in mice

To counter select against clones displaying *cre* expression under laboratory conditions, the R-IVET library was cultured for approximately 20 generations in the presence of erythromycin. Subsequently, full-grown cultures were used for gastric administration to Balb/c mice. After recovery from faecal samples 6,000 of the R-IVET clones were analyzed for their erythromycin resistance or sensitivity phenotype by replica-plating, revealing 198 (3.3%) clones that displayed an erythromycin sensitive phenotype. The partial sequence of 132 of the chromosomal inserts present in the pNZ7125 derivatives originating from the selected clones was determined and corresponded to 119 unique loci of the *L. plantarum* genome, since one locus was found three times, while 11 loci were found twice. According to the current genome annotation database of *L*. *plantarum* WCFS 1 (Kleerebezem *et al.* 2003) these loci harbour 71 unique genes and their upstream transcription regulatory sequences (SEQ ID NO: 1-62 or fragments or variants thereof) in the proper orientation to explain the observed induction of *cre* expression (Table 1, supra). As noted above, 8 loci contained more than one putative 5'-end of an annotated ORF and their regulatory sequence. Remarkably, two independent *Sau*3AI clones corresponding to Lp_0291 and its upstream sequence (SEQ ID NO: 47) were found that differ in size (about 1.0 and 1.5 kb). One clone contained the whole of SEQ ID 47 as insert (1551 bp, i.e. about 1.5 kb), while the other contained a smaller fragment of about 1 kb (965 bp, starting at nucleotide 148 of SEQ ID NO: 47). Hence, the *in vivo* induction of this gene was independently confirmed twice during the R-IVET procedure. The identified GI-tract-induced genes appeared to be randomly located within the *L. plantarum* genome. Moreover, the genes appeared to be randomly distributed among the functional classes of the *L. plantarum* genome. The genes identified in this R-IVET screen were functionally categorized in groups involved in nutrient acquisition and synthesis (18 genes), stress response and adaptation (4 genes), extracellular proteins (4 genes), regulation (3 genes) and others (18 genes). The remaining 24 genes encoded (conserved) hypothetical proteins of unknown function (Table 1, supra).

To verify the primary results of the R-IVET screen, the 132 sequenced pNZ7125 derivatives originating from the resolved clones after the first animal experiment were divided in four groups of 33 pNZ7125 derivatives which were collectively reintroduced into NZ7109. Replica-plating of the transformants revealed that all tested colonies displayed an erythromycin resistant phenotype, confirming the absence of *cre* expression in these clones under laboratory conditions. A second round passage in mice revealed a dramatic increase in the percentage of erythromycin sensitive colonies that was recovered from the faecal samples of all individual mice used during this animal experiment as compared to the experimentally identical first round passage (Table 2). The fact that the second round passage resulted in an average of 38.1% erythromycin sensitive clones in the 8 mice as compared to 3.3% in the first round passage confirmed the R-IVET based enrichment of *L. plantarum* chromosomal fragments harbouring *in vivo* induced transcription regulatory sequences.

Table 1: see above

**Table 2: A second round passage of R-IVET positives through mice results in an increased percentage of erythromycin sensitive (ery^{s}) NZ7109 colonies as compared to the first round passage (3.3%).**

| Mouse: | Promoter groups | ery^{S} (%): |
|---|---|---|
| 1 | 1 | 58.0 |
| 2 | 1 | 71.7 |
| 3 | 2 | 52.9 |
| 4 | 2 | 28.8 |
| 5 | 3 | 34.2 |
| 6 | 3 | 33.3 |
| 7 | 4 | 31.5 |
| 8 | 4 | 37.5 |
| 1-8 | 1-4 | 38.1 |

### 2.4 Analysis of in vivo GI-tract-induced genes

This is the first R-IVET approach in a food-grade bacterium, which resulted in the identification of 71 transcription regulatory sequences (SEQ ID NO: 1-62) that are induced *in situ* in *L. plantarum* WCFS 1 during passage of the mouse GI-tract. The identified genes appeared to be randomly distributed over the chromosome. Moreover, genes from many functional classes were identified and grouped into 6 functional classes (Table 1, supra).

Nine of the genes identified using R-IVET are involved in sugar transport and utilization (Table 1; transcription regulatory sequences SEQ ID NO: 1-8), including 5 (components of) PTS (phosphotransferase) systems, specific for N-acetylglucosamine, sorbitol, sucrose (2x) and cellobiose, a ribose permease and a ribose kinase, and two di-and polysaccharide hydrolyzing enzymes. A diverse carbohydrate potential has been associated with several gram-positive microbes inhabiting the GI-tract, including *L. plantarum* (Kleerebezem *et al.* 2003), *Listeria inocua* and *Listeria monocytogenes* (Glaser et al. 2001, Science 294, 849-52) and *Bifidobacterium longum* (Schell et al., 2002, PNAS 99, 14422-7). The finding that several of these functions are induced *in situ* in the GI-tract, confirms their importance for survival and persistence under GI-tract conditions. Moreover, genes involved in the metabolism of the same sugars have been reported to be induced *in vivo* in a host system in several pathogens, including *Klebsiella pneumoniae* (ribose, Lai et al. 2001, Infect. Immun. 69, 7140-5), *Salmonella typhi* (ribose, Daigle et al. 2001, Mol. Microbiol. 41, 1211-22, *Pasteurella multocida* (sorbitol, Hunt et al. 2001, Infect. Immun. 69, 3004-12), *Listeria monocytogenes* (cellobiose, Gahan and Hill 2000, Mol. Microbiol. 36, 498-507) and *S. gordonii* (sucrose and cellobiose, Kilic et al. 1999, Plasmid 42, 67-72). Remarkably, the IIC transport component of the cellobiose PTS system found (Lp_1164; transcription regulatory sequence SEQ ID NO: 2) is not located in an operon-like structure. In *Listeria monocytogenes* similar "orphan" cellobiose-PTS-IIC components have been shown to play a role in host-specific signalling, leading to modulation of virulence gene expression (Kreft and Vazquez-Boland, 2001, Int. J. Med. Microbiol. 291, 145-57), suggesting host-factor mediated gene regulation in bacteria, possibly including *L. plantarum.*

Nine genes were identified that are involved in the acquisition of non-sugar compounds, including factors involved in amino acid, nucleotide and cofactor metabolism (Table 1; transcription regulatory sequences SEQ ID NO: 9-17). These results suggest that limiting amounts of these compounds are available under the starvation conditions in the GI-tract, leading to activation or derepression of these *L. plantarum* genes. Accordingly, *in vitro* studies in different bacteria confirm the induction of several of these metabolic pathways under end-product limiting conditions (Muse et al. 2003, J. Bacteriol. 185, 2920-6; Khan & Isaacson 2002, Infect. Immun. 70, 3404-12; Cronan 1988, J. Biol. Chem. 263, 10332-6; Mack et al. 1998, J. Bacteriol. 180, 950-5). Moreover, *in vivo* approaches have demonstrated that genes in arginine and proline metabolism in *V. cholerae* (Camilli & Mekalanos 1995, Mol. Microbiol. 18, 671-83) and *Helicobacter pylori* (Kavermann et al. 2003, J. Exp. Med. 197, 813-22), respectively, are induced during mouse infection. In one of the closest relatives of *L. plantarum* (*Listeria monocytogenes*) proline metabolism is induced under high osmolarity conditions (Sleator et al. 2001, Appl. Environm. Microbiol. 67, 2571-7). Such conditions could potentially be found in the colon and might suggest differential colonic expression of Lp_0017 (transcription regulatory sequence SEQ ID NO: 9). Similarly, other experiments in the inventors lab have identified Lp_3473 (transcription regulatory sequence SEQ ID NO: 4), encoding an α-rhamnosidase, as induced by high osmolarity, supporting its colonic induction.

The copper transporting ATPase identified here (Lp_3055, Table 1, transcription regulatory sequence SEQ ID NO: 19) could be involved in copper acquisition. Alternatively, this transporter could act as an exporter, thereby preventing accumulation of copper in the cytoplasm. Arbitrarily, this gene was categorized as a stress-related protein, involved in copper detoxification. Three other genes were categorized as stress-related, namely *clpC* (transcription regulatory sequence SEQ ID NO: 18), a multidrug transporter (transcription regulatory sequence SEQ ID NO: 21) and a cation efflux protein (Table 1, transcription regulatory sequence SEQ ID NO: 20), suggesting that the efficient transport of toxic compounds is important for GI-tract persistence *of L. plantarum.* Genes important in the transport of metals have been identified in many IVET screens (Mahan *et al.* 2000, supra), including a heavy metal transporter in *S. typhimurium* possibly involved in Cu²⁺ homeostasis during infection (Heithoff et al. 1997, PNAS 94, 934-9). Moreover, in *Helicobacter pylori* an STM strategy revealed two genes encoding multidrug transporters to be essential for gastric colonization of mice (Kavermann *et al.* 2003, supra). In several gram-positive microorganisms *clp* genes were demonstrated to be involved in stress response (Varmanen et al. 2000, Microbiol. 146, 1447-55; Nair et al. 2000, Mol. Microbiol. 35, 800-11; Derre et al. 1999, Mol. Microbiol. 31, 117-31). In *S. mutants* the *clpC* operon is induced at low pH (Lemos & Burne 2002, J. Bacteriol. 184, 6357-66), which could suggest that SEQ ID NO: 18 might be induced in *L. plantarum* in the mouse stomach. In *L. plantarum* the *clpC* gene is chromosomally located directly downstream of the regulator encoding gene *ctsR.* In *Listeria monocytogenes* a similar operon structure was found and mutants in the *ctsR* gene display a lower ability to survive the initial stages of murine infection in mice (Karatsas et al. 2003, Mol. Microbiol. 49, 1227-38).

Four genes encoding extracellular proteins were identified in the R-IVET screen, including two proteins (Lp_0800 and Lp_2940, Table 1 transcription regulatory sequences SEQ ID NO: 22 and 23) that contain an LPXTG-like motif involved in anchoring them to the bacterial cell wall (Mazmanian et al. 1999, Science 285, 760-3). Lp_0141 (transcription regulatory sequence SEQ ID NO: 25) contains a high number of positive charges, which could be involved in the interaction of the encoded protein with the cell wall. No putative binding domains were found in the protein encoded by Lp_1403 (transcription regulatory sequence SEQ ID NO: 25), suggesting secretion of this protein. The bacterial surface is the primary site of interaction with the host and numerous surface exposed adhesion factors have been described (Klemm & Schembri, 2000, Int. J. Med. Microbiol. 290, 27-35). Therefore, the two proteins containing an LPXTG-like motif identified here might represent factors that directly interact with host cells in the GI-tract or with components excreted in the GI-tract lumen of the host. Lp_0800 encodes a protein that is extremely rich in serine and threonine. For a serine-rich surface protein encoded by *Streptococcus pneumoniae* it has been suggested that the serine residues might be glycosylated by glycosyltransferases that are encoded by genes flanking the surface protein encoding gene (Tettelin et al. 2001, Science 293, 498-506). These glycosylated serine residues could resemble mucin like structures that coat the bacterial surface or interact with host cell mucins (Tettelin *et al.* 2001). Although no glycosyltransferase encoding genes appear to be genetically linked to Lp_0800, a similar role might be fulfilled in *L. plantarum* by the Lp_0800 encoded protein.

Three regulators were found to be induced *in vivo,* including a regulator belonging to the BglB, AraC and serine/threonine protein kinase family (corresponding transcription regulatory sequence SEQ ID NO: 26-28). The *bgl* operon in *L. plantarum* was previously shown to be down-regulated in the presence of glucose (Marasco et al. 2000, FEMS Microbiol. Letter 186, 269-73). Therefore, the BglG transcription antiterminator might be involved in the regulation of the response to the different sugars *L. plantarum* ferments during passage of the GI-tract. Remarkably, among the best homologues of Lp_3514 in the *Listeria monocytogenes* genome is the *bvrA* gene (33% identity), which encodes a BglG-family antiterminator involved in regulation of virulence gene expression (Brehm et al. 1999, J. Bacteriol. 181, 5024-32).

Eighteen genes are involved in diverse pathways, including DNA and energy metabolism, protein fate and synthesis, and fermentation (Table 1, corresponding transcription regulatory sequences SEQ ID NO: 29-43, 7, 16). Several genes in these pathways have previously been described as being important for pathogenesis in various bacteria, including the 1,3-propanediol regulator and a short chain dehydrogenase of *K. pneumonia* (Maroncle et al. 2002, Infect. Immun. 70, 4729-34), a bifunctional protein possessing alcohol and acetaldehyde dehydrogenase activity in *E. coli* K1 (Martindale et al. 2000, Mol. Microbiol. 37, 1293-305) and a DNA polymerase in *S. gordonii* (Kilic *et al.* 1999, supra). Another interesting observation is the apparent induction of PlnI, an immunity protein against plantaricin (Diep et al. 1996, J. Bacteriol. 178, 4472-83), suggesting that the production of this bacteriocin is important for *L. plantarum* in the highly competitive environment that exists in the GI-tract. Finally, 24 hypothetical proteins (16 conserved and 8 unique) apparently play a role during passage of *L. plantarum* through the GI-tract (Table 1, transcription regulatory sequence SEQ ID NO: 44-62, 19, 21). Although, these putative genes were used for extensive analysis, using the available (R)-IVET literature and blast searches, no significant homologies could be found between these *L. plantarum* hypothetical proteins and other *in vivo* induced genes found in other species. The role of these genes in *L. plantarum* GI-tract persistence remains to be determined.

Overall, a striking amount of parallels can be drawn between the pathogenic and non-pathogenic *in vivo* response, strongly suggesting that survival rather than virulence is the explanation for the importance of these genes during host residence. This suggestion is further enhanced by the fact that the gene encoding peptide methionine sulfoxide reductase has previously been identified using IVET in the food-associated microbe *L. reuteri* (Walter *et al.* 2003, supra) and the pathogen *S. gordonii* (Kilic *et al.,* 1999, supra). The similarities found for the *L. plantarum* R-IVET screen presented here are most prominent with *in vivo* screens that are performed in the same host (mice) using pathogens that infect the same host-organ (GI-tract), suggesting that organ- and host-specific factors play a key role in the determination of the microbial response. In addition, a number of functions that are specifically induced in *L. plantarum* during passage of the mouse GI-tract, which (so far) have not been identified in similar *in vivo* studies in other bacteria, might contribute to specific interactions between this bacterium and its host. Moreover, parallels between the *in vivo* study presented here and *in vitro* studies performed in the same bacterium (osmolarity induction, see above) or closely related species (osmolarity induction or low pH, see above) might hint towards spatial differentiation of *L. plantarum* gene expression during passage of the mouse GI-tract, i.e. specific induction in the stomach, small intestine or colon (see above). Interesting in this respect is the recent observation that expression of Lp_0237 (transcription regulatory sequence SEQ ID NO: 29) and Lp_0775 (transcription regulatory sequence SEQ ID NO: 12) is induced by bile, suggesting *in vivo* induction in the duodenum, as this is the site of bile release by the host. In conclusion, R-IVET performed in *L. plantarum* is a very efficient method for identifying *in vivo* induced genes and their transcription regulatory sequences.

### 2.5 Activity of GI-tract induced transcription regulatory sequences in human subjects

Preliminary experiments using human GI-tract biopsies have shown that several of the GI-tract specific genes identified above are also found to be expressed by *L. plantarum* in the human GI-tract. The identified transcription regulatory sequences of the invention are thus also specifically induced in the human GI-tract and in the vertebrate GI-tract. This is due to the fact that the physicochemical conditions encountered by bacteria in the GI-tract of mice, humans and other vertebrates are comparable (for example low pH in the stomach, stress conditions induced by bile in the duodenum, etc.). Moreover, and without limiting the invention, it may be that the interactions between the bacteria and the mucosal surfaces found in vertebrate GI-tracts require expression of the same (or very similar) genes.

## Claims

1. Use of a bacterial host in the preparation of a composition for producing a biologically active substance *in vivo* in the body cavity of a subject, wherein the bacterial host is capable of producing the biologically active substance, whereby the bacterial host contains a chimeric gene comprising a transcription regulatory sequence that is induced in at least a body cavity of the subject, whereby the transcription regulatory sequence is operably linked to a sequence encoding the biologically active substance or to a sequence encoding a polypeptide involved in the biosynthesis of the biologically active substance, **characterized in that** the transcription regulatory sequence is selected from:
(a) a sequence comprising any of the sequences of SEQ ID NO: 1-62;
(b) a sequence comprising a fragment of any of the sequences of SEQ ID NO: 1-62 whereby the fragment retains the ability to be specifically induced in a body cavity of a subject; or,
(c) a sequence which shares at least about 75 percent sequence identity with any of the sequences of (a) or (b) and which retains the ability to be specifically induced in a body cavity of a subject.

2. The use according to claim 1, whereby the transcription regulatory sequence is induced *in vivo* in at least one of the following body cavities of the subject: the stomach, the duodenum, the jejunum or the ileum, the cecum, the colon, the rectum.

3. The use according to claim 1 or 2, whereby the bacterial host is a lactic acid bacterium.

4. The use according to any of the preceding claims, wherein the bacterial host is selected from the group consisting of a food grade bacterium, a commensal bacterium or an attenuated pathogenic bacterium.

5. The use according to claim 4, wherein the bacterium is one of the genus *Lactobacillus, Lactococcus, Leuconostoc, Streptococcus, Carnobacterium, Bifidobacterium, Bacteroides, Eubacterium, Clostridium, Fusobacterium, Propionibacterium, Enterococcus, Staphylococcus, Peptostreplococcus,* or *Escherichia.*

6. A chimeric gene comprising a GI-tract-inducible transcription regulatory sequence and further comprising a coding sequence operably linked to the transcription regulatory sequence, whereby the coding sequence is not natively associated with the transcription regulatory sequence, **characterized in that** the GI-tract-inducible transcription regulatory sequence is selected from:
(a) a sequence comprising any of the sequences of SEQ ID NO: 1-62;
(b) a sequence comprising a fragment of any of the sequences of SEQ ID NO: 1-62 whereby the fragment retains the ability to be specifically induced in the GI-tract of a subject; or,
(c) a sequence which shares at least about 75 percent sequence identity with any of the sequences of (a) or (b) and which retains the ability to be specifically induced in the GI-tract of a subject.

7. A nucleic acid vector, **characterized in that** the vector comprises the chimeric gene according to claim 6.

8. A host cell **characterized in that** the host cell comprises a chimeric gene according to claim 6 or a vector according to claim 7.

9. A host cell according to claim 8, whereby the host cell is a bacterium that belongs to a genus selected from the group consisting of *Lactobacillus, Lactococcus, Leuconostoc, Carnobacterium, Streptococcus, Bifidobacterium, Bacteroides, Eubacterium. Clostridium, Fusobacterium, Propionibacterium, Enterococcus, Staphylococcus, Peptostreptococcus,* and *Escherichia.*

10. A composition comprising a host cell according claim 8 or 9.

11. A method for the production of a composition as defined in claim 10, wherein the method comprises culturing a host cell as defined in claim 8 or 9, and optionally recovery of the recombinant host cell.

12. Use of GI-tract-inducible transcription regulatory sequence in the production of a composition capable of producing a biologically active substance *in vivo* in a body cavity of a subject, **characterized in that** the GI-tract-inducible transcription regulatory sequence is selected from:
(a) a sequence comprising any of the sequences of SEQ ID NO: 1-62;
(b) a sequence comprising a fragment of any of the sequences of SEQ ID NO: 1-62 whereby the fragment retains the ability to be specifically induced in the GI-tract of a subject; or,
(c) a sequence which shares at least about 75 percent sequence identity with any of the sequences of (a) or (b) and which retains the ability to be specifically induced in the GI-tract of a subject.

13. Use of a chimeric gene as defined in claim 6, a nucleic acid vector as defined in claim 7, a recombinant host cell as defined in claim 8 or 9, in the production of a composition capable of producing a polypeptide or a biologically active substance in the body cavity of a subject.

14. Use according to claim 1-5, 12 or 13, wherein the composition is a medicament for preventing or treating an infection by a pathogenic micro-organism of a body cavity of a subject.

15. Use according to claim 14, wherein the body cavity is the gastrointestinal tract or part thereof.

16. A GI-tract lytic strain comprising a transcription regulatory sequence selected from:
(a) a sequence comprising any of the sequences of SEQ ID NO: 1-62;
(b) a sequence comprising a fragment of any of the sequences of SEQ ID NO: 1-62 whereby the fragment retains the ability to be specifically induced in a body cavity of a subject; or,
(c) a sequence which shares at least about 75 percent sequence identity with any of the sequences of (a) or (b) and which retains the ability to be specifically induced in a body cavity of a subject,
wherein said transcription regulatory sequence is operably linked to a coding sequence encoding a peptide or protein which is able to cause lysis of the GI-tract lytic strain, wherein said GI-tract lytic strain releases its cytoplasmic content following induction of said transcription regulatory sequence and wherein said cytoplasmic content comprises a biologically active substance.

## Patentansprüche

1. Verwendung eines bakteriellen Wirts bei der Zubereitung einer Zusammensetzung zur *in vivo*-Herstellung einer biologisch aktiven Substanz in der Körperhöhle eines Patienten, wobei der bakterielle Wirt zur Herstellung der biologisch aktiven Substanz in der Lage ist, wobei der bakterielle Wirt ein chimäres Gen enthält, enthaltend eine Transkriptionsregulationssequenz, die in mindestens einer Körperhöhle des Patienten induziert wird, wobei die Transkriptionsregulationssequenz in funktionsfähiger Weise mit einer für die biologisch aktive Substanz kodierenden Sequenz oder mit einer für ein an der Biosynthese der biologisch aktiven Substanz beteiligtes Polypeptid kodierenden Sequenz verbunden ist,
**dadurch gekennzeichnet, dass**
die Transkriptionsregulationssequenz aus
a) einer Sequenz, die eine beliebige der Sequenzen SEQ ID Nr. 1 bis 62 enthält,
b) einer Sequenz, die ein Fragment von einer beliebigen der Sequenzen SEQ ID Nr. 1 bis 62 enthält, wobei das Fragment die Fähigkeit beibehält, in einer Körperhöhle eines Patienten spezifisch induziert zu werden, oder
c) einer Sequenz, die mindestens etwa 75 % Sequenzidentität mit einer beliebigen der Sequenzen von a) oder b) teilt, und die die Fähigkeit beibehält, spezifisch in einer Körperhöhle eines Patienten induziert zu werden,
ausgewählt ist.

2. Verwendung nach Anspruch 1, wobei die Transkriptionsregulationssequenz *in vivo* in mindestens einer der folgenden Körperhöhlen des Patienten induziert wird: Magen, Duodenum, Jejunum oder Ileum, Caecum, Kolon, Rektum.

3. Verwendung nach Anspruch 1 oder 2, wobei der bakterielle Wirt ein Milchsäurebakterium ist.

4. Verwendung nach einem der vorangehenden Ansprüche, worin der bakterielle Wirt aus der Gruppe bestehend aus einem Bakterium in Nahrungsmittelqualität, einem kommensalen Bakterium oder einem pathogenen Bakterium in abgeschwächter Form ausgewählt ist.

5. Verwendung nach Anspruch 4, worin das Bakterium eines aus der Gattung *Lactobacillus, Lactococcus, Leuconostoc, Streptococcus, Carnobacterium, Bifidobacterium, Bacteroides, Eubacterium, Clostridium, Fusobacterium, Propionibacterium, Enterococcus, Staphylococcus, Peptostreptococcus oder Escherichia* ist.

6. Chimäres Gen, enthaltend ein im GI-Trakt induzierbares Transkriptionsregulationselement und weiterhin enthaltend eine in funktionsfähiger Weise mit dem Transkriptionsregulationselement verbundene kodierende Sequenz, wobei die kodierende Sequenz nicht nativ mit dem Transkriptionsregulationselement assoziiert ist,
**dadurch gekennzeichnet, dass**
das im Gastrointestinaltrakt induzierbare Transkriptionsregulationselement aus
a) einer Sequenz, die eine beliebige der Sequenzen SEQ ID Nr. 1 bis 62 enthält,
b) einer Sequenz, die ein Fragment von einer beliebigen der Sequenzen SEQ ID Nr. 1 bis 62 enthält, wobei das Fragment die Fähigkeit beibehält, in einer Körperhöhle eines Patienten spezifisch induziert zu werden, oder
c) einer Sequenz, die mindestens etwa 75 % Sequenzidentität mit einer beliebigen der Sequenzen von a) oder b) teilt, und die die Fähigkeit beibehält, spezifisch in einer Körperhöhle eines Patienten induziert zu werden,
ausgewählt ist.

7. Nukleinsäurevektor,
**dadurch gekennzeichnet, dass**
der Vektor das chimäre Gen nach Anspruch 6 enthält.

8. Wirtzelle,
**dadurch gekennzeichnet, dass**
die Wirtszelle ein chimäres Gen nach Anspruch 6 oder einen Vektor nach Anspruch 7 enthält.

9. Wirtszelle nach Anspruch 8, wobei die Wirtszelle ein Bakterium ist, das zu einer aus der Gruppe bestehend aus *Lactobacillus, Lactococcus, Leuconostoc, Streptococcus, Carnobacterium, Bifidobacterium, Bacteroides, Eubacterium, Clostridium, Fusobacterium, Propionibacterium, Enterococcus, Staphylococcus, Peptostreptococcus oder Escherichia* ausgewählten Gattung gehört.

10. Zusammensetzung enthaltend eine Wirtszelle nach Anspruch 8 oder 9.

11. Verfahren zur Herstellung einer wie in Anspruch 10 definierten Zusammensetzung, worin das Verfahren das Kultivieren einer wie in Anspruch 8 oder 9 definierten Wirtszelle enthält ggf. die Gewinnung einer rekombinanten Wirtszelle.

12. Verwendung einer im GI-Trakt induzierbaren Transkriptionsregulationssequenz bei der Herstellung einer Zusammensetzung, die zur *in vivo-*Herstellung einer biologisch aktiven Substanz in einer Körperhöhle eines Patienten in der Lage ist,
**dadurch gekennzeichnet, dass**
die im Gastrointestinaltrakt induzierbare Transkriptionsregulationssequenz aus
a) einer Sequenz, die eine beliebige der Sequenzen von SEQ ID Nr. 1 bis 62 enthält,
b) einer Sequenz, die ein Fragment einer beliebigen der Sequenzen von SEQ ID Nr. 1 bis 62 enthält, wobei das Fragment die Fähigkeit beibehält, im GI-Trakt eines Patienten spezifisch induziert zu werden, oder
c) einer Sequenz, die mindestens 75 % Sequenzidentität mit einer beliebigen der Sequenzen von a) und b) teilt und die die Fähigkeit beibehält, im GI-Trakt eines Patienten spezifisch induziert zu werden,
ausgewählt ist.

13. Verwendung eines wie in Anspruch 6 definierten chimären Gens, eines wie in Anspruch 7 definierten Nukleinsäurevektors, einer wie in Anspruch 8 oder 9 definierten rekombinanten Wirtszelle bei der Herstellung einer Zusammensetzung, die zur Herstellung eines Polypeptids oder einer biologisch aktiven Substanz in der Körperhöhle eines Patienten in der Lage ist.

14. Verwendung nach Anspruch 1 bis 5, 12 oder 13, worin die Zusammensetzung ein Medikament zur Verhütung oder Behandlung einer Infektion mit einem pathogenen Mikroorganismus in einer Körperhöhle eines Patienten ist.

15. Verwendung nach Anspruch 14, worin die Körperhöhle der Gastrointestinaltrakt oder ein Teil davon ist.

16. Lytischer Stamm im GI-Trakt enthaltend eine aus
a) einer Sequenz, die eine beliebige der Sequenzen SEQ ID Nr. 1 bis 62 enthält,
b) einer Sequenz, die ein Fragment von einer beliebigen der Sequenzen SEQ ID Nr. 1 bis 62 enthält, wobei das Fragment die Fähigkeit beibehält, in einer Körperhöhle eines Patienten spezifisch induziert zu werden, oder
c) einer Sequenz, die mindestens etwa 75 % Sequenzidentität mit einer beliebigen der Sequenzen von a) oder b) teilt und die die Fähigkeit beibehält, spezifisch in einer Körperhöhle eines Patienten induziert zu werden,
ausgewählte Transkriptionsregulationssequenz,
worin die Transkriptionsregulationssequenz in funktionsfähiger Weise mit einer kodierenden Sequenz verbunden ist, die für ein Peptid oder Protein kodiert, das zur Bewirkung der Lyse des lytischen Stamms im GI-Trakt in der Lage ist, worin der lytische Stamm im GI-Trakt seinen zytoplasmatischen Inhalt freisetzt, gefolgt von der Induktion der Transkriptionsregulationssequenz und worin der zytoplasmatische Inhalt eine biologisch aktive Substanz enthält.

## Revendications

1. Utilisation d'un hôte bactérien dans la préparation d'une composition pour produire une substance biologiquement active *in vivo* dans la cavité corporelle d'un sujet, où l'hôte bactérien est capable de produire la substance biologiquement active, où l'hôte bactérien contient un gène chimérique comprenant une séquence de régulation de la transcription qui est induite dans au moins une cavité corporelle du sujet, où la séquence de régulation de la transcription est liée de manière fonctionnelle à une séquence codant la substance biologiquement active ou à une séquence codant un polypeptide impliqué dans la biosynthèse de la substance biologiquement active, **caractérisée en ce que** la séquence de régulation de la transcription est choisie parmi :
(a) une séquence comprenant l'une quelconque des séquences de SEQ ID NO : 1-62 ;
(b) une séquence comprenant un fragment de l'une quelconque des séquences de SEQ ID NO : 1-62 où le fragment conserve l'aptitude à être induit spécifiquement dans une cavité corporelle d'un sujet ; ou
(c) une séquence qui partage au moins environ 75 % d'identité de séquence avec l'une quelconque des séquences de (a) ou (b) et qui conserve l'aptitude à être induite spécifiquement dans une cavité corporelle d'un sujet.

2. Utilisation selon la revendication 1 où la séquence de régulation de la transcription est induite *in vivo* dans au moins l'une des cavités corporelles suivantes du sujet : l'estomac, le duodénum, le jéjunum ou l'iléon, le cæcum, le côlon, le rectum.

3. Utilisation selon la revendication 1 ou 2 où l'hôte bactérien est une bactérie lactique.

4. Utilisation selon l'une quelconque des revendications précédentes où l'hôte bactérien est choisi dans le groupe consistant en une bactérie de qualité alimentaire, une bactérie commensale et une bactérie pathogène atténuée.

5. Utilisation selon la revendication 4 où la bactérie est une bactérie du genre *Lactobacillus, Lactococcus, Leuconostoc, Streptococcus, Carnobacterium, Bifidobacterium, Bacteroides, Eubacterium, Clostridium, Fusobacterium, Propionibacterium, Enterococcus, Staphylococcus, Peptostreptococcus* ou *Escherichia.*

6. Gène chimérique comprenant une séquence de régulation de la transcription inductible dans les voies GI et comprenant en outre une séquence codante liée de manière fonctionnelle à la séquence de régulation de la transcription, où la séquence codante n'est pas associée de manière native avec la séquence de régulation de la transcription, **caractérisé en ce que** la séquence de régulation de la transcription inductible dans les voies GI est choisie parmi :
(a) une séquence comprenant l'une quelconque des séquences de SEQ ID NO : 1-62 ;
(b) une séquence comprenant un fragment de l'une quelconque des séquences de SEQ ID NO : 1-62 où le fragment conserve l'aptitude à être induit spécifiquement dans les voies GI d'un sujet ; ou
(c) une séquence qui partage au moins environ 75 % d'identité de séquence avec l'une quelconque des séquences de (a) ou (b) et qui conserve l'aptitude à être induite spécifiquement dans les voies GI d'un sujet.

7. Vecteur d'acide nucléique **caractérisé en ce que** le vecteur comprend le gène chimérique selon la revendication 6.

8. Cellule hôte **caractérisée en ce que** la cellule hôte comprend un gène chimérique selon la revendication 6 ou un vecteur selon la revendication 7.

9. Cellule hôte selon la revendication 8 où la cellule hôte est une bactérie qui appartient à un genre choisi dans le groupe consistant en *Lactobacillus, Lactococcus, Leuconostoc, Carnobacterium, Streptococcus, Bifidobacterium, Bacteroides, Eubacterium, Clostridium, Fusobacterium, Propionibacterium, Enterococcus, Staphylococcus, Peptostreptococcus* et *Escherichia.*

10. Composition comprenant une cellule hôte selon la revendication 8 ou 9.

11. Procédé pour la production d'une composition selon la revendication 10 où le procédé comprend la culture d'une cellule hôte selon la revendication 8 ou 9 et éventuellement la récupération de la cellule hôte recombinante.

12. Utilisation d'une séquence de régulation de la transcription inductible dans les voies GI dans la production d'une composition capable de produire une substance biologiquement active *in vivo* dans une cavité corporelle d'un sujet, **caractérisée en ce que** la séquence de régulation de la transcription inductible dans les voies GI est choisie parmi :
(a) une séquence comprenant l'une quelconque des séquences de SEQ ID NO : 1-62 ;
(b) une séquence comprenant un fragment de l'une quelconque des séquences de SEQ ID NO : 1-62 où le fragment conserve l'aptitude à être induit spécifiquement dans les voies GI d'un sujet ; ou
(c) une séquence qui partage au moins environ 75 % d'identité de séquence avec l'une quelconque des séquences de (a) ou (b) et qui conserve l'aptitude à être induite spécifiquement dans les voies GI d'un sujet.

13. Utilisation d'un gène chimérique selon la revendication 6, d'un vecteur d'acide nucléique selon la revendication 7, d'une cellule hôte recombinante selon la revendication 8 ou 9 dans la production d'une composition capable de produire un polypeptide ou une substance biologiquement active dans la cavité corporelle d'un sujet.

14. Utilisation selon les revendications 1-5, 12 ou 13 où la composition est un médicament pour prévenir ou traiter une infection par un micro-organisme pathogène d'une cavité corporelle d'un sujet.

15. Utilisation selon la revendication 14 où la cavité corporelle est les voies gastro-intestinales ou une partie de celles-ci.

16. Souche lytique des voies GI comprenant une séquence de régulation de la transcription choisie parmi :
(a) une séquence comprenant l'une quelconque des séquences de SEQ ID NO : 1-62 ;
(b) une séquence comprenant un fragment de l'une quelconque des séquences de SEQ ID NO : 1-62 où le fragment conserve l'aptitude à être induit spécifiquement dans une cavité corporelle d'un sujet ; ou
(c) une séquence qui partage au moins environ 75 % d'identité de séquence avec l'une quelconque des séquences de (a) ou (b) et qui conserve l'aptitude à être induite spécifiquement dans une cavité corporelle d'un sujet,
où ladite séquence de régulation de la transcription est liée de manière fonctionnelle à une séquence codante codant un peptide ou une protéine qui est capable de provoquer la lyse de la souche lytique des voies GI, où ladite souche lytique des voies GI libère son contenu cytoplasmique après induction de ladite séquence de régulation de la transcription et où ledit contenu cytoplasmique comprend une substance biologiquement active.
